# EUROPEAN PATENT APPLICATION

(11) **EP 2 298 924 A2**
(43) Date of publication of application: **23.03.2011**
(21) Application number: 10174369.8
(22) Date of filing: 08.06.2004
(51) Int. Cl.: C12N 15/86, A61K 39/12, C12N 7/00

(54) **Improved method for the recovery of non-segmented, negative-stranded RNA viruses from cDNA**

(30) Priority: 09.06.2003 US 477389 P
(62) Divisional of application: 04754803.7
(71) Applicant: Wyeth LLC, Madison, NJ 07940 (US)
(72) Inventor: Parks, Christopher L., Boonton, NJ 07005-2310 (US); Udem, Stephen A., New York, NY 10023 (US); Sidhu, Modinderjit S., New York, NY 10956-5426 (US)
(74) Representative: Ricol, David Gerard

(57) **Abstract**

Methods for producing infectious, non-segmented, negative-stranded RNA viruses of the Order *Mononegavirales* are provided that involve coexpression of a viral cDNA along with essential viral proteins, N, P, and L in a host cell transiently transfected with an expression vector encoding an RNA polymerase. In alternate methods, after the host cell is transfected with a viral cDNA expression vector and one or more vectors encoding the RNA polymerase, N protein, P protein, and L protein, the host cell is exposed to an effective heat shock under conditions sufficient to increase recovery of the recombinant virus. In other alternate embodiments, the host cells are transferred after viral rescue begins into co-culture with a plaque expansion cell, typically a monolayer of expansion cells, and the assembled infectious, non-segmented, negative-stranded RNA virus is recovered from the co-culture. Also provided within the invention are compositions for producing infectious, non-segmented, negative-stranded RNA virus of the Order *Mononegavirales,* recombinant viruses produced using the foregoing methods and compositions, and immunogenic compositions and methods employing the recombinant viruses. In additional embodiments, the methods and compositions of the invention are employed to produce growth- or replication-defective non-segmented negative-stranded RNA viruses and subviral particles.

## Description

### Cross Reference to Related Applications

The present application claims priority from U.S. Provisional Application 60/477,389, filed June 9, 2003, which is incorporated herein by reference.

### Field of the Invention

The invention relates to improved methods and compositions for producing infectious, replicating, non-segmented, negative-stranded RNA viruses of the order designated *Mononegavirales.* The methods and compositions are also adapted for producing growth- or replication- defective versions of these viruses.

### Background of the Invention

Enveloped, non-segmented, negative-sense, single-stranded RNA viruses are uniquely organized and expressed. The genomic RNA of negative-sense, single- stranded viruses serves two template functions in the context of a nucleocapsid: as a template for the synthesis of messenger RNAs (mRNAs) and as a template for the synthesis of the antigenome (+) strand. Negative-sense, single-stranded RNA viruses encode and package their own RNA-dependent RNA polymerase. Messenger RNAs are only synthesized once the nucleocapsid has entered the cytoplasm of the infected cell. Viral replication occurs after synthesis of the mRNAs and requires continuous synthesis of viral proteins. The newly synthesized antigenome (+) strand serves as the template for generating further copies of the (-) strand genomic RNA.

The RNA-dependent polymerase complex actuates and achieves transcription and replication by engaging cis-acting signals at the 3' end of the genome, in particular, the promoter region. Viral genes are then transcribed from the genome template unidirectionally from its 3' to its 5' end. There is less mRNA made from the downstream genes (e.g., the polymerase gene (L)) relative to their upstream neighbors (*e.g.,* the nucleoprotein gene (N)). Therefore, there is a gradient of mRNA abundance according to the position of the genes relative to the 3'-end of the genome.

For many non-segmented, negative-stranded RNA viruses, no effective vaccines of any kind are available. Thus, there is a compelling need to develop improved immunogenic compositions and methods directed against such human and animal pathogens.

A variety of approaches can be employed in seeking to develop immunogenic compositions and methods directed against non-segmented, negative-stranded RNA viruses, including the use of: (1) purified individual viral protein vaccines (subunit vaccines); (2) inactivated whole virus preparations; and (3) live, attenuated viruses.

Subunit vaccines have the desirable feature of being pure, definable and relatively easily produced in abundance by various means, including recombinant DNA expression methods. To date, with the notable exception of hepatitis B surface antigen, viral subunit vaccines have generally only elicited short-lived and/or inadequate immunity, particularly in naive recipients.

Formalin inactivated whole virus preparations of polio (IPV) and hepatitis A have proven safe and efficacious. In contrast, immunization with similarly inactivated whole viruses such as respiratory syncytial virus and measles virus vaccines elicited unfavorable immune responses and/or response profiles which predisposed vaccinees to exaggerated or aberrant disease when subsequently confronted with naturally-occurring or "wild-type" virus.

Early attempts (1966) to immunize young children used a parenterally administered formalin-inactivated RSV vaccine. Unfortunately, several field trials of this vaccine revealed serious adverse reactions, including severe illness with unusual features, following subsequent natural infection with RSV (Kapikian et al., Am J Epidemiol. 4:405-21, 1969; Kim et al., Am J Epidemiol. 4:422-34, 1969; Collins et al., Respiratory Syncytial Virus. In "Field's Virology", 3rd Edition. Lippincott-Raven, 1443-1475, 2001; Collins et al., Adv. Virus. Res., 54:423-451, 1999). It has been suggested that this formalinized RSV antigen elicited an abnormal or unbalanced immune response profile, predisposing the vaccinee to RSV disease (Collins et al., Respiratory Syncytial Virus. In "Field's Virology", 3rd Edition. Lippincott-Raven, 1443-1475, 2001; Collins et al., Adv. Virus. Res., 54:423-451).

Thereafter, live, attenuated RSV vaccine candidates were generated by cold passage or chemical mutagenesis. These RSV strains were found to have reduced virulence in seropositive adults. Unfortunately, they proved either over or under-attenuated when given to seronegative infants; in some cases, they also were found to lack genetic stability (Collins et al., Respiratory Syncytial Virus. In "Field's Virology", 3rd Edition. Lippincott-Raven, 1443-1475, 2001; Collins et al., Adv. Virus. Res., 54:423-451). Another immunization approach using parenteral administration of live virus was ineffective and efforts along this line were discontinued (Collins et al., Respiratory Syncytial Virus. In "Field's Virology", 3rd Edition. Lippincott-Raven, 1443-1475,2001; Collins et al., Adv. Virus. Res., 54:423-451). Notably, these live RSV vaccines were never associated with disease enhancement as observed with the formalin-inactivated RSV vaccine described above. Currently, there are no RSV vaccines approved for administration to humans, although clinical trials are now in progress with various attenuated mutants of RSV.

Appropriately attenuated live derivatives of wild-type viruses offer distinct advantages for use in immunogenic compositions and methods. As live, replicating agents they initiate infection in recipients during which viral gene products are expressed, processed and presented in the context of the immunized subject's specific MHC class I and II molecules. In this manner, live attenuated viral candidates are effective for eliciting humoral and cell-mediated immune responses, as well as stimulating coordinate cytokine and chemokine patterns, which parallel the immune response profile of survivors of natural infection.

This favorable immune response pattern is contrasted with the delimited responses elicited by inactivated or subunit vaccines, which typically are largely restricted to the humoral immune surveillance arm. Further, the immune response profile elicited by some formalin inactivated whole virus vaccines, e.g., measles and respiratory syncytial virus vaccines developed in the 1960's, have not only failed to provide sustained protection, but in fact have led to a predisposition to aberrant, exaggerated, and even fatal illness, when the vaccine recipient later confronted the wild-type virus.

While live, attenuated viruses have highly desirable characteristics for use in immunogenic compositions and methods, they have proven to be difficult to generate and evaluate. The crux of the difficulty lies in the need to isolate a derivative of the wild-type virus which has lost its disease-producing potential (*i*.*e*., virulence), while retaining sufficient replication competence to infect the recipient and elicit the desired immune response profile in adequate abundance.

Historically, this delicate balance between virulence and attenuation has been achieved by serial passage of a wild-type viral isolate through different host tissues or cells under varying growth conditions (such as temperature). This process presumably favors the growth of viral variants (mutants), some of which have the favorable characteristic of attenuation. Occasionally, further attenuation is achieved through chemical mutagenesis as well.

This propagation/passage scheme typically leads to the emergence of virus derivatives which are temperature sensitive (*ts*), cold-adapted (*ca*) and/or altered in their host range (*hr*)-one or all of which are changes from the wild-type, disease-causing viruses -- *i*.*e*., changes that may be associated with attenuation.

Several live virus vaccines, including those for the prevention of measles and mumps (which are paramyxoviruses), and for protection against polio and rubella (which are positive-stranded RNA viruses), have been generated by this approach and provide the mainstay of current childhood immunization regimens throughout the world.

Nevertheless, this process for generating attenuated live virus vaccine candidates is lengthy and can be unpredictable, relying largely on the selective outgrowth of randomly occurring genomic mutants with desirable attenuation characteristics. Often the resultant viral candidates for use in immunogenic compositions remain underattenuated or overattenuated in immunized target subjects.

Even for current vaccines in use, there is still a need for more efficacious, live attenuated viral candidates to yield improved immunogenic compositions and protocols. For example, the current measles vaccines provide reasonably good protection. However, recent measles epidemics suggest deficiencies in the efficacy of current vaccines. Despite maternal immunization, high rates of acute measles infection have occurred in children under age one, reflecting the vaccines' inability to induce anti-measles antibody levels comparable to those developed following wild-type measles infection (Griffin, Measles Virus. In "Field's Virology", 3rd Edition. Lippincott-Raven, 1401-1442, 2001; Cutts et al., J. Infectious Dis., 170:S32-S41, 1994). As a result, vaccine-immunized mothers are less able to provide their infants with sufficient transplacentally-derived passive antibodies to protect the newborns beyond the first few months of life.

Acute measles infections in previously immunized adolescents and young adults point to an additional problem. These secondary vaccine failures indicate limitations in the current vaccines' ability to induce and maintain antiviral protection that is both abundant and long-lived (Atkinson et al., United States. Annu. Rev. Med., 43:451-463, 1992). Recently, yet another potential problem was revealed. The hemagglutinin protein of wild-type measles isolated over the past 15 years has shown a progressively increasing distance from the vaccine strains (Bellini et al., Emerging Infectious Diseases, 4:29-35, 1998). This "antigenic drift" raises legitimate concerns that the vaccine strains may not contain the ideal antigenic repertoire needed to provide optimal protection. Thus, there is a need for improved immunogenic compositions.

The recovery of recombinant, non-segmented negative-stranded RNA viruses from cDNA requires a specialized rescue system, because introduction of viral genomic RNA (vRNA) into a cell by itself is not sufficient to initiate virus replication. Initiation of the virus replication cycle also requires the intracellular synthesis of three or more viral *trans*-acting proteins that include components of the viral nucleocapsid and polymerase complex.

A number of different rescue protocols have been attempted for recovery of recombinant non-segmented negative stranded RNA viruses from cDNA. Most of these reported protocols include a co-transfection step wherein host cells are transfected with multiple plasmids, including a viral genomic cDNA clone plus expression vectors encoding essential viral proteins, for example the nucleocapsid protein (N or NP), phosphoprotein (P), and the large polymerase subunit (L).

In rescue protocols designed specifically for the non-segmented, negative-stranded viruses, intracellular expression of T7 RNA polymerase has been employed to facilitate transcription of transfected DNAs. The T7 RNA polymerase has been exploited for this purpose because: i) with an appropriately designed viral cDNA plasmid, T7 polymerase can be forced to initiate vRNA synthesis at the precise 5' end of the viral genome, ii) T7 RNA polymerase directs high level expression in the cell cytoplasm where this class of virus replicates, and iii) cytoplasmic synthesis of RNA eliminates the possibility that vRNA may be processed by nuclear splicing or polyadenylation pathways. These characteristics make it highly advantageous to design rescue systems based on T7 RNA polymerase.

Although there are important advantages associated with using T7 RNA polymerase in a rescue system, there are also disadvantages. To achieve high-level, intracellular expression of T7 RNA polymerase, most rescue systems require that the transfected cells also be infected with a recombinant vaccinia virus that expresses a phage polymerase gene (for example, MVA/T7) (*see, e.g.,* Pattnaik et al., J. Virol. 64:2948-2957, 1990). Adopting this general rescue method, Lawson et al. (Proc. Natl. Acad. Sci. USA 92:4477-4481, 1995) reported successful recovery of vesicular stomatitis virus (VSV). Similarly, U.S. Patent No. 6,033,886 issued to Conzelmann on March 7, 2000 reports successful recovery of rabies virus (RV) from cDNA by cotransfecting a RV cDNA genome with plasmids encoding RV N, P, and L proteins into baby hamster kidney (BHK) cells infected with a recombinant vaccinia virus vTF7-3 to express the T7 RNA polymerase.

Despite these and other successful reports involving vaccinia-based recovery systems, there remain important disadvantages associated with the use of recombinant vaccinia viruses in viral recovery systems. In particular, this recovery method requires that the desired virus be purified free from the vaccinia helper virus. In particular, the vaccinia helper virus must be separated from the rescued virus to achieve recovery. Complicating this recovery, vaccinia can actually interfere with replication of the virus to be rescued. For viruses like RV and VSV, which replicate, to high titers, these factors are less of a problem. However, many other non-segmented negative stranded RNA viruses grow more slowly and may be difficult to recover in vaccinia-based systems, for example, respiratory syncytial virus (RSV), human parainfluenza viruses (HPIVs), bovine parainfluenza virus type 3 (BPIV3), and measles virus (MV). A related drawback to vaccinia-based recovery systems arises from the extensive cytopathic effect (CPE) that vaccinia can cause in the host cell population, even after several passages. CPE caused by vaccinia virus can obscure detection of plaques formed by the rescued virus, and may also reduce yields of rescued virus by diminishing the ability of the cell culture to support replication. It is also likely that vaccinia virus-induced CPE will further hinder, or even preclude, rescue of slow growing, attenuated viruses by limiting the time window during which the transfected cells remain viable. Finally, residual vaccinia virus contamination is a significant concern if the rescued, recombinant virus is intended to become a seed stock for preparing immunogenic compositions or developing gene therapy vectors.

Alternative rescue systems for non-segmented negative stranded RNA viruses employ specialized "helper" cell lines that constitutively express a phage polymerase gene. Exemplary T7 helper cell lines have been reported to effectively circumvent problems associated with vaccinia virus-based systems. One system reported by Billeter et al. (International Publication No. WO 97/06270, incorporated herein by reference) was developed to recover infectious measles virus (MV) from cDNA. The system of Billeter et al. replaces helper virus with a helper cell line that constitutively expresses an RNA polymerase, for example the T7 RNA polymerase. This helper cell line also provides constitutive, "genomic expression" of the N and P proteins of MV. Into this specially modified cell line, a cDNA plasmid is introduced comprising an entire (+) strand sequence of MV operatively linked to an expression control sequence. Along with the MV cDNA, a plasmid encoding the L protein of MV is cotransfected and expressed transiently in the helper cell line.

This alternative, T7 helper cell-based recovery system, although reported as a successful tool for MV recovery, suffers from a distinct set of drawbacks over the vaccinia helper systems discussed above. Most importantly, the system of Billeter et al. that replaces helper virus with a specific helper cell line constitutively expressing the T7 RNA polymerase, along with the N and P proteins of MV, would not appear to be applicable for rescue of other non-segmented negative stranded RNA viruses. Instead, when such specialized helper cell lines are used, a new cell line may have to be constructed for each distinct virus to be rescued.

An alternate cell line for recovery of non-segmented negative-stranded RNA viruses also features constitutive expression of only the T7 polymerase in the helper cell. Thus, Buchholz et al. (J. Virol. 73:251-259, 1999, incorporated herein by reference) constructed a BHK-21 cell line that constitutively expresses a T7 RNA polymerase. The advantage to this system is that the cell line is less specialized because it does not also constitutively express an N, P, or L protein of a specific virus, and the cell line is reportedly stable in its expression of the T7 polymerase after 100 successive passages.

Nonetheless, all helper cell lines feature similar drawbacks in terms of their difficulty of construction, and the limited usefulness of the cell lines to provide for recovery of a broad range of different non-segmented negative-stranded RNA viruses. Currently, only a few T7 helper cell lines have been developed that express high levels of the polymerase and will support viral rescue. Experience has shown that it can be difficult to isolate new T7 cell lines to fill a specific need for different viruses and emerging strains. This greatly limits the number of T7 cell lines available for rescue, and presents serious problems for producing qualified cell lines for many non-segmented negative stranded RNA viruses. A related problem arises for non-segmented negative stranded RNA viruses that display specific cell tropism and will not grow in any available T7 helper cell lines.

Although there have been numerous advances toward recovery and production of live-attenuated non-segmented negative stranded RNA viruses, there remains a clear need in the art for additional tools and methods to engineer safe and effective immunogenic compositions to alleviate the serious health problems attributable to these pathogens. Among the remaining challenges in this context is the need for additional tools to more efficiently rescue non-segmented negative stranded RNA viruses using cDNA-based recovery systems. To facilitate these goals, existing methods for recovery of non-segmented negative stranded RNA viruses from cDNA must be expanded. In this context, it is particularly desirable to develop methods and compositions for recovery and genetic manipulation of non-segmented negative stranded RNA viruses that do not require the use of recombinant vaccinia virus or specialized cell lines modified to constitutively express a T7 RNA polymerase. Surprisingly, the present invention satisfies these needs and fulfills additional objects and advantages as described herein below.

### Summary of the Invention

The instant invention provides for efficient recovery of a large array of non-segmented, negative-stranded RNA viruses from cloned cDNA, using compositions and methods that are independent of helper virus (e.g., vaccinia virus) and do not require specialized helper cell lines engineered to constitutively express an RNA polymerase.

In one aspect of the invention, a method for producing an infectious, non-segmented, negative-stranded RNA virus of the Order *Mononegavirales* involves introducing a viral cDNA expression vector comprising a polynucleotide encoding a genome or antigenome of a non-segmented negative-stranded RNA virus operably linked with an expression control sequence to direct synthesis of viral RNA transcripts from the cDNA expression vector into a host cell, wherein the host cell expresses an N protein, a P protein, and an L protein, and transiently expresses an RNA polymerase from a transiently transfected expression vector, and thereafter recovering assembled infectious, non-segmented, negative-stranded RNA virus from the host cells.

Within alternate embodiments of the invention, a method for producing a growth- or replication-defective, non-segmented, negative-stranded RNA virus of the Order *Mononegavirales* is provided. For these embodiments, it is desired to produce a defective virus that is growth- or replication-defective or incompetent following rescue, for example a virus that is limited in its ability to grow or replication *in vitro, or in vivo*. Within these embodiments of the invention, a cDNA expression construct encoding a genome or antigenome of a growth- or replication-defective non-segmented negative-stranded RNA virus operably linked with an expression control sequence to direct synthesis of viral RNA transcripts from the cDNA expression vector is introduced into a host cell that expresses an N protein, a P protein, and an L protein, and transiently expresses an RNA polymerase from a transiently transfected expression vector. The growth- or replication-defective, non-segmented, negative-stranded RNA virus is thereafter recovered from the host cells for use in immunogenic compositions or for other purposes. Often, growth- and replication-defective viruses and subviral particles will be employed as "vectors", as described in detail herein below.

Within other embodiments of the invention, a method for producing an infectious, non-segmented, negative-stranded RNA virus of the Order *Mononegavirales* involves introducing a viral cDNA expression vector comprising a polynucleotide that encodes a genome or antigenome of a non-segmented negative-stranded RNA virus operably linked with an expression control sequence to direct synthesis of viral RNA transcripts from the cDNA expression vector, into a host cell transiently expressing:
(1) an RNA polymerase,
(2) an N protein,
(3) a P protein, and
(4) an L protein,
   wherein each of the RNA polymerase and N, P, and L proteins is expressed from a transiently transfected expression vector; and thereafter recovering an assembled infectious, non-segmented, negative-stranded RNA virus from the host cells.

In yet another embodiment, a method for producing an infectious, non-segmented, negative-stranded RNA virus of the Order *Mononegavirales* involves introducing into a suitable host cell a viral cDNA expression vector comprising a polynucleotide encoding a genome or antigenome of a non-segmented negative-stranded RNA virus operably linked with an expression control sequence to direct synthesis of viral RNA transcripts from the viral cDNA expression vector, and one or more transient expression vectors that encode(s) and direct(s) transient expression of an RNA polymerase, an N protein, a P protein, and an L protein, and thereafter recovering assembled infectious, non-segmented, negative-stranded RNA virus from the host cells.

Within more detailed aspects of the invention, a method for producing an infectious, non-segmented, negative-stranded RNA virus of the Order *Mononegavirales* involves introducing into a suitable host cell a viral cDNA expression vector comprising a polynucleotide encoding a genome or antigenome of a non-segmented negative-stranded RNA virus operably linked with an expression control sequence to direct synthesis of viral RNA transcripts from the viral cDNA expression vector, and one or more transient expression vectors that encode(s) and direct(s) transient expression of an RNA polymerase, an N protein, a P protein, and an L protein. After one or more of the viral cDNA expression vectors and the one or more transient expression vectors encoding the RNA polymerase, N protein, P protein, and L protein are introduced into the host cell, the host cell is exposed to an effective heat shock under conditions sufficient to increase recovery of the recombinant virus.

Within other aspects of the invention, a method for producing an infectious, non-segmented, negative-stranded RNA virus of the Order *Mononegavirales* involves introducing into a suitable host cell a viral cDNA expression vector comprising a polynucleotide encoding a genome or antigenome of a non-segmented negative-stranded RNA virus operably linked with an expression control sequence to direct synthesis of viral RNA transcripts from the viral cDNA expression vector, and one or more transient expression vectors that encode(s) and direct(s) transient expression of an RNA polymerase, an N protein, a P protein, and an L protein under conditions sufficient to permit co-expression of the vectors and production of the recombinant virus. The host cells are thereafter transferred into co-culture with a plaque expansion cell, typically provided as a monolayer of expansion cells, and the assembled infectious, non-segmented, negative-stranded RNA virus is recovered from the co-culture.

Also provided within the invention are compositions for producing an infectious, non-segmented, negative-stranded RNA virus of the Order *Mononegavirales*. The compositions include a viral cDNA expression vector comprising a polynucleotide encoding a genome or antigenome of a non-segmented negative-stranded RNA virus operably linked with an expression control sequence to direct synthesis of viral RNA transcripts from the cDNA in a suitable host cell, and one or more transient expression vectors that encode(s) and direct(s) transient expression in the host cell of:
(1) an RNA polymerase,
(2) an N protein,
(3) a P protein,
(4) an L protein,
   and a transient expression vector that encodes and directs transient expression in said host cell of an RNA polymerase.

In alternative embodiments, compositions are provided as above, wherein the non-segmented negative-stranded RNA virus is a growth- or replication-defective virus. These and related compositions within the invention can be provided in various preparative or active formulations and mixtures. For example, the foregoing composition may be provided, wherein the viral cDNA expression vector and the one or more transient expression vector(s) that encode(s) and direct(s) transient expression of the RNA polymerase, N, P, and L proteins are combined in a co-transfection mixture. Alternatively, the above components can be combined within a co-transfected host cell or co-culture of a transfected host cell and plaque expansion cell.

Additional embodiments of the invention relate to the recombinant, non-segmented negative-stranded RNA virus produced according to the foregoing methods, as well as immunogenic compositions containing such viruses and methods for eliciting an immune response in mammalian subjects involving administration of the subject viruses and immunogenic compositions.

### Brief Description of the Figures

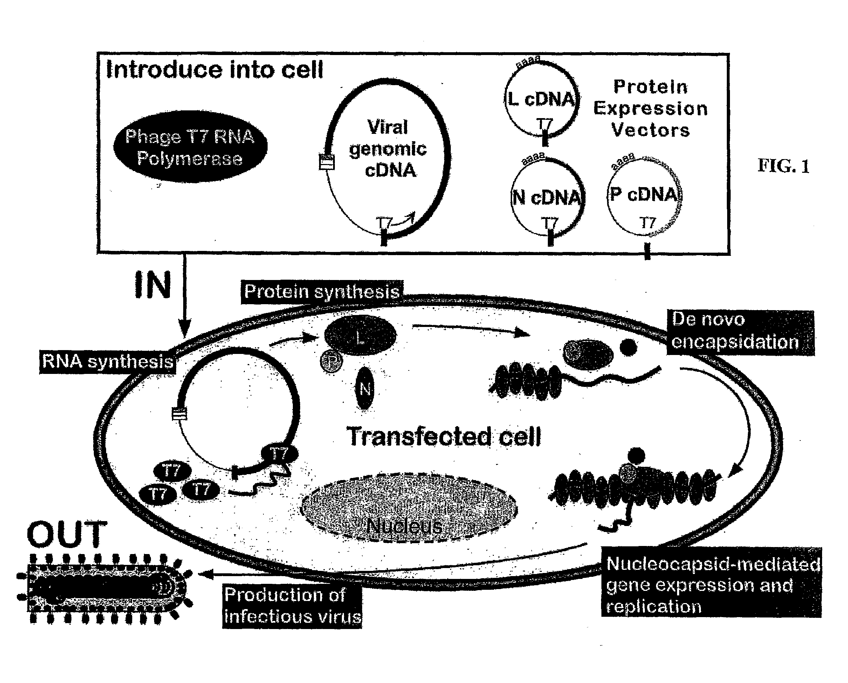

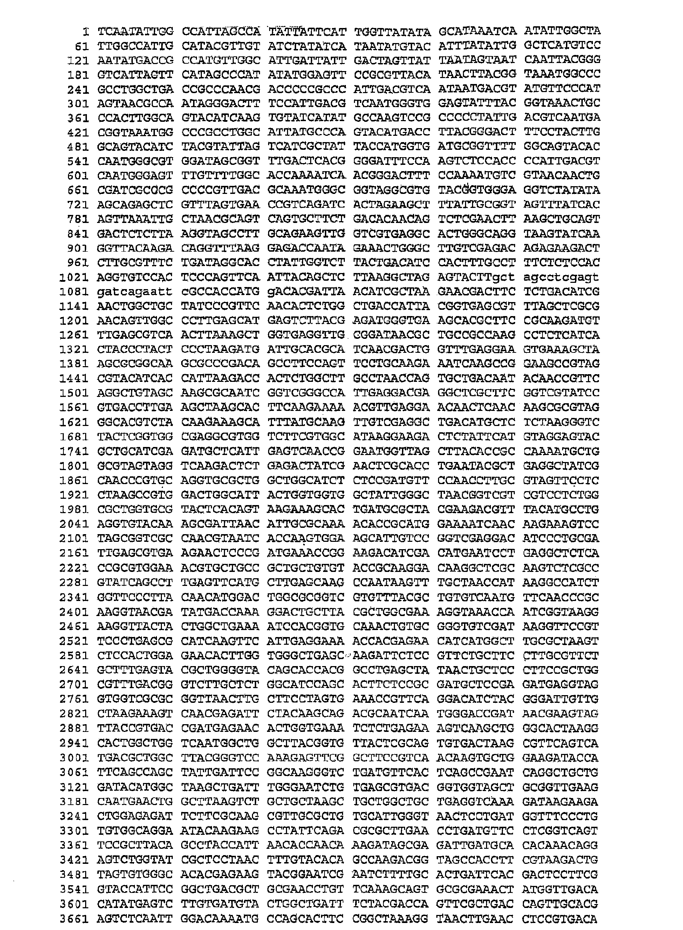

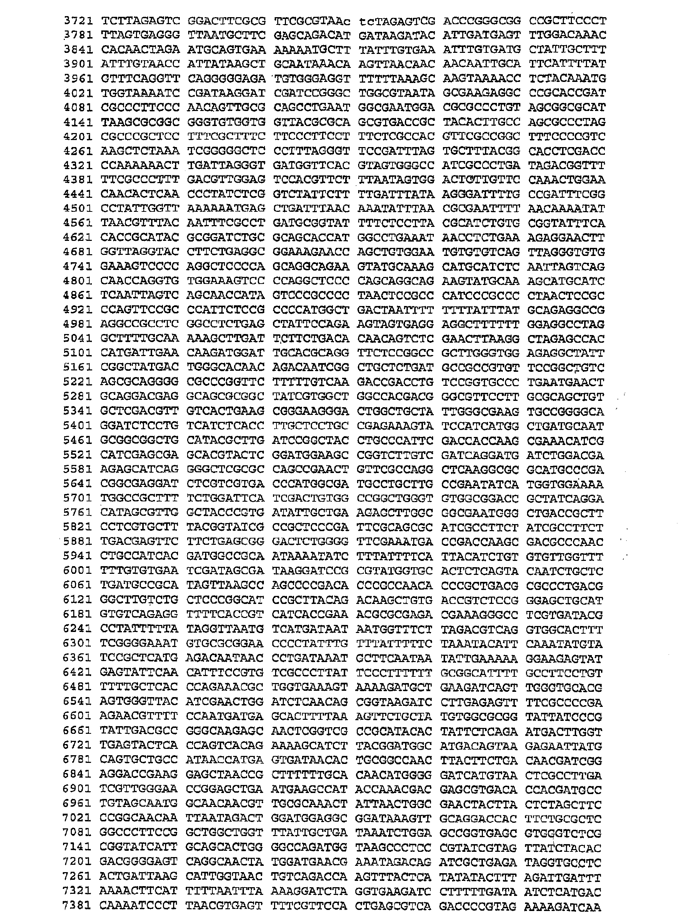

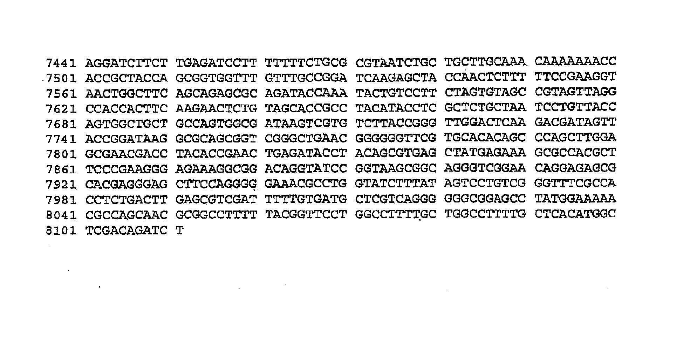

Figure 1 depicts the rescue of RSV using a plasmid to supply T7 rather than MVA-T7.

Figures 2A-2C provide an exemplary sequence (SEQ ID NO: 1) of a transient T7 polymerase expression vector, plasmid pCl-Neo-Bcl-T7. ATG is the T7 pol initiator codon. The second codon of T7 pol was changed from AAC to gAC to optimize the translation initiation context (Kozak sequence). The sequence GCCACC was also added to optimize the translation initiation context. The lower case portion of the sequence, gct agcctcgagt gatcagaatt c, was inserted in place of the T7 promomter in the pCI-Neo vector before adding the T7 RNA polymerase coding sequence. The sequence includes NheI, XhoI, BclI, and EcoRI restriction sites.

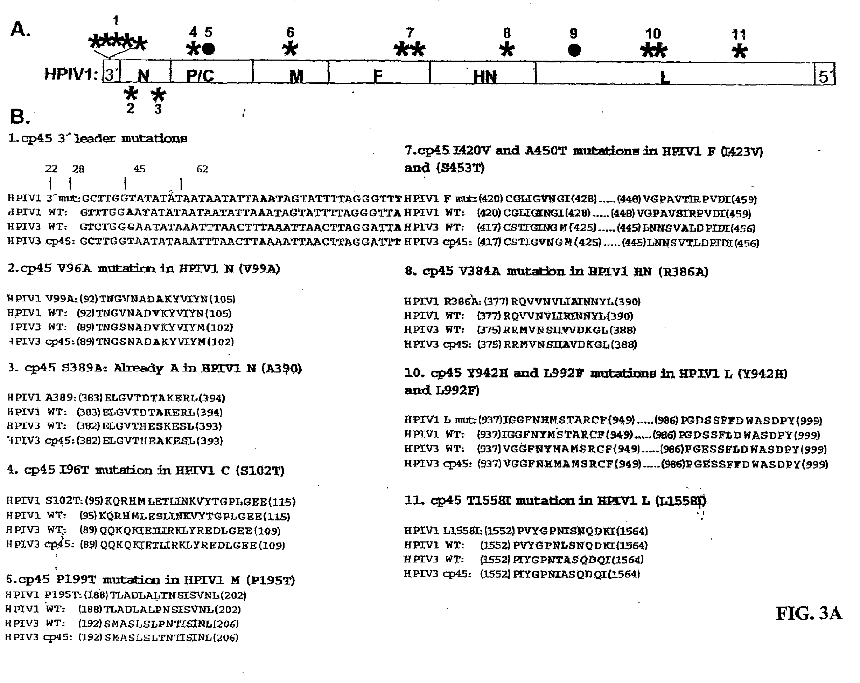

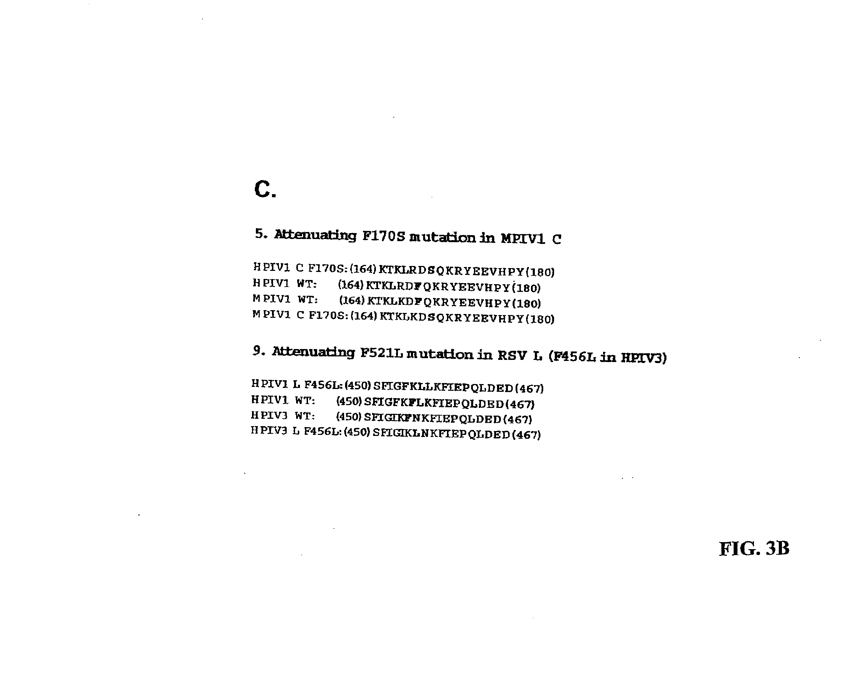

Figures 3A and 3B (Panels A-C) demonstrate that various mutations identified in a heterologous negative stranded RNA virus can be incorporated into a recombinant non-segmented negative-stranded RNA virus of the invention (in this case into a HPIV1 construct) to yield attenuation or other desired phenotypic changes.

### Detailed Description of the Invention

The instant invention provides for efficient recovery of a large array of non-segmented, negative-stranded RNA viruses from cloned cDNA, using compositions and methods that are independent of helper virus (e.g., vaccinia virus) and do not require specialized helper cell lines engineered to constitutively express an RNA polymerase.

In one aspect of the invention, methods and compositions for efficient rescue of a non-segmented negative-stranded RNA virus are provided that incorporate a transient expression vector to transiently express an RNA polymerase in a host cell capable of supporting rescue of the subject virus. Within this transient polymerase vector rescue system, an expression vector encoding, for example, a T7 RNA polymerase is coordinately introduced into the host cell along with a viral genomic or antigenomic cDNA, wherein the host cell expresses essential viral support proteins (e.g., N, P and L proteins) necessary to support viral rescue. An illustration of the rescue system of the invention as applied for rescue of RSV using a plasmid to supply T7 is depicted in Figure 1.

Thus, in the viral recovery methods and compositions of the invention, a host cell is programmed to express an RNA polymerase by a transiently transfected plasmid that is transcribed in the nucleus of the cell, thereby supporting viral recovery in a vaccinia-free system and in cells adaptable to rescue of different species or types of non-segmented negative-stranded RNA viruses.

The utility and versatility of the methods and compositions described herein (e.g., in comparison to vaccinia-based and helper cell-based recovery systems) overcome such projected obstacles as low transfection efficiency and short-term expression that ordinarily attend transient vector expression systems. In contrast to T7 helper cell systems, where all cells constitutively express a T7 polymerase, transfection efficiency in a transient expression system is generally as low as about 10-20%. In addition, transient expression, e.g., of a T7 polymerase, is predicted to decay and shut off within a limited time period of approximately 72 hours. On this basis, the disclosure herein of a versatile system for efficiently rescuing non-segmented negative-stranded RNA viruses, including a diverse array of slow growing and attenuated species and strains, using a transient RNA polymerase cotransfection system, was unexpected.

The rescue of negative-stranded RNA viruses using the transient RNA polymerase expression system of the invention provides several advantages over existing systems. In particular, there is no need to infect cells with recombinant vaccinia virus to provide an RNA polymerase, thereby eliminating CPE and other deleterious effects on the efficiency and fidelity of viral recovery and development of effective immunogenic compositions. A related advantage is that the transfected cells remain viable and support virus rescue for a longer time period after initiation of rescue, for the duration of transfection. This helps promote rescue of slow growing species and strains that are common among non-segmented negative-stranded RNA viruses. In addition, the present rescue system eliminates the need to purify recombinant vaccinia virus away from the desired recombinant virus. For developing immunogenic compositions and gene therapy vectors, concerns regarding recombinant vaccinia virus contamination in the rescued virus preparation are also eliminated. Finally, the instant methods and compositions obviate the need to develop special, helper cell lines, and any cell line that can be transfected may be used for recovery of a diverse array of non-segmented negative-stranded RNA virus.

The methods and compositions of the instant invention can be implemented to successfully recover a diverse array of recombinant, non-segmented negative-stranded RNA viruses from cDNA. Based on the revised reclassification in 1993 by the International Committee on the Taxonomy of Viruses, the Order of non-segmented negative-stranded RNA viruses designated *Mononegavirales* was established. This Order contains three families of enveloped viruses with single- stranded, non-segmented RNA genomes of minus polarity (negative-sense). These families of non-segmented, negative-stranded RNA viruses are the Paramyxoviridae, Rhabdoviridae and Filoviridae. The family Paramyxoviridae has been further divided into two subfamilies, Paramyxovirinae and Pneumovirinae. The subfamily Paramyxovirinae contains three genera, *Respirovirus* (formerly *Paramyxovirus*), *Rubulavirus* and *Morbillivirus*. The subfamily Pneumovirinae contains the genus *Pneumovirus*. Table 1 presents the current taxonomical classification of the *Mononegavirales,* along with nonlimiting examples of each genus representing of the species and genera of non-segmented negative-stranded RNA viruses amenable to rescue according to the methods and compositions of the invention.

### Table 1

### Classification of Non-segmented, Negative-sense, Single-stranded RNA Viruses of the Order Mononegavirales

### Family Paramyxoviridae

### Subfamily Paramyxovirinae

### Genus Respirovirus

Sendai virus (mouse parainfluenza virus type 1)
Human parainfluenza virus (PIV) types 1 and 3
Bovine parainfluenza virus (BPV) type 3

### Genus Rubulavirus

Simian virus 5 (SV) (Canine parainfluenza virus type 2)
Mumps virus
Newcastle disease virus (NDV) (avian Paramyxovirus 1)
Human parainfluenza virus types 2, 4a and 4b

### Genus Morbillivirus

Measles virus (MV)
Dolphin Morbillivirus
Canine distemper virus (CDV)
Peste-des-petits-ruminants virus
Phocine distemper virus
Rinderpest virus

### Unclassified

Hendra virus
Nipah virus

### Subfamily Pneumovirinae

### Genus Pneumovirus

Human respiratory syncytial virus (RSV)
Bovine respiratory syncytial virus
Pneumonia virus of mice

### Genus Metapneumovirus

Human metapneumovirus
Avian pneumovirus (formerly Turkey rhinotracheitis virus)

### Family Rhabdoviridae

### Genus Lyssavirus

Rabies virus

### Genus Vesiculovirus

Vesicular stomatitis virus

### Genus Ephemerovirus

Bovine ephemeral fever virus

### Family Filoviridae

### Genus Filovirus

Marburg virus

The current classification scheme for members of the *Mononegavirales* is based upon morphological criteria, organization of the viral genome, biological activities and the sequence relatedness of viral genes and gene products. The morphological distinguishing feature among enveloped viruses for the subfamily Paramyxovirinae is the size and shape of the nucleocapsids. Relevant biological characteristics are 1) antigenic cross-reactivity between members of a genus, and 2) the presence of neuraminidase activity in the genera *Respirovirus, Rubulavirus* and its absence in genus *Morbillivirus.* In addition, variations in the coding potential of the P gene are considered, as is the presence of an extra gene (SH) in Rubulaviruses.

Pneumoviruses can be distinguished from Paramyxovirinae morphologically because they contain narrow nucleocapsids. In addition, pneumoviruses have major differences in the number of protein-encoding cistrons (10 in pneumoviruses versus 6 in Paramyxovirinae) and an attachment protein (G) that is distinct from that of Paramyxovirinae. Although the paramyxoviruses and pneumoviruses have six proteins that appear to correspond in function (N, P, M, G/H/HN, F and L), only the latter two proteins exhibit significant sequence relatedness between the two subfamilies. Several pneumoviral proteins lack counterparts in most of the paramyxoviruses, namely the nonstructural proteins NS1 and NS2, the small hydrophobic protein SH, and a second protein M2. Some paramyxoviral proteins, namely C and V, lack counterparts in pneumoviruses. However, the basic genomic organization of pneumoviruses and paramyxoviruses is the same, as is also true of the rhabdoviruses and filoviruses.

General procedures for recovery of non-segmented negative-stranded RNA viruses according to the invention can be summarized as follows. A cloned DNA equivalent (which is positive-strand, message sense) of the desired viral genome is placed between a suitable DNA-dependent RNA polymerase promoter (e.g., a T7, T3 or SP6 RNA polymerase promoter) and a self-cleaving ribozyme sequence (e.g., the hepatitis delta ribozyme) which is inserted into a suitable transcription vector (e.g. a propagatable bacterial plasmid). This transcription vector provides the readily manipulable DNA template from which the RNA polymerase (e.g., T7 RNA polymerase) can faithfully transcribe a single-stranded RNA copy of the viral antigenome (or genome) with the precise, or nearly precise, 5' and 3' termini. The orientation of the viral DNA copy of the genome and the flanking promoter and ribozyme sequences determine whether antigenome or genome RNA equivalents are transcribed.

Also required for rescue of new virus progeny according to the invention are virus-specific trans-acting support proteins needed to encapsidate the naked, single-stranded viral antigenome or genome RNA transcripts into functional nucleocapsid templates. These generally include the viral nucleocapsid (N) protein, the polymerase-associated phosphoprotein (P) and the polymerase (L) protein. In different virus families or genera, the nomenclature for these proteins may vary slightly. For example, in the family Rhabdoviridae, the phosphoprotein can be called either "P" or "NS". In the Respirovirus and Rubulavirus genera, the nucleocapsid protein is called "NP". As used herein, the terms "N protein" and "N gene" refer to either the N or NP nucleocapsid proteins and genes, and the terms "P protein" and "P gene" refer to either the P or NS phosphoprotein proteins and genes, as appropriate. Certain viruses selected for rescue may require additional proteins. Typically, although not necessarily exclusively, rescue of non-segmented negative-stranded RNA viruses also requires an RNA polymerase to be expressed in host cells carrying the viral cDNA, to drive transcription of the cDNA-containing transcription vector and of the vectors encoding the support proteins.

Within the present invention, a novel rescue method for recovering non-segmented negative-stranded RNA viruses from cDNA involves introducing a viral cDNA expression vector encoding a genome or antigenome of the subject virus into a host cell, and coordinately introducing a transient polymerase expression vector encoding and directing transient expression of an RNA polymerase. Useful RNA polymerases in this context include, but are not limited to, a T7, T3, or SP6 phage polymerase. The host cells also express, before, during, or after coordinate introduction of the viral cDNA and transient polymerase expression vectors, the N, P, and L support proteins necessary for production of a replicating, infectious virus or subviral particle in the host cell.

Typically, the viral cDNA and transient polymerase expression vectors will be coordinately transfected into the host cell with one or more additional expression vector(s) that encode(s) and direct(s) expression of the support proteins. The support proteins may be wild-type or mutant proteins of the virus being rescued, or may be selected from corresponding support protein(s) of a heterologous non-segmented negative-stranded RNA virus. In alternate embodiments, additional viral proteins may be co-expressed in the host cell, for example a polymerase elongation factor (such as M2-1 for RSV) or other viral proteins that may enable or enhance recovery or provide other desired results within the subject methods and compositions. In other embodiments, one or more of the support protein(s) may be expressed in the host cell by constitutively expressing the protein(s) in the host cell, or by co-infection of the host cell with a helper virus encoding the support protein(s).

In more detailed aspects of the invention, the viral cDNA expression vector comprises a polynucleotide encoding a genome or antigenome of the non-segmented negative-stranded RNA virus operably linked with an expression control sequence to direct synthesis of viral RNA transcripts from the cDNA expression vector. The viral cDNA vector is introduced into a host cell transiently expressing an RNA polymerase, an N protein, a P protein, and an L protein. Each of the RNA polymerase and N, P, and L proteins may be expressed from one or more transiently transfected expression vector(s). Often, each of the RNA polymerase and N, P, and L proteins will be expressed from separate expression vectors, commonly from transient expression plasmids. Following a sufficient time and under suitable conditions, an assembled infectious, non-segmented, negative-stranded RNA virus is recovered from the host cells.

To produce infectious non-segmented negative-stranded RNA viruses from a cDNA-expressed genome or antigenome, the genome or antigenome is coexpressed with those viral proteins necessary to produce a nucleocapsid capable of RNA replication, and render progeny nucleocapsids competent for both RNA replication and transcription. Transcription by the genome nucleocapsid provides the other viral proteins and initiates a productive infection. Alternatively, additional viral proteins needed for a productive infection can be supplied by coexpression.

In certain embodiments of the invention, complementing sequences encoding proteins necessary to generate a transcribing, replicating viral nucleocapsid are provided by one or more helper viruses. Such helper viruses can be wild type or mutant. In certain embodiments, the helper virus can be distinguished phenotypically from the virus encoded by the recombinant viral cDNA. For example, it may be desirable to provide monoclonal antibodies that react immunologically with the helper virus but not the virus encoded by the recombinant viral cDNA. Such antibodies can be neutralizing antibodies. In some embodiments, the antibodies can be used in affinity chromatography to separate the helper virus from the recombinant virus. To aid the procurement of such antibodies, mutations can be introduced into the viral cDNA to provide antigenic diversity from the helper virus, such as in a glycoprotein gene.

A recombinant viral genome or antigenome may be constructed for use in the present invention by, e.g., assembling cloned cDNA segments, representing in aggregate the complete genome or antigenome, by polymerase chain reaction or the like (PCR; described in, e.g., U.S. Patent Nos. 4,683,195 and 4,683,202, and PCR Protocols: A Guide to Methods and Applications, Innis et al., eds., Academic Press, San Diego, 1990) of reverse-transcribed copies of viral mRNA or genome RNA. For example, a first construct may be generated which comprises cDNAs containing the left hand end of the antigenome, spanning from an appropriate promoter (e.g., T7, T3, or SP6 RNA polymerase promoter) and assembled in an appropriate expression vector (such as a plasmid, cosmid, phage, or DNA virus vector). The vector may be modified by mutagenesis and/or insertion of a synthetic polylinker containing unique restriction sites designed to facilitate assembly. The right hand end of the antigenome plasmid may contain additional sequences as desired, such as a flanking ribozyme and single or tandem T7 transcriptional terminators. The ribozyme can be hammerhead type, which would yield a 3' end containing a single nonviral nucleotide, or can be any of the other suitable ribozymes such as that of hepatitis delta virus (Perrotta et al., Nature 350:434-436, 1991) that would yield a 3' end free of non-viral nucleotides.

Alternative means to construct cDNA encoding the viral genome or antigenome include reverse transcription-PCR using improved PCR conditions (e.g., as described in Cheng et al., Proc. Natl. Acad. Sci. USA 91:5695-5699, 1994, incorporated herein by reference) to reduce the number of subunit cDNA components to as few as one or two pieces. In other embodiments different promoters can be used (e.g., T3 or SPQ). Different DNA vectors (e.g., cosmids) can be used for propagation to better accommodate the larger size genome or antigenome.

By "infectious clone" or "infectious cDNA" of a non-segmented negative-stranded RNA virus is meant cDNA or its product, synthetic or otherwise, as well as RNA capable of being directly incorporated into infectious virions which can be transcribed into genomic or antigenomic viral RNA capable of serving as a template to produce the genome of infectious viral or subviral particles. As noted above, defined mutations can be introduced into an infectious viral clone by a variety of conventional techniques (e.g., site-directed mutagenesis) into a cDNA copy of the genome or antigenome. The use of genomic or antigenomic cDNA subfragments to assemble a complete genome or antigenome cDNA as described herein has the advantage that each region can be manipulated separately, where small cDNA constructs provide for better ease of manipulation than large cDNA constructs, and then readily assembled into a complete cDNA.

Certain of the recombinant viruses of the invention will be constructed or modified to limit the growth potential, replication competence, or infectivity of the recombinant virus. Such growth- or replication-defective viruses and subviral particles are useful as vectors and immunogens, but do not pose certain risks that would otherwise attend administration of a fully infectious (i.e., having approximately a wild-type level of growth and/or replication competence) virus to a host. By replication-defective is meant a virus or subviral particle that is limited in its ability to grow or replicate in a host cell or a mammalian subject, or is otherwise defective in its ability to infect and/or propagate in or between cells. Often, the replication-defective virus is limited in its replication potential to only one or two rounds of replication in a host cell or subject. Often, growth- and replication-defective viruses and subviral particles will be employed as "vectors", as described in detail herein below.

Thus, various methods and compositions are provided for producing growth- and/or replication-defective, non-segmented, negative-stranded RNA viruses. In more detailed embodiments, the growth- or replication-defective virus will exhibit growth, replication and/or infectivity characteristics that are substantially impaired in comparison to growth, replication and/or infectivity of a corresponding wild-type or parental virus. In this context, growth, replication, and/or infectivity may be impaired in vitro and/or in vivo by at least approximately 10-20%, 20-50%, 50-75% and up to 95% or greater compared to wild-type or parental growth, replication and/or infectivity levels.

Viruses with varying degrees of growth or replication defect will typically be rescued using the combined heat shock/T7-plasmid rescue system described in detail below. Exemplary strains include highly attenuated strains of VSV that incorporate modifications as described below (e.g., a C-terminal G protein truncation, or translocated genes) (see, e.g., Johnson et al., J. Virol. 71:5060-5078, 1997, Schnell et al., Proc. Natl. Acad. Sci. USA 93:11359-11365, 1996; Schnell et al., Cell 90:849-857, 1997; Roberts et al., J. Virol. 72:4704-4711, 1998; and Rose et al., Cell 106:539-549, 2001, each incorporated herein by reference). Some of the subject viruses will contain defects that are only partial impairments, for example wherein viruses can still be propagated without genetic complementation. Such defects can be achieved in accordance with the description herein by introducing amino acid substitutions, nucleotide substitutions, partial or complete gene deletions or knock-outs, and the like in coding, noncoding, and regulatory sequences, for example to specify *ts* or *ca* phenotypes as described for RSV, PIV and VSV.

Often, the recombinant non-segmented negative-stranded RNA viruses will have a partial or complete gene deletion or gene "knock-out" that does not result in significant growth impairment when the virus is propagated in cell lines, but does substantially restrict replication in the natural host. Exemplary mutants in this context include RSV having a partial or complete deletion or knock-out of one or more of the SH, NS1, and NS2 genes or proteins. In other embodiments, a growth- or replication-defective virus is constructed by introduction of a mutation or other change that impairs gene function but does not block replication. For example, the foregoing references describe useful VSV candidates with C-terminal deletions affecting the G protein cytoplasmic tail. These and comparable growth- or replication-defective viruses are useful as "vectors", e.g., by incorporation of a heterologous antigenic determinant into a recombinant vector genome or antigenome. In specific examples, a MV or HIV glycoprotein, glycoprotein domain, or one or more antigenic determinant(s) is incorporated into a VSV vector or "backbone".

Additional growth- or replication-defective viruses will have base substitutions or small deletions that prevent expression of a protein that is not essential for growth in cell lines but affects pathogenicity in a mammalian host. Exemplary constructs in this regard include a HPIV having one or more of the C, D, and/or V ORFs ablated or deleted in whole or in part (see, e.g., Durbin et al., Virology 261:319-30, 1999, incorporated herein by reference), and a MV having all or part of the V protein deleted or knocked out Schneider et al., Virology 227:314-22, 1997, incorporated herein by reference), or mutated (see, e.g., United States Patent No. 6,664,066, issued to Parks on December 16, 2003, incorporated herein by reference).

Still additional growth- or replication-defective viruses will have partial or complete gene deletions or other types of modifications that ablate (knock-out) or reduce gene expression, wherein the defect partially impairs, but does not completely block replication in cell lines. Examples of useful candidates in this context include MV with a matrix protein deletion or knock out, Cathomen et al., EMBO J. 17:3899-908, 1998 (incorporated herein by reference), and RSV having one or more deletions or knock-outs of NS1, NS2, M2-2, and/or SH (Jin et al., Virology 273:210-8, 2000, incorporated herein by reference.

In other embodiments, growth- or replication-defective viruses have rearranged or positionally "shuffled" genes that result in impaired growth but do not block replication. Various such positionally-shifted RSV mutants are described in the references incorporated herein. Other exemplary recombinants in this class are described for VSV (see, e.g., United States Patent No. 6,596,529, issued to Wertz et al. on July 22, 2003; United States Patent No. 6,136,585, issued to Ball et al. on October 24, 2000; and Wertz et al., Proc. Natl. Acad. Sci. USA 95:3501-6, 1998, each incorporated herein by reference).

Within other embodiments, growth- or replication-defective viruses will have a partial or complete gene substitution that impairs growth. In this regard, one or more viral genes can be substituted with genes from a related virus that infects another species, as exemplified by various host-range restricted human-bovine PIVs (HBPIV) expressing HPIV or RSV antigenic determinants (Skiadopoulos et al., J. Virol. 77:1141-8, 2003; Bailly et al., J. Virol. 74:3188-95, 2000, Schmidt et al., J. Virol. 75:4594-603, 2001, each incorporated herein by reference), and by RSVs having their F and/or G genes substituted by the F and/or G genes of another RSV, or of VSV (see, e.g., Oomens et al., J. Virol. 77:3785-98, 2003, incorporated herein by reference).

In other detailed embodiments of the invention, growth- or replication-defective viruses are constructed with defects that must be complemented by complementing cell lines, helper virus, transfection or some other means of providing the lost function. Exemplary constructs include VSV wherein expression of the G protein is reduced or ablated. The lost G protein expression is provided by transfection or some type of helper-virus system ('pseudotyping') (see, e.g., Lefkowitz et al., Virology 178:373-83, 1990, incorporated herein by reference). Alternatively, the lost G function can be provided by a complementing cell line (see, e.g., Schnell et al., Cell 90:849-57, 1997, incorporated herein by reference) or with a G protein fusion (e.g., wherein the VSV G protein includes the membrane spanning sequence and cytoplasmic domain of VSV fused to a heterologous sequence such as from the measles F or H genes) (see, e.g., Schnell et al., Proc. Natl. Acad. Sci. USA 93:11359-11365, 1996; Schnell et al., Cell 90:849-857, 1997; Roberts et al., J. Virol. 72:4704-4711, 1998; and Rose et al., Cell 106:539-549, 2001, each incorporated herein by reference). In one exemplary embodiment described herein, the recombinant VSV with compromised G function is recovered through a second step of co-culturing viral transfected cells with cells transfected with a plasmid encoding a functional G protein. In other exemplary candidates, the VSV G protein is replaced with a hybrid G protein that creates a receptor that targets the virus to specific cells, such as CD4+ cells (Johnson et al., J. Virol. 71:5060-5078, 1997, incorporated herein by reference).

Additional viruses that are partially growth- or replication-defective are constructed by modification of a leader or trailer region, for example wherein a trailer region is replaced with a copy of a weaker promoter found in a leader sequence (Finke et al., J. Virol. 73:3818-25, 1999, incorporated herein by reference).

For ease of preparation the N, P, L and other desired viral proteins can be assembled in one or more separate vectors. Many suitable expression vectors are known in the art which are useful for incorporating and directing expression of polynucleotides encoding the RNA polymerase and support proteins, including for example plasmid, cosmid, or phage vectors, defective viral vectors, so-called "replicons" (e.g. sindbis or Venezuelan equine encephalitis replicons) and other vectors useful for directing transient and/or constitutive expression. Transient expression of the RNA polymerase and, where applicable, the N, P, and L proteins, is directed by a transient expression control element operably integrated with the polymerase and/or support vector(s). In one exemplary embodiment, the transient expression control element for the RNA polymerase is an RNA polymerase II regulatory region, as exemplified by the immediate-early human cytomegalovirus [hCMV] promoter and enhancer (see, e.g., U.S. Patent 5,168,062, incorporated herein by reference).

The vectors encoding the viral cDNA, the transiently-expressed RNA polymerase, and the N, P, and L proteins may be introduced into appropriate host cells by any of a variety of methods known in the art, including transfection, electroporation, mechanical insertion, transduction or the like. In certain exemplary embodiments, the subject vectors are introduced into cultured cells by calcium phosphate-mediated transfection (Wigler et al., Cell 14:725, 1978; Corsaro et al., Somatic Cell Genetics 7:603, 1981; Graham et al., Virology 52:456, 1973, electroporation (Neumann et al., EMBO J. 1:841-845, 1982), DEAE-dextran mediated transfection (Ausubel et al., (ed.) Current Protocols in Molecular Biology, John Wiley and Sons, Inc., NY, 1987), or cationic lipid-mediated transfection (Hawley-Nelson et al., Focus 15:73-79, 1993). In alternate embodiments, a transfection facilitating reagent is added to increase DNA uptake by cells. Many of these reagents are known in the art. LIPOFECTACE® (Life Technologies, Gaithersburg, MD) and EFFECTENE® (Qiagen, Valencia, CA) are common examples. LIPOFECTACE® and EFFECTINE® are both. These reagents are cationic lipids that coat DNA and enhance DNA uptake by cells. LIPOFECTACE® forms a liposome that surrounds the DNA while EFFECTINE® coats the DNA but does not form a liposome. Another useful commercial reagent to facilitate DNA uptake is LIPOFECTAMINE-2000® (Invitrogen, Carlsbad, CA).

Suitable host cells for use within the invention are capable of supporting a productive infection of the subject non-segmented negative-stranded RNA virus, and will permit expression of the requisite vectors and their encoded products necessary to support viral production. Such host cells can be selected from a prokaryotic cell or a eukaryotic cell. Suitable cells include insect cells such as Sf9 and Sf21, bacterial cells with an appropriate promoter such as *E*. *coli*, and yeast cells such as S. cerevisiae. Host cells are typically chosen from vertebrate, e.g., primate, cells. Since many of the RNA viruses employed in this invention are human pathogens, a primate cell will often be employed in such instances. For example, measles virus (MV) is primarily restricted to primate cell types. There are exceptions such as canine distemper virus and other morbilliviruses that infect non-human mammals. Some eukaryotic cell lines work better than others for propagating viruses and some cell lines do not work at all for some viruses. Typically, a cell line is employed that yields a detectable cytopathic effect in order that rescue of viable virus may be easily detected. Often, the host cells are derived from a human cell, such as a human embryonic kidney (HEK) cell. Vero cells (African green monkey kidney cells), as well as many other types of cells can also used as host cells. In the case of measles and potentially other viruses, the transfected cells are grown on Vero cells because the virus spreads rapidly on Vero cells and makes easily detectable plaques. The following are examples of other suitable host cells: (1) Human Diploid Primary Cell Lines: e.g. WI-38 and MRC-5 cells; (2) Monkey Diploid Cell Line: e.g. Cos, Fetal Rhesus Lung (FRhL) cells; (3) Quasi-Primary Continuous Cell Line: e.g. AGMK -african green monkey kidney cells.; (4) Human 293 cells (qualified) and (5) rodent (e.g., CHO, BHK), canine e.g., Madin-Darby Canine Kidney (MDCK), and primary chick embryo fibroblasts. Exemplary specific cell lines that are useful within the methods and compositions of the invention include HEp-2, HeLa, HEK (e.g., HEK 293), BHK, FRhL-DBS2, LLC-MK2, MRC-5, and Vero cells.

Within the methods and compositions provided herein, coordinate introduction of the RNA polymerase vector, viral cDNA clone, and support vector(s) (e.g., plasmid(s) encoding N, P, and L) into a host cell will be simultaneous. For example, all of the subject DNAs may be combined in a single DNA transfection (e.g., calcium-phosphate transfection) mixture and added to a host cell culture simultaneously to achieve coordinate transfection. In alternate embodiments separate transfections may be performed for any two or more of the subject polymerase and support vectors and the viral cDNA vector. Typically, separate transfections will be conducted in close temporal sequence to coordinately introduce the polymerase and support vectors and viral cDNA vector in an effective cotransfection procedure. In one such coordinate transfection protocol, the viral cDNA and/or N, P, and L support plasmid(s) is/are introduced into the host cell prior to transfection of the RNA polymerase plasmid. In other embodiments, the viral cDNA and/or the N, P, L support plasmid(s) is/are introduced into the host cell simultaneous with or following transfection of the RNA polymerase plasmid into the cell, but before substantial expression of the RNA polymerase begins (e.g., before detectable levels of a T7 polymerase have accumulated, or before levels of T7 sufficient to activate expression of plasmids driven by a T7 promoter have accumulated) in the host cell.

Within other detailed aspects of the invention, the method for producing the infectious, non-segmented, negative-stranded RNA virus involves an additional heat shock treatment of the host cell to increase recovery of the recombinant virus. After one or more of the viral cDNA expression vector and the one or more transient expression vectors encoding the RNA polymerase, N protein, P protein, and L protein are introduced into the host cell, the host cell is exposed to an effective heat shock stimulus that increases recovery of the recombinant virus.

In one such method, the host cell is exposed to an effective heat shock temperature for a time period sufficient to effectuate heat shock of the cells, which in turn stimulates enhanced viral recovery. An effective heat shock temperature is a temperature above the accepted, recommended or optimal temperature considered in the art for performing rescue of the subject virus. In many instances, an effective heat shock temperature is above 37°C. When a modified rescue method of the invention is carried out at an effective heat shock temperature, there results an increase in recovery of the desired recombinant virus over the level of recovery of recombinant virus when rescue is performed in the absence of the increase in temperature. The effective heat shock temperature and exposure time may vary based upon the rescue system used. Such temperature and time variances can result from differences in the virus selected or host cell type.

Although the temperature may vary, an effective heat shock temperature can be readily ascertained by conducting several test rescue procedures with a particular recombinant virus, and establishing a rate percentage of recovery of the desired recombinant virus as temperature and time of exposure are varied. Certainly, the upper end of any temperature range for performing rescue is the temperature at which the components of the transfection are destroyed or their ability to function in the transfection is depleted or diminished. Exemplary effective heat shock temperature ranges for use within this aspect of the invention are: from about 37°C to about 50°C, from about 38°C to about 50°C, from about 39°C to about 49°C, from about 39°C to about 48°C, from about 40°C to about 47°C, from about 41°C to about 47°C, from about 41°C to about 46°C. Often, the selected effective heat shock temperature range will be from about 42°C to about 46°C. In more specific embodiments, effective heat shock temperatures of about 43°C, 44°C, 45°C or 46°C are employed.

In conducting the tests to establish a selected effective heat shock temperature or temperature range, one can also select an effective time period for conducting the heat shock procedure. A sufficient time for applying the effective heat shock temperature is a time over which there is a detectable increase in recovery of the desired recombinant virus over the level of recovery of recombinant virus when rescue is performed in the absence of an increase in temperature as noted above. The effective heat shock period may vary based upon the rescue system, including the selected virus and host cell. Although the time may vary, the amount of time for applying an effective heat shock temperature can be readily ascertained by conducting several test rescue procedures with a particular recombinant virus, and establishing a rate or percentage of recovery of the desired recombinant virus as temperature and time are varied. The upper limit for any time variable used in performing rescue is the amount of time at which the components of the transfection are destroyed or their ability to function in the transfection is depleted or diminished. The amount of time for the heat shock procedure may vary from several minutes to several hours, as long as the desired increase in recovery of recombinant virus is obtained. Exemplary effective heat shock periods for use within this aspect of the invention, in minutes, are: from about 5 to about 300 minutes, from about 5 to about 200 minutes, from about 15 to about 300, from about 15 to about 240, from about 20 to about 200, from about 20 to about 150. Often, the effective heat shock period will be from about 30 minutes to about 150 minutes.

Numerous means can be employed to determine the level of improved recovery of a recombinant non-segmented negative-stranded RNA virus through exposure of host cells to effective heat shock. For example, a chloramphenicol acetyl transferase (CAT) reporter gene can be used to monitor rescue of the recombinant virus according to known methods. The corresponding activity of the reporter gene establishes the baseline and improved level of expression of the recombinant virus. Other methods include detecting the number of plaques of recombinant virus obtained and verifying production of the rescued virus by sequencing. One exemplary method for determining improved recovery involves preparing a number of identically transfected cell cultures and exposing them to different conditions of heat shock (time and temperature variable), and then comparing recovery values for these cultures to corresponding values for control cells (e.g., cells transfected and maintained at a constant temperature of 37°C). After 72 hours post-transfection, the transfected cells are transferred to a 10cm plate containing a monolayer of about 75% confluent Vero cells (or cell type of choice for determining plaque formation of the recombinant virus) and continuing incubation until plaques are visible. Thereafter, the plaques are counted and compared with the values obtained from control cells. Optimal heat shock conditions should maximize the number of plaques.

According to these embodiments of the invention, improved viral recovery will be at least about 10% or 25%, and often at least about 40%. In certain embodiments, the increase in the recombinant virus recovered attributed to effective heat shock exposure is reflected by a 2-fold, 5-fold, and up to 10-fold or greater increase in the amount of recombinant virus observed or recovered.

In another embodiment of the invention the host cell in which the viral cDNA and transient RNA polymerase vectors (and optionally one or more vector(s) encoding the necessary support proteins) have been introduced is subjected to a "plaque expansion" step. This procedure is typically conducted after a period of time (e.g., post-transfection) sufficient to permit expression of the viral cDNA expression vector and one or more transient expression vectors that encode(s) and direct(s) transient expression of the RNA polymerase, N protein, P protein, and L proteins. To achieve plaque expansion, the host cell, which often has become impaired in its ability to support further viral expansion, is co-cultured with a plaque expansion cell of the same or different cell type. This co-culture step allows spread of rescued virus to the plaque expansion cell, which is more amenable to vigorous expansion of the virus. Typically, a culture of host cells is transferred onto one or more layer(s) of plaque expansion cells. For example, a culture of host cells can be spread onto a monolayer of plaque expansion cells and the recombinant non-segmented negative-stranded RNA virus will thereafter infect the plaque expansion cells and expand further therein. In alternate embodiments, the host cell is of the same, or different, cell type as the plaque expansion cell.

The modified, plaque expansion methods and compositions of the invention provide improved rescue methods for producing a recombinant Mononegavirales virus. Typically, the method entails, introducing into a host cell a transcription vector comprising an isolated nucleic acid molecule encoding a genome or antigenome of a nonsegmented, negative-sense, single stranded RNA virus of the Order Mononegavirales and a transient expression vector encoding and directing transient expression an RNA polymerase, along with at least one expression vector which comprises at least one isolated nucleic acid molecule encoding the trans-acting proteins necessary for encapsidation, transcription and replication, under conditions sufficient to permit co-expression of said vectors and production of the recombinant virus. Thereafter the host cell is transferred onto at least one layer of plaque expansion cells.

In order to achieve plaque expansion, the transfected cells are typically transferred to co-culture containers of plaque expansion cells. Any of the various plates or containers known in the art can be employed for the plaque expansion step. In certain embodiments, the transfected cells are transferred onto a monolayer of plaque expansion cells that is at least about 50% confluent. Alternatively, the plaque expansion cells are at least about 60% confluent, or even at least about 75% confluent. In certain embodiments, the surface area of the plaque expansion cells is greater than the surface area used for preparing the transfected virus. An enhanced surface area ratio of from 2:1 to 100:1 can be employed as desired. An enhanced surface area of at least 10:1 is often desired.

The plaque expansion cells are selected based on the successful growth of the native or recombinant virus in such cells. Often, the host cell employed in conducting the transfection is not an optimal host for growth of the desired recombinant virus. The recovery of recombinant virus from the transfected cells can therefore be enhanced by selecting a plaque expansion cell in which the native virus or the recombinant virus exhibits enhanced growth. Various plaque expansion cells can be selected for use within this aspect of the invention, in accordance with the foregoing description. Exemplary specific plaque expansion cells that can be used to support recovery and expansion of recombinant non-segmented negative-stranded RNA viruses of the invention are selected from HEp-2, HeLa, HEK, BHK, FRhL-DBS2, LLC-MK2, MRC-5, and Vero cells. Additional details concerning heat shock and plaque expansion methods for use within the invention are provided in PCT publication WO 99/63064, incorporated herein by reference.

Also provided within the invention are compositions for producing an infectious, non-segmented, negative-stranded RNA virus of the Order *Mononegavirales*. The compositions include a viral cDNA expression vector comprising a polynucleotide encoding a genome or antigenome of a non-segmented negative-stranded RNA virus operably linked with an expression control sequence to direct synthesis of viral RNA transcripts from the cDNA, and one or more transient expression vectors that encode(s) and direct(s) transient expression in the host cell of an RNA polymerase, an N protein, a P protein, and an L protein. Within this aspect of the invention, the composition can comprise the viral cDNA expression vector and the one or more transient expression vector(s) that encode(s) and direct(s) transient expression of the RNA polymerase, N, P, and L proteins in a cell-free co-transfection mixture. Alternatively, the viral cDNA expression vector and the one or more transient expression vector(s) encoding and directing transient expression of said RNA polymerase, N, P, and L proteins can be provided within a host cell (e.g., following transfection of the subject vectors into the host cell). In certain embodiments, the RNA polymerase is selected from a T7, T3, or SP6 RNA polymerase. In other embodiments, the transient expression vector encoding the RNA polymerase is a plasmid. In yet additional embodiments, the foregoing compositions further include a plaque expansion cell of the same or different cell type as the host cell that allow spread of rescued virus from the host cell to the plaque expansion cell and facilitates expansion of recombinant virus.

In one exemplary embodiment of the invention described below, a transient T7 RNA polymerase expression vector (pSC6-T7, provided by Dr. Martin Billeter, Univ. of Zurich) was cotransfected into Vero cells with a full-length measles virus (MV+) cDNA clone, along with plasmids encoding MV N, P, and L proteins operably linked with an expression control sequence (e.g., a T7 RNA polymerase promoter), yielding successful recovery of MV.

In other exemplary embodiments, the methods and compositions of the invention were employed for successful recovery of 40 different recombinant constructs of respiratory syncytial virus (RSV), including RSV Type A and Type B, chimeric AB RSVs, and recombinant RSVs incorporating one or more attenuating mutations and/or other recombinant modifications discussed below (e.g., gene deletions). Additional examples are provided demonstrating successful recovery of, e.g., human parainfluenza virus type 3 (HPIV3), chimeric human parainfluenza virus 3-1 (where glycoprotein genes F and HN of PIV-3 have been replaced by glycoprotein genes F and HN of human parainfluenza virus type 1), chimeric human parainfluenza virus 3-2CT (where the cytoplasmic tail portions of glycoprotein genes F and HN of PIV-3 have been replaced by glycoprotein genes F and HN of human parainfluenza virus type 2), a bovine PIV type 3 (BPIV3), chimeric human-bovine PIV-3 (where glycoprotein genes F and HN of bovine parainfluenza virus type 3 have been replaced by glycoprotein genes F and HN of human PIV-3), and vesicular stomatitis virus (VSV).

Within other detailed embodiments of the invention, a transient T7 RNA polymerase expression plasmid was constructed based on the pCl-Neo backbone (Promega, Madison WI). This vector was modified, before insertion of the T7 RNA polymerase gene, by removing the T7 promoter located at the 5' side of the multiple cloning site to generate pCl-Neo-Bcl. The T7 promoter was removed to increase the stability of the T7 RNA polymerase gene insert during bacterial propagation. Over-expression of T7 RNA polymerase can be toxic to bacteria, and this could present problems if the T7 RNA polymerase gene was cloned downstream of a T7 RNA polymerase promoter.

A T7 gene used for cloning was PCR-amplified and cloned using *Eco*RI (5') and *Xba*I (3') sites that were incorporated into the PCR primers. A Kozak consensus sequence was also included 5' of the ATG translation initiation codon to ensure high-efficiency protein synthesis. Insertion of the T7 gene into pCl-Neo-Bcl produced pCL-Neo-Bcl-T7, which resulted in the T7 gene being placed under control of the hCMV promoter and enhancer. The resulting plasmid pCl-Neo-Bcl-T7 has the sequence set forth in Figure 2 (SEQ ID NO: 1), and contains the HCMV promoter/enhancer, followed in order by an intron, T7 RNA polymerase SV40 polyA, SV40 promoter/enhancer and Neo^{R} elements. An optional selection marker, represented by the neomycin resistance gene, controlled by the SV40 promoter/enhancer, was located 3' of the T7 gene. The neomycin marker adds versatility to this plasmid, since it can be used to select stably transformed cell lines. It is also noteworthy that the position of the selectable marker gene places the SV40 enhancer at the 3' end of the T7 RNA polymerase gene. The well-documented bidirectional transcriptional stimulatory effect of the SV40 enhancer may significantly contribute to overall expression of T7 RNA polymerase in plasmid pCl-Neo-Bcl-T7.

The methods of the invention can be carried out in conjunction with various recombinant modifications of the rescued viruses, each of which is well-known in the art. For example, the viral genome or antigenome can be modified by deletion of an entire gene, deletion of a portion of a gene, point mutation(s) in coding region, replacement of part or all of a gene by a heterologous nucleic acid sequence (from another virus, another pathogen, encoding a pharmaceutically useful polypeptide, or encoding an immune modulator), insertion of a heterologous nucleic acid sequence, for example, in an intergenic region, and rearrangement of gene order, among other modifications. Because the rescue systems provided herein are sufficiently robust and efficient, recovery of such growth-impaired and/or attenuated recombinant or mutant derivatives can be successfully implemented.

Thus, infectious, recombinant, non-segmented negative-stranded RNA viruses according to the invention are produced by a novel coexpression system that permits introduction of defined changes into the recombinant virus and provides for the generation, with high frequency and fidelity, of viral candidates for use in immunogenic compositions having a defined genome sequence. These modifications are useful in a wide variety of applications, including the development of live attenuated viral strains bearing predetermined, defined attenuating mutations. Infectious non-segmented negative-stranded RNA viruses of the invention are produced by intracellular coexpression of one or more isolated polynucleotide molecules that encode the viral genome or antigenome RNA, together with one or more polynucleotides encoding the viral proteins desired, or at least necessary, to generate a transcribing, replicating nucleocapsid.

cDNAs encoding a non-segmented negative-stranded RNA virus genome or antigenome are constructed for intracellular coexpression with the selected viral proteins to form an infectious virus. By "non-segmented negative-stranded RNA virus antigenome" is meant an isolated positive-sense polynucleotide molecule which serves as a template for synthesis of progeny viral genome. Typically a cDNA is constructed which is a positive-sense version of the viral genome corresponding to the replicative intermediate RNA, or antigenome, so as to minimize the possibility of hybridizing with positive-sense transcripts of complementing sequences encoding proteins necessary to generate a transcribing, replicating nucleocapsid. Nothwithstanding the foregoing, a negative-sense version of the viral genome may be used (see, e.g., Kato et al., Genes to Cells 1:569-579, 1996, incorporated herein by reference)

In some embodiments of the invention the genome or antigenome of a recombinant non-segmented negative-stranded RNA virus need only contain those genes or portions thereof necessary to render the viral or subviral particles encoded thereby infectious. Further, the genes or portions thereof may be provided by more than one polynucleotide molecule, i.e., a gene may be provided by complementation or the like from a separate nucleotide molecule. In other embodiments, the viral genome or antigenome encodes all functions necessary for viral growth, replication, and infection without the participation of a helper virus or viral function provided by a plasmid or helper cell line.

By "recombinant non-segmented negative-stranded RNA virus" is meant a complete virus or an infectious subviral particle derived directly or indirectly from a recombinant expression system or propagated from virus or subviral particles produced therefrom. The recombinant expression system will employ a recombinant viral expression vector which comprises an operably linked transcriptional unit comprising an assembly of at least a genetic element or elements having a regulatory role in viral gene expression, for example, a promoter, a structural or coding sequence which is transcribed into viral RNA, and appropriate transcription initiation and termination sequences.

By providing methods for efficient rescue of infectious clones of non-segmented negative-stranded RNA viruses, the invention permits a wide range of alterations to be recombinantly produced within the genome (or antigenome) of diverse viral subjects, yielding defined mutations that specify desired phenotypic changes. The compositions and methods of the invention for producing recombinant non-segmented negative-stranded RNA viruses permit ready detailed analysis and manipulation of viral molecular biology and pathogenic mechanisms using, e.g., defmed mutations to alter the function or expression of selected viral proteins. Related diagnostic uses will be readily appreciated by those skilled in the art. Using the methods and compositions of the invention, one can readily distinguish mutations responsible for desired phenotypic changes from silent incidental mutations, and select phenotype-specific mutations for incorporation into a recombinant non-segmented negative-stranded RNA viral genome or antigenome.

In this context, a variety of nucleotide insertions, deletions, substitutions, and rearrangements can be made in the recombinant viral genome or antigenome during or after construction of the cDNA. For example, specific desired nucleotide sequences can be synthesized and inserted at appropriate regions in the cDNA using convenient restriction enzyme sites. Alternatively, such techniques as site-specific mutagenesis, alanine scanning, PCR mutagenesis, or other such techniques well known in the art can be used to introduce mutations into the cDNA.

Recombinant modifications of non-segmented negative-stranded RNA viruses provided within the invention are directed toward the production of candidate viruses for use in diagnostic and immunogenic compositions, e.g., to enhance viral attenuation and immunogenicity, to ablate epitopes associated with undesirable immunopathology, to accommodate antigenic drift, etc. To achieve these and other objectives, the compositions and methods of the invention allow for a wide variety of modifications to be introduced into a non-segmented negative-stranded RNA viral genome or antigenome for incorporation into an infectious, recombinant virus. For example, foreign genes or gene segments encoding antigenic determinants (e.g., protective antigens or immunogenic epitopes) may be added within a viral clone to generate recombinant viruses capable of inducing immunity to the parental or "background" virus and another "donor" virus or pathogenic agent from which the antigenic determinant(s) was/were derived. Alternatively, foreign genes may be inserted, in whole or in part, encoding modulators of the immune system, such as cytokines, to enhance immunogenicity of a candidate virus. Other mutations that may be included within viral clones rescued according to the methods of the invention, for example, substitution of heterologous genes or gene segments (e.g., a gene segment encoding a cytoplasmic tail of a glycoprotein gene) with a counterpart gene or gene segment in a recombinant viral clone. Alternatively, the relative order of genes within a viral clone can be changed, a viral genome promoter or other regulatory element can be replaced with its antigenome counterpart, or selected viral gene(s) rendered non-functional (e.g., by functional ablation involving introduction of a stop codon to prevent expression of the gene). Other modifications in a non-segmented negative-stranded RNA virus can be made to facilitate manipulations, such as the insertion of unique restriction sites in various non-coding or coding regions of the viral genome or antigenome. In addition, nontranslated gene sequences can be removed to increase capacity for inserting foreign sequences.

As noted above, it is often desirable to adjust the phenotype of recombinant non-segmented negative-stranded RNA viruses by introducing additional mutations that increase or decrease attenuation or otherwise alter the phenotype of the recombinant virus. Useful materials and methods for producing recombinant non-segmented negative-stranded RNA viruses from cDNA, and for selecting, introducing, and testing various attenuating mutations and other modifications within the present invention have been exemplified in detail for parainfluenza viruses, and are generally applicable to PIVs and other non-segmented negative-stranded RNA viruses within the methods and compositions of the invention (see, e.g., Durbin et al., Virology 235:323-332, 1997; U.S. Patent Application Serial No. 09/083,793, filed May 22, 1998; U.S. Patent Application Serial No. 09/458,813, filed December 10, 1999; U.S. Patent Application Serial No. 09/459,062, filed December 10, 1999; U.S. Patent Application Serial No. 09/083,793, filed May 22, 1998 (corresponding to International Publication No. WO 98/53078) and its priority U.S. Provisional Applications Nos. 60/047,575, filed May 23, 1997 and 60/059,385, filed September 19, 1997, each incorporated herein by reference). In particular, the foregoing incorporated references describe methods for producing infectious recombinant HPIV3 by construction and expression of cDNA encoding a PIV genome or antigenome coexpressed with essential PIV proteins, including descriptions of exemplary plasmids that may be employed to produce infectious viral clones. Methods for producing infectious recombinant HPIV1 by construction and expression of cDNA encoding a HPIV1 recombinant or chimeric genome or antigenome coexpressed with essential PIV proteins are similarly described in U.S. Provisional Application No. 60/331,961, filed November 21, 2001, and in Newman et al., Virus Genes 24:77-92, 2002 (each incorporated herein by reference). Methods for producing infectious recombinant HPIV2 by construction and expression of cDNA encoding a HPIV2 recombinant or chimeric genome or antigenome coexpressed with essential PIV proteins are similarly described in U.S. Provisional Application No. 60/412,053, filed September 18, 2002 (incorporated herein by reference).

Similarly, exemplary methods and materials for cloning of respiratory syncytial viruses (RSVs) from cDNA and other disclosure pertaining to selection, introduction, and testing of attenuating mutations and other modifications for use in constructing recombinant non-segmented negative-stranded RNA viruses within the present invention have been described (see, e.g., U.S. Patent Application No. 08/720,132, filed September 27, 1996, corresponding to International Publication WO 97/12032 published April 04/03/97, and priority U.S. Provisional Patent Application No. 60/007,083, filed September 27, 1995; U.S. Provisional Patent Application No. 60/021,773, filed July 15, 1996; U.S. Provisional Patent Application No. 60/046,141, filed May 9, 1997; U.S. Provisional Patent Application No. 60/047,634, filed May 23, 1997; U.S. Patent Application No. 08/892,403, filed July 15, 1997 corresponding to International Publication No. WO 98/02530 published on January 22, 1998; U.S. Patent Application No. 09/291,894, filed on April 13, 1999 corresponding to International Publication No. WO 00/61611 published October 19, 2000, and priority U.S. Provisional Patent Application Serial Nos. 60/129,006, filed on April 13, 1999; U.S. Patent Application No. 09/602,212, filed June 23, 2000 and corresponding International Publication No. WO 01/04335 published on January 18,2001, and priority U.S. Provisional Patent Application No.s 60/129,006, filed April 13, 1999, 60/143,097, filed July 9, 1999, and 60/143,132, filed July 9, 1999; International Publication No. WO 00/61737 published on October 19,2000; Collins et al., Proc Nat. Acid. Sci. U.S.A. 92:11563-11567, 1995; Bukreyev et al., J. Virol. 70:6634-41, 1996, Juhasz et al., J. Virol. 71:5814-5819, 1997; Durbin et al., Virology 235:323-332, 1997; He et al. Virology 237:249-260, 1997; Baron et al. J. Virol. 71:1265-1271, 1997; Whitehead et al., Virology 247:232-9, 1998a; Whitehead et al., J. Virol. 72:4467-4471, 1998b; Jin et al. Virology 251:206-214, 1998; and Whitehead et al., J. Virol. 73:3438-3442, 1999, and Bukreyev et al., Proc. Nat. Acad. Sci. U.S.A. 96:2367-72, 1999, each incorporated herein by reference in its entirety for all purposes).

These above-incorporated references describe methods and procedures for mutagenizing, isolating and characterizing non-segmented negative-stranded RNA viruses to obtain attenuated mutant strains (e.g., temperature sensitive (*ts*), cold passaged (*cp*) cold-adapted (*ca*), small plaque (*sp*) and host-range restricted (*hr*) mutant strains) and for identifying the genetic changes that specify the attenuated phenotype. In conjunction with these methods, the foregoing incorporated references detail procedures for determining replication, immunogenicity, genetic stability and immunogenic efficacy of biologically derived and recombinantly produced attenuated non-segmented negative-stranded RNA viruses in accepted model systems reasonably correlative of human activity, including hamster or rodent and non-human primate model systems. In addition, these references describe general methods for developing and testing immunogenic compositions, including monovalent and bivalent immunogenic compositions against non-segmented negative-stranded RNA viruses.

Also disclosed in the above-incorporated references are methods for constructing and evaluating infectious recombinant non-segmented negative-stranded RNA viruses that are modified to incorporate phenotype-specific mutations identified in biologically derived mutants, e.g., cold passaged (cp), cold adapted (ca), host range restricted (hr), small plaque (sp), and/or temperature sensitive (ts) mutants, for example the HPIV3 JS cp45 mutant strain (deposited under the terms of the Budapest Treaty with the American Type Culture Collection (ATCC) of 10801 University Boulevard, Manassas, Virginia 20110-2209, U.S.A. under Patent Deposit Designation PTA-2419; deposit incorporated herein by reference). Mutations identified in this and other mutant viruses (e.g., RSV-see above references) can be readily incorporated into recombinant PIVs or other non-segmented negative-stranded RNA viruses of the instant invention, as described in further detail below.

Thus, attenuating mutations and other modifications can be routinely introduced into recombinant non-segmented negative-stranded RNA viruses of the invention. In certain aspects of the invention, a mutation from a biologically derived mutant virus is identified, evaluated and introduced into a recombinant strain of the same or different virus. Attenuating mutations in biologically derived non-segmented negative-stranded RNA viruses for incorporation within recombinant viruses of the invention may occur naturally or may be introduced into wild-type strains and thereafter identified and characterized by well known mutagenesis and analytic procedures. For example, incompletely attenuated parental viral mutant strains can be produced by chemical mutagenesis during virus growth in cell cultures to which a chemical mutagen has been added, by selection of virus that has been subjected to passage at suboptimal temperatures in order to introduce growth restriction mutations, or by selection of a mutagenized virus that produces small plaques (sp) in cell culture, as described in the above incorporated references.

By "biologically derived" is meant any virus not produced by recombinant means. Thus, biologically derived viruses include naturally occurring viruses having a wild-type genomic sequence and viruses having allelic or mutant genomic variations from a reference wild-type sequence, e.g., biologically derived viruses having a mutation specifying an attenuated phenotype. Likewise, biologically derived viruses include mutants derived from a parental strain by, inter alia, artificial mutagenesis and selection procedures not involving direct recombinant DNA manipulation.

Mutations identified in biologically derived mutant non-segmented negative-stranded RNA viruses are introduced as desired, singly or in combination, to adjust a recombinant virus of the invention to an appropriate level of attenuation, immunogenicity, genetic resistance to reversion from an attenuated phenotype, etc. In accordance with the foregoing description, the ability to efficiently and produce infectious recombinant non-segmented negative-stranded RNA viruses from cDNA permits introduction of specific engineered changes within a broad assemblage of recombinant viruses. These, infectious, recombinant viruses can be employed for further identification of specific mutation(s) in biologically derived, attenuated strains, for example mutations that specify *ts*, *ca, att* and other phenotypes. Desired mutations identified by this and other methods are introduced into other recombinant viral strains. The unique capabilities for producing new non-segmented negative-stranded RNA viruses from cDNA provided herein allows for routine incorporation of these mutations, individually or in various selected combinations, into novel viral cDNA clones, whereafter the phenotypes of rescued recombinant viruses containing the introduced mutations to be readily determined.

In other embodiments of the invention, desired mutations identified in a heterologous negative stranded RNA virus are "transferred" by mapping the mutation in the heterologous mutant virus, identifying by routine sequence alignment the corresponding site in the recipient, recombinant virus, and mutating the native sequence in the recipient virus to an identical or conservative mutation as observed in the heterologous mutant genotype. This general rational design method for transferring mutations is described in International Application No. PCT/US00/09695, filed April 12, 2000, published as WO 00/61737 on 10/19/00 corresponding to U.S. National Phase application 09/958,292, filed on 01/08/02, and claiming priority to U.S. Provisional Patent Application Serial No. 60/129,006, filed on April 13, 1999, each incorporated herein by reference. Additional description pertaining to this aspect of the invention is provided in Newman et al., Virus Genes 24:77-92, 2002; Feller et al., Virology 10;276:190-201, 2000; Skiadopoulos et al., Virology 260:125-35, 1999; and Durbin et al., Virology 261:319-30, 1999, each incorporated herein by reference.

It is often desired to modify the recipient recombinant viral genome or antigenome to encode an alteration at the subject site of mutation that corresponds conservatively to the alteration identified in the heterologous mutant virus. For example, if an amino acid substitution marks a site of mutation in the mutant virus compared to the corresponding wild-type sequence, then a similar substitution can be engineered at the corresponding residue(s) in the recombinant virus. Typically the substitution will specify an identical or conservative amino acid to the substitute residue present in the mutant viral protein. However, it is also possible to alter the native amino acid residue at the site of mutation non-conservatively with respect to the substitute residue in the mutant protein (e.g., by using any other amino acid to disrupt or impair the function of the wild-type residue). Negative stranded RNA viruses from which exemplary mutations are identified and transferred into a recombinant viruses of the invention include PIVs (e.g., HPIV1, HPIV2, HPIV3, BPIV3 and MPIV1), RSV, Newcastle disease virus (NDV), simian virus 5 (SV5), measles virus (MV), rinderpest virus, canine distemper virus (CDV), mumps virus, rabies virus (RaV) and vesicular stomatitis virus (VSV), among others.

As noted above, production of a sufficiently attenuated biologically derived viral mutant can be accomplished by several known methods. One such procedure involves subjecting a partially attenuated virus to passage in cell culture at progressively lower, attenuating temperatures. For example, partially attenuated mutants are produced by passage in cell cultures at suboptimal temperatures. Thus, a cold-adapted (ca) mutant or other partially attenuated viral strain is adapted to efficient growth at a lower temperature by passage in culture. This selection of mutant virus during cold-passage substantially reduces any residual virulence in the derivative strains as compared to the partially attenuated parent. Alternatively, specific mutations can be introduced into biologically derived viruses by subjecting a partially attenuated parent virus to chemical mutagenesis, e.g., to introduce *ts* mutations or, in the case of viruses which are already *ts*, additional *ts* mutations sufficient to confer increased attenuation and/or stability of the *ts* phenotype of the attenuated derivative. Means for the introduction of *ts* mutations into non-segmented negative-stranded RNA viruses include replication of the virus in the presence of a mutagen such as 5-fluorouridine according to generally known procedures. Other chemical mutagens can also be used. Attenuation can result from a *ts* mutation in almost any viral gene, although a particularly amenable target for this purpose has been found to be the polymerase (L) gene. The level of temperature sensitivity of replication in exemplary attenuated viruses for use within the invention is determined, for example, by comparing replication at a permissive temperature with that at several restrictive temperatures. The lowest temperature at which the replication of the virus is reduced 100-fold or more in comparison with its replication at the permissive temperature is termed the shutoff temperature. In experimental animals and humans, both the replication and virulence of non-segmented negative-stranded RNA viruses correlate with the mutant's shutoff temperature.

As exemplified in Figure 3 (Panels A-C) various mutations identified in a heterologous negative stranded RNA virus can be incorporated into a recombinant non-segmented negative-stranded RNA virus of the invention to yield attenuation or other desired phenotypic changes. The figure provides exemplary sequence alignments between HPIV2 wild-type (wt), HPIV3 wt, HPIV1 wt, or BPIV3 wt identifying regions containing known attenuating mutations in the heterologous virus. Based on these and similar comparisons, mutations previously identified in a heterologous virus are mapped to a corresponding position in a subject virus of the invention for "transfer" (i.e., by introduction of an identical, conservative or non-conservative mutation, potentially including a substitution, deletion or insertion, at a homologous or corresponding position identified by alignment) into the subject recombinant virus of the invention. A large assemblage of such mutations are available (see, e.g., Newman et al., Virus Genes 24:77-92, 2002; Feller et al., Virology 276:190-201, 2000; Skiadopoulos et al., Virology 260:125-35, 1999; and Durbin et al., Virology 261:319-30, 1999, each incorporated herein by reference) for incorporation into recombinant non-segmented negative-stranded RNA viruses of the invention. As depicted in Figures 3A and 3C (Panels A-C), a corresponding amino acid position previously shown to confer a phenotypic change in a heterologous virus (when the indicated wild-type residue is altered, e.g., by substitution) is identified by conventional sequence alignment. The corresponding amino acid position in a subject virus of the invention (e.g., a HPIV1) is thereby identified as a target site for mutation in the recombinant virus to yield attenuation or other desired phenotypic changes.

In certain detailed embodiments, the genome or antigenome of a non-segmented negative-stranded RNA virus of the invention is recombinantly modified to incorporate one or any combination of mutation(s) selected from mutations specifying previously identified amino acid substitution(s) in the L protein of the virus at a position corresponding to Tyr942, Leu992, and/or Thr1558 of HPIV3 JS cp45. For example, corresponding targets of wild-type (wt) HPIV2 L for incorporation of these exemplary mutations are Tyr948, Ala998, and Leu1566. In other exemplary embodiments, the recombinant HPIV or other non-segmented negative-stranded RNA virus genome or antigenome is modified to incorporate an attenuating mutation at an amino acid position corresponding to an amino acid position of an attenuating mutation identified in a heterologous, mutant nonsegmented negative stranded RNA virus, for example, another HPIV, a non-human PIV such as a bovine PIV3 (BPIV3) or murine PIV1 (MPIV1), or a non-PIV virus such as respiratory syncytial virus (RSV). In one exemplary embodiment, an attenuating mutation identified in a BPIV3 virus L protein, at amino acid position Thr 1711, is incorporated in a recombinant HPIV2 at the corresponding position (Ser1724), as identified by conventional alignment methods (Figures 3A and 3C, Panels A-C; see also, Schmidt et al., J. Virol. 74:8922-9, 2000, incorporated herein by reference).

In other detailed embodiments of the invention, the genome or antigenome of the non-segmented negative-stranded RNA virus incorporates a recombinant modification that specifies an attenuating mutation at an amino acid position corresponding to an amino acid position of an attenuating mutation identified in a respiratory syncytial virus (RSV). In one specific embodiment, the attenuating mutation identified in RSV comprises an amino acid substitution of phenylalanine at position 521 of the RSV L protein, which aligns, for example, with conserved positions in the HPIV2, HPIV3, and HPIV1L proteins. In HPIV2, the exemplary conserved target site for mutation corresponds to the Phe460 of the HPIV2 L protein Figures 3A and 3B (Panels A-C). Thus, in one exemplary embodiment, Phe460 of the HPIV2 L protein is substituted to a Leu residue or, alternatively, to another amino acid.

Various exemplary mutations from RSV for incorporation into recombinant RSV and other non-segmented negative-stranded RNA viruses of the invention are noted and described below in Table 2 and further detailed in the Examples herein below.

**Table 2 RSV cDNA MUTATIONS Sequence comparison of wt RSV and RSV cDNA mutations.**

| *Mutation* | Gene | Nucleotide position | Restriction site | Nucleotide sequence | Amino Acid position | Amino acid change |
|---|---|---|---|---|---|---|
| | | | | wt. AAA TCA GTT AAA | 267 | V>I |
| cp | N | 1938 | | mut. AAA TCA ATT AAA | | |
| | | | | wt. ATA TCA AAT ATA GAA ACT GTG mut. | 218 | E>A |
| cp | F | 6314 | | mut. ATA TCA AAT ATA GCA ACT GTG | | |
| cp | F | 7229 | | wt. mut. | 523 | T>I |
| | | | | wt. GGA GAT TGT ATA | 319 | C>Y |
| cp | L | 9454 | *Accl* (lost) | mut. GGA GAT TAC ATA | | |
| | | | | wt. CAT ATA AGG ATT GCT AAT TCT GAA TTA GAA | 1690 | H>Y |
| cp | L | 13566 | ***Sac 1*** | mut. TAC ATA AGG ATT GCT AAT TCT GAG CTC GAA | | |
| | | | | wt. ACT CAT GCT CAA GCA GAT | | |
| L248 | L | 10990 | | mut. ACT CAT GCT TTA GCA GAT | 831 | Q>L |
| | | | | wt. CTT CCA TTG GAT TGT AAC | | |
| L404 | L | 12047 | ***Xho I*** | mut. CTT CCA CTC GAG TGT AAC | 1183 | D>E |
| | | | | wt. CGT TTC TAT CGT GAG TTT CGG TTG CCT | | |
| L530 | L | 10061 | ***Bst EII*** | mut. CGT CTA TAT CGT GAG TTT CGG TTA CCT | 521 | F>L |
| | | | | wt. CTC AAA AAT GAT | | |
| L955 | L | 8626 | Na | mut. CTC AAA ATA GAT | 43 | N>I |
| | | | | wt. GCC ACT GAG ATG ATG AGG | | |
| L1009 | L | 12003 | ***Bst XI*** | mut. GCC ACT GAG ATG GTC AGG | 1169 | M>V |
| | | | | wt. ACC CTT GGG TTA ACA TAT GAA | | |
| L1030 | L | 12459 | ***Bsu 36I*** | mut. ACC TTA GGG TTA ACA AAT GAA | 1321 | Y>N |
| | | | | wt. GGGGCAAAT ATG TCA CGA | | |
| | | | | mut GGGGCAAAC ATG TCA CGA | na | na |
| M2 404 | M2 | 7606 | ***Xba I*** | wt. GGGGCAAAT ATG TCT AGA | | |
| | | | | wt. CCA ACA TCA | | |
| Site | P | 2999, 3002 | ***Aat II*** | mut. CCG AGG TCA | na | na |
| | | | | wt. TAATTTAAAA. TTAAGGAGAGAT | | |
| Site | NS2/N | 1099 | ***Afl II*** | rA2 TAATTTAAAACTTAAGGAGAGAT | na | na |
| | | | | wt. GGGGCAAATACAAAG ATG GCT | | |
| Site | N | 1139-40 | Nco I | rA2 GGGGCAAATACAACC ATG GCT | na | na |
| | | | Pst I | wt. ATC CTC ACT GCA GTC | | |
| Site | F | 5709 | (missing) | rA2 ATC CTC ACC GCA GTC | 16 | silent |
| | G/F | | | wt. TTATCACAAAAAGCCATGACCAACTT | | |
| Site | IGR | 5612,16 | Stu I | rA2 TTATCACAAAAGGCCTTGACCAACTT | na | na |
| | | | | Wt. ACGCGAAAAAATGCGTACAACAAACT | | |
| *4C* | leader | 4 | na | rA2 ACGGGAAAAAATGCGTACAACAAACT | na | na |
| | | | | wt. TAGTTACAAAAAAAAGGAAAGGGT | | |
| 7 vs 8 As | ***P*** | 3243 | na | rA2 TAGTTACAAAAAAA. GGAAAGGGT | na | na |

Many of the foregoing exemplary mutations which can be engineered in a recombinant non-segmented negative-stranded RNA viruses of the invention have been successfully engineered and recovered in recombinant HPIV3 based candidates (Durbin et al., Virology 235:323-332, 1997; Skiadopoulos et al., J. Virol. 72:1762-1768, 1998; Skiadopoulos et al., J. Virol. 73:1374-1381, 1999; U.S. Patent Application Serial No. 09/083,793, filed May 22, 1998; U.S. Patent Application Serial No. 09/458,813, filed December 10, 1999; U.S. Patent Application Serial No. 09/459,062, filed December 10, 1999; U.S. Provisional Application No. 60/047,575, filed May 23, 1997 (corresponding to International Publication No. WO 98/53078), and U.S. Provisional Application No. 60/059,385, filed September 19, 1997, each incorporated herein by reference). In addition, the above-incorporated references describe construction of chimeric PIV recombinants, e.g., having the HN and F genes of HPIV1 substituted into a partial HPIV3 background genome or antigenome, which is further modified to bear one or more of the attenuating mutations identified in HPIV3 JS cp45. One such chimeric recombinant incorporates all of the attenuating mutations identified in the L gene of cp45. It has since been shown that all of the cp45 mutations outside of the heterologous (HPIV1) HN and F genes can be incorporated in a HPIV3-1 recombinant to yield an attenuated, chimeric candidate.

As noted above, these and other mutations from biologically derived, nonsegmented, negative stranded RNA viruses represent a large "menu" from which individual mutations can be selected and combined with other mutations for adjusting the level of attenuation, immunogenicity and genetic stability in recombinant viruses of the invention. In this context, many recombinant viral candidates will include one or more, and often two or more, mutations from a biologically derived non-segmented negative-stranded RNA virus, for example, any one or combination of mutations identified from HPIV3 JS cp45, BPIV3, and RSV. Many recombinant viruses within the invention will incorporate a plurality of mutations thus identified. Often, these mutations are stabilized against reversion in recombinant viruses by multiple nucleotide substitutions in a codon specifying the mutation(s).

By identifying and incorporating specific mutations associated with desired phenotypes, e.g., a *ca* or *ts* phenotype, into infectious recombinant viruses, the invention provides for other, site-specific modifications at, or within close proximity to, the identified mutation. Whereas most attenuating mutations produced in biologically derived non-segmented negative-stranded RNA viruses are single nucleotide changes, other "site specific" mutations can also be incorporated by recombinant techniques into a recombinant virus of the invention. As used herein, site-specific mutations include insertions, substitutions, deletions or rearrangements of from 1 to 3, up to about 5-15 or more altered nucleotides (e.g., altered from a wild-type viral sequence, from a sequence of a selected mutant viral strain, or from a parent recombinant viral clone subjected to mutagenesis). Such site-specific mutations may be incorporated at, or within the region of, a selected, biologically derived point mutation. Alternatively, the mutations can be introduced in various other contexts within a recombinant viral clone, for example at or near a cis-acting regulatory sequence or nucleotide sequence encoding a protein active site, binding site, immunogenic epitope, etc. In various embodiments, site-specific nucleotide substitutions, additions, deletions or rearrangements are introduced upstream or downstream, e. g., from 1 to 3, 5-10 and up to 15 nucleotides or more 5' or 3', relative to a targeted nucleotide position, e.g., to construct or ablate an existing cis-acting regulatory element.

In addition to single and multiple point mutations and site-specific mutations, changes to recombinant non-segmented negative-stranded RNA viruses of the invention include deletions, insertions, substitutions or rearrangements of one or more gene(s) or genome segment(s). Particularly useful are deletions involving one or more gene(s) or genome segment(s) that yield additional desired phenotypic effects. Various modifications in this context are described for HPIVs (see, e.g., U.S. Patent No. 6,410,023 issued to Durbin et al. on June 25, 2002, incorporated herein by reference). For example, in HPIV3, expression of one or more of the C, D, or V open reading frame(s) (ORF(s) or another auxillary gene can be reduced or ablated by modifying the recombinant HPIV3 genome or antigenome, e.g., to incorporate a mutation that alters the coding assignment of an initiation codon, introduces one or one or more stop codon(s), or deletes all or part of the coding sequence of the ORF (see, e.g., U.S. Patent No. 6,410,023 issued to Durbin et al. on June 25, 2002, incorporated herein by reference). Other exemplary modifications for HPIV1 and HPIV2 will include modifications that alter or ablate expression of one or more of the C, C', Y1, and/or Y2 ORF(s) or other auxiliary gene(s). These recombinants will possess highly desirable phenotypic characteristics for development of immunogenic compositions. In particular, the subject modifications will be highly stable and may specify one or more desired phenotypic changes including (i) altered growth properties in cell culture, (ii) attenuation in the upper and/or lower respiratory tract of mammals, (iii) a change in viral plaque size, (iv) a change in cytopathic effect, and (v) a change in immunogenicity.

Thus, in more detailed aspects of the instant invention, a recombinant non-segmented negative-stranded RNA virus incorporates one or more partial or complete gene deletions, knock out mutations, or mutations that simply reduce or increase expression of a selected gene. This can be achieved, e.g., by introducing a frame shift mutation or termination codon within a selected coding sequence, altering translational start sites, changing the position of a gene or introducing an upstream start codon to alter its rate of expression, changing gene-start (GS) and/or gene end (GE) transcription signals to alter phenotype, or modifying an RNA editing site (e.g., growth, temperature restrictions on transcription, etc.). In more detailed aspects of the invention, recombinant non-segmented negative-stranded RNA viruses are provided in which expression of one or more gene(s) is ablated at the translational or transcriptional level without deletion of the gene or of a segment thereof, by, e.g., introducing multiple translational termination codons into a translational open reading frame, altering an initiation codon, or modifying an editing site. These forms of knock-out virus will often exhibit reduced growth rates and small plaque sizes in tissue culture. Thus, these methods provide yet additional types of attenuating mutations which ablate expression of a viral gene, typically a gene that is not one of the major viral protective antigens. In this context, knock-out virus phenotypes produced without deletion of a gene or genome segment can be alternatively produced by deletion mutagenesis, as described, to effectively preclude correcting mutations that may restore synthesis of a target protein. Other gene knock-outs can be made using alternate designs and methods that are known in the art (as described, for example, in (Kretschmer et al., Virology 216:309-316,1996; Radecke et al Virology 217:418-421, 1996; Kato et al., EMBO J. 16:578-587, 1987; and Schneider et al., Virology 277:314-322, 1996, each incorporated herein by reference).

Nucleotide modifications that may be introduced into recombinant non-segmented negative-stranded RNA viruses of the invention may alter small numbers of bases (e.g., from 15-30 bases, up to 35-50 bases or more), large blocks of nucleotides (e.g., 50-100, 100-300, 300-500, 500-1,000 bases), or nearly complete or complete genes (e.g., 1,000-1,500 nucleotides, 1,500-2,500 nucleotides, 2,500-5,000, nucleotides, 5,000-6,000 nucleotides or more) in the genome or antigenome, depending upon the nature and desired effect of the modification. For example, a small number of bases may be changed to insert or ablate an immunogenic epitope or change a small genome segment, whereas large block(s) of bases are involved when genes or large genome segments are added, substituted, deleted or rearranged.

In related aspects, the invention provides for supplementation of mutations adopted in a recombinant non-segmented negative-stranded RNA virus from a biologically derived non-segmented negative-stranded RNA virus (e.g., *ca* and *ts* mutations), with additional types of mutations involving the same or different genes in a further modified recombinant virus. For example, one or more native or heterologous genes can be selectively altered in terms of expression levels, or can be added, deleted, or substituted, in whole or in part, alone or in combination with other desired modifications, to yield a recombinant virus exhibiting novel characteristics. Alternatively or in addition, the order of genes in the recombinant virus can be changed, a genome promoter can be replaced with its antigenome counterpart or vice versa. Different or additional modifications in the sequence can be made to facilitate manipulations, such as the insertion of unique restriction sites in various intergenic regions or elsewhere. Nontranslated gene sequences can be removed to increase capacity for inserting foreign sequences.

Other mutations for incorporation into recombinant non-segmented negative-stranded RNA viruses of the invention include mutations directed toward cis-acting signals, which can be readily identified, e.g., by mutational analysis of viral minigenomes. For example, insertional and deletional analysis of the leader, trailer and/or flanking sequences identifies viral promoters and transcription signals and provides a series of mutations associated with varying degrees of reduction of RNA replication or transcription. Saturation mutagenesis (whereby each position in turn is modified to each of the nucleotide alternatives) of these cis-acting signals also can be employed to identify many mutations that affect RNA replication or transcription. Any of these mutations can be inserted into a viral antigenome or genome as described herein. Evaluation and manipulation of trans-acting proteins and cis-acting RNA sequences using the complete antigenome cDNA is assisted by the use of viral minigenomes as described in the above-incorporated references.

Additional mutations within recombinant non-segmented negative-stranded RNA viruses of the invention may also include replacement of the 3' end of genome with its counterpart from antigenome or vice versa, which is associated with changes in RNA replication and transcription. In one exemplary embodiment, the level of expression of specific viral proteins, such as the protective HN and/or F antigens of PIV, can be increased by substituting the natural sequences with ones which have been made synthetically and designed to be consistent with efficient translation. In this context, it has been shown that codon usage can be a major factor in the level of translation of mammalian viral proteins (Haas et al., Current Biol. 6:315-324, 1996, incorporated herein by reference). Optimization by recombinant methods of the codon usage of the mRNAs encoding the subject proteins of the recombinant virus will provide improved expression for these genes.

In another exemplary embodiment, a sequence surrounding a translational start site (often including a nucleotide in the -3 position relative to the AUG start site) of a selected viral gene is modified, alone or in combination with introduction of an upstream start codon, to modulate gene expression by specifying up- or down-regulation of translation. Alternatively, or in combination with other recombinant modifications disclosed herein, gene expression of any selected gene(s) of a recombinant virus can be modulated by altering a transcriptional GS or GE signal. In alternative embodiments, levels of gene expression in a recombinant non-segmented negative-stranded RNA viruses are modified at the level of transcription. In one aspect, the position of a selected gene in the gene map of the virus is changed to a more promoter-proximal or promoter-distal position, whereby the gene will be expressed more or less efficiently, respectively. According to this aspect, modulation of expression for specific genes can be achieved yielding reductions or increases of gene expression from two-fold, more typically four-fold, up to ten-fold or more compared to wild-type levels often attended by a commensurate decrease in expression levels for reciprocally, positionally substituted genes. These and other transpositioning changes yield novel recombinant viruses having attenuated phenotypes, for example due to decreased expression of selected viral proteins involved in RNA replication, or having other desirable properties such as increased antigen expression.

In other embodiments, recombinant viruses useful in immunogenic compositions can be conveniently modified to accommodate antigenic drift in circulating virus. Typically the modification will be in an antigenic glycoprotein, for example the HN and/or F proteins of PIV, or the F, G, or SH proteins of RSV. An entire glycoprotein gene, or a genome segment encoding a particular immunogenic region thereof, from one viral strain or group is incorporated into a recombinant genome or antigenome cDNA by replacement of a corresponding region in a recipient clone of a different strain or group, or by adding one or more copies of the gene or genome segment, such that multiple antigenic forms are represented. Progeny virus produced from the modified recombinant virus can then be used in immunization protocols against emerging strains.

In certain aspects of the invention, a coding sequence or non-coding sequence (e.g., a promoter, gene-end, gene-start, intergenic or other cis-acting element) of the non-segmented negative-stranded RNA virus will be replaced with a heterologous (i.e., from a different viral species or strain) counterpart sequence to yield a chimeric virus having a variety of possible attenuating and other phenotypic effects. For example, host range and other desired effects can be engineered by importing a bovine PIV3 (BPIV3) or murine PIV1 (MPIV1) gene or genome segment into a recombinant HPIV "background" genome or antigenome, wherein the bovine or murine gene does not function as efficiently in a human cell (e.g., from incompatibility of the heterologous sequence or protein with a biologically interactive HPIV sequence or protein that ordinarily cooperates with the substituted sequence or protein for viral transcription, translation, assembly, etc., or, more typically in a host range restriction, with a cellular protein which is different between the permissive and less permissive host).

In exemplary embodiments, bovine PIV sequences are selected for introduction into HPIV based on known aspects of bovine and heterologous human PIV structure and function. In more detailed aspects, the invention provides methods for attenuating recombinant HPIV candidates based on the construction of chimeras between HPIV and a non-human PIV, for example MPIV1 (Sendai virus), BPIV3, SV5, SV41, and NDV (e.g., as disclosed in U.S. Patent Application Serial No. 09/586,479, filed June 1, 2000 by Schmidt et al. (corresponding to PCT Publication WO 01/04320); Schmidt et al., J. Virol. 74:8922-9, 2000, each incorporated herein by reference). This method of attenuation is based on host range effects due to the introduction of one or more gene(s) or genome segment(s) of the non-human PIV into a human PIV vector-based chimeric virus. For example, there are numerous nucleotide and amino acid sequence differences between BPIV and HPIVs, which are reflected in host range differences. Between HPIV3 and BPIV3 the percent amino acid identity for each of the following proteins is: N (86%), P (65%), M (93%), F (83%), HN (77%), and L (91%). The host range difference is exemplified by the highly permissive growth of HPIV3 in rhesus monkeys, compared to the restricted replication of two different strains of BPIV3 in the same animal (van Wyke Coelingh et al., J. Infect. Dis. 157:655-662, 1988, incorporated herein by reference). Although the basis of the host range differences between HPIV3 and BPIV3 remains to be fully elucidated, it has been shown to involve multiple gene and multiple amino acid differences. The involvement of multiple genes and possibly cis-acting regulatory sequences, each involving multiple amino acid or nucleotide differences, gives a broad basis for attenuation, one which is highly stable to reversion. This is in contrast to the situation with other live attenuated HPIV3 viruses that are attenuated by one or several point mutations. In this case, reversion of any individual mutation may yield a significant reacquisition of virulence or, in a case where only a single residue specified attenuation, complete reacquisition of virulence. In exemplary embodiments of the invention, the recombinant HPIV genome or antigenome is combined with a heterologous gene or genome segment, such as an N, P, M, or L, ORF derived from a BPIV3, or another animal paramyxoviruses.

Chimeric human-bovine or human-murine recombinant HPIV are therefore provided herein that include a partial or complete "background" HPIV genome or antigenome derived from or patterned after HPIV combined with one or more heterologous gene(s) or genome segment(s) of a non-human PIV to form the chimeric PIV genome or antigenome. In certain aspects of the invention, chimeric HPIV of this type incorporate a partial or complete HPIV background genome or antigenome combined with one or more heterologous gene(s) or genome segment(s), e.g., from a bovine PIV. The partial or complete background genome or antigenome typically acts as a recipient backbone into which the heterologous genes or genome segments of the counterpart, non-human PIV are incorporated. Heterologous genes or genome segments from the counterpart PIV represent "donor" genes or polynucleotides that are combined with, or substituted within, the background genome or antigenome to yield a chimeric HPIV that exhibits novel phenotypic characteristics compared to one or both of the contributing PIVs. For example, addition or substitution of heterologous genes or genome segments within a selected recipient HPIV strain may result in an increase or decrease in attenuation, growth changes, altered immunogenicity, or other desired phenotypic changes as compared with a corresponding phenotype(s) of the unmodified recipient and/or donor (U.S. Patent Application Serial No. 09/586,479, filed June 1, 2000 by Schmidt et al.; Schmidt et al., J. Virol. 74:8922-9, 2000, each incorporated herein by reference).

Genes and genome segments that may be selected for use as heterologous substitutions or additions within chimeric PIV vectors include genes or genome segments encoding a PIV N (alternatively designated NP, nucleocapsid protein), V, P, M, F, HN and/or L protein(s) or portion(s) thereof. In addition, genes and genome segments encoding proteins found in other PIV viruses, as well as non-PIV proteins (e.g., an SH protein as found in mumps, RSV, and SV5 viruses), may be incorporated within additional chimeric HPIV recombinants of the invention, and vice versa. Regulatory regions, such as the extragenic 3' leader or 5' trailer regions, and gene-start, gene-end, intergenic regions, or 3' or 5' non-coding regions, are also useful as heterologous substitutions or additions. In exemplary aspects, a chimeric HPIV bearing one or more bovine or murine PIV gene(s) or genome segment(s) exhibits a high degree of host range restriction, e.g., in the respiratory tract of mammalian models of human PIV infection such as hamsters and non-human primates. In more detailed embodiments an HPIV is attenuated by the addition or substitution of one or more bovine PIV3 gene(s) or genome segment(s) selected from N, M, L, V, and P genes and genome segments to a partial or complete HPIV background genome or antigenome.

Typically, the degree of host range restriction exhibited by human-bovine and other chimeric viruses for use in immunogenic compositions of the invention is comparable to the degree of host range restriction exhibited by the respective non-human virus or other "donor" strain. In certain embodiments, the restriction will correspond to a true host range phenotype, i.e., it should be specific to the host in question and should not restrict replication in vitro in a suitable cell line. In addition, chimeric viruses bearing one or more heterologous gene(s) or genome segment(s) elicit a desired immunogenic response in hosts susceptible to infection by the recipient virus. Thus, the invention provides for attenuation of live non-segmented negative-stranded RNA viruses for developing immunogenic compositions against these viruses and other pathogens based on host range effects.

In combination with the host range phenotypic effects provided in chimeric viruses of the invention, it is often desirable to adjust the attenuation phenotype by introducing additional mutations that increase or decrease attenuation of the chimeric virus. Thus, in additional aspects of the invention, attenuated chimeric viruses are produced in which the chimeric genome or antigenome is further modified by introducing one or more attenuating mutations specifying an attenuating phenotype in the resultant virus or subviral particle. These can include mutations generated de novo and tested for attenuating effects according to a rational design mutagenesis strategy described above. For example, the attenuating mutations may be identified in existing biologically derived mutant viruses and thereafter incorporated into a chimeric virus of the invention. Exemplary mutations specify lesions in RNA regulatory sequences or in encoded proteins.

In certain attenuated viral and chimeric viral candidates of the invention, attenuation marked by replication in the lower and/or upper respiratory tract in an accepted animal model (e.g., hamsters, rhesus monkeys or chimpanzees--wherein the virus exhibits correlative replication and immunogenic activity as seen in humans), is reduced by at least about two-fold, more often about 5-fold, 10-fold, or 20-fold, at times 50-100-fold and up to 1,000-fold or greater overall (e.g., as measured between 3-8 days following infection) compared to growth of the corresponding wild-type or mutant parental viral strain.

Various additional or substituted genes or genome segments can be introduced routinely into recombinant and chimeric viruses of the invention. For example, various supernumerary heterologous gene(s) or genome segment(s) can be inserted at any of a variety of sites within the recombinant or chimeric genome or antigenome, for example in HPIV at a position 3' to N, between the N/P, P/M, and/or HN/L genes, or at another intergenic junction or non-coding region of the HPIV genome or antigenome. The inserted genes may be under common control with recipient genes, or may be under the control of an independent set of transcription signals. Genes of interest in this context include genes encoding cytokines, for example, an interleukin (IL-2 through IL-18, e.g., interleukin 2 (IL-2), interleukin 4 (IL-4), interleukin 5 (IL-5), interleukin 6 (IL6), interleukin 12 (IL-12), interleukin 18 (IL-18), tumor necrosis factor alpha (TNFα), interferon gamma (IFNγ), or granulocyte-macrophage colony stimulating factor (GM-CSF) (see, e.g., U.S. Application No. 09/614,285, filed July 12,2000 and priority U.S. Provisional Application Serial No. 60/143,425 filed July 13, 1999, each incorporated herein by reference). Coexpression of these additional proteins provides the ability to modify and improve immune responses against recombinant viruses of the invention quantitatively and/or qualitatively.

In other aspects of the invention, insertion of heterologous nucleotide sequences into recombinant non-segmented negative-stranded RNA viruses are employed separately to modulate the level of attenuation of candidate recombinants, e.g., for the upper respiratory tract. Thus, it is possible to insert nucleotide sequences into a recombinant non-segmented negative-stranded RNA virus that both direct the expression of a foreign protein and attenuate the virus in an animal host, or to use nucleotide insertions separately to attenuate candidate viruses. To define some of the rules that govern the effect of gene insertion on attenuation, gene units of varying lengths may be inserted into a wild type viral backbone and the effects of gene unit length on attenuation examined. Gene unit insertions are contemplated in this regard that do not contain a significant ORF, permitting identification of the effect of gene unit length independently of an effect of the expressed protein of that gene. These heterologous sequences may be inserted as an extra gene unit of various sizes, e.g., from about 150 or more nts in length up to 3,000 nts or more in length. As demonstrated herein, gene insertions or extensions larger than about 3,000 nts in length.

Gene unit (GU) insertions of about 1,000 or 2,000 nts in length will substantially attenuate non-segmented negative-stranded RNA viruses of the invention for the upper respiratory tract of mammalian subjects. In addition, gene unit insertions can have the dual effect of both attenuating a candidate virus and inducing an immunogenic response against a second virus. Alternately, gene extensions in the 3'-noncoding region (NCR) of a viral gene, which cannot express additional proteins, can also be attenuating in and of themselves. Within these methods of the invention, gene insertion length is a determinant of attenuation.

GU and NCR insertions within recombinant non-segmented negative-stranded RNA viruses of the invention produce an attenuation phenotype characterized by efficient replication in vitro and decreased replication in vivo. The mechanism of attenuation resulting from a GU insertion may result from one or more of the following factors acting predominantly in vivo. The addition of an extra gene unit may decrease the level of transcription of downstream genes since there is a transcriptional gradient in which more promoter-proximal genes are transcribed at a higher rate than the more promoter-distal genes. The decreased expression of the downstream gene products resulting from the decreased abundance of their mRNAs could result in attenuation if their gene product is limiting or if a specific ratio of gene products that is required for efficient replication is altered. It is thought that the transcription gradient is a consequence of the transcriptase complex falling off the template during transcription as well as during the transfer across gene junctions. Alternatively, the increase in the overall length of the genome and the extra mRNAs transcribed may increase the level of viral double stranded RNA made which in turn may induce a higher level of the antiviral activity of the interferon system. Finally, the overall level of genome replication may be reduced due to the increase in length of the genome and the antigenome. This may result from a disengagement of replicase complexes from the template during replication of the genomic RNA or antigenomic RNA. The decreased amount of genome available for packaging into virions may result in a decrease in virus yield that results in attenuation.

Deletions, insertions, substitutions and other mutations involving changes of whole viral genes or genome segments within recombinant non-segmented negative-stranded RNA viruses of the invention yield highly stable candidates, which are particularly important in the case of immunosuppressed individuals. Many of these changes will result in attenuation of resultant strains, whereas others will specify different types of desired phenotypic changes. For example, accessory (i.e., not essential for in vitro growth) genes are excellent candidates to encode proteins that specifically interfere with host immunity (see, e.g., Kato et al., EMBO. J. 16:578-87, 1997, incorporated herein by reference). Ablation of such genes in candidate viruses is expected to reduce virulence and pathogenesis and/or improve immunogenicity.

In other detailed embodiments of the invention, chimeric non-segmented negative-stranded RNA viruses are constructed using a "vector" genome or antigenome that is recombinantly modified to incorporate one or more antigenic determinants of a heterologous pathogen. The vector genome or antigenome is comprised of a partial or complete non-segmented negative-stranded RNA viral genome or antigenome, which may itself incorporate nucleotide modifications such as attenuating mutations. The vector genome or antigenome is modified to form a chimeric structure through incorporation of a heterologous gene or genome segment. More specifically, chimeric viruses of the invention are constructed through a cDNA-based virus recovery system that yields recombinant viruses that incorporate a partial or complete vector or "background" viral genome or antigenome combined with one or more "donor" nucleotide sequences encoding the heterologous antigenic determinant(s). In exemplary embodiments a non-segmented negative-stranded RNA viral vector genome or antigenome is modified to incorporate one or more genes or genome segments that encode antigenic determinant(s) of one or more heterologous non-segmented negative-stranded RNA viruses (e.g., of an HPIV, HMPV, MV, or RSV), and/or a non-viral pathogen. Thus constructed, chimeric viruses of the invention may elicit an immune response against a specific non-segmented negative-stranded RNA virus (e.g., HPIV1, HPIV2, HPIV3, RSV-A, RSV-B, HMPV, or MV) or against a non-viral pathogen. Alternatively, compositions and methods are provided employing a chimeric virus to elicit a polyspecific immune response against multiple non-segmented negative-stranded RNA viruses (e.g., multiple HPIVs, or an HPIV and RSV or MV). In yet additional embodiments, compositions and methods are provided employing chimeric viruses to elicit an immune response against a segmented negative-stranded RNA virus (e.g., influenza A or B or a corona virus), a positive-stranded RNA virus (e.g., Togaviruses, alphaviruses (including, encephalitis viruses), flaviruses (including West Nile virus, Dengue virus), and picomaviruses (including polio virus and other enteroviruses)), or DNA viruses (e.g., herpes simplex and zoster viruses, hepatitis B virus, Epstein-Barr virus, and cytomegalovirus).

In more detailed aspects, the partial or complete vector genome or antigenome generally acts as a backbone into which heterologous genes or genome segments of a different pathogen are incorporated. Often, the heterologous pathogen is a different non-segmented negative-stranded RNA virus from which one or more gene(s) or genome segment(s) is/are combined with, or substituted within, the vector genome or antigenome. In addition to providing novel immunogenic characteristics, the addition or substitution of heterologous genes or genome segments within the vector virus may confer an increase or decrease in attenuation, growth changes, or other desired phenotypic changes as compared with the corresponding phenotype(s) of the unmodified vector and donor viruses.

The introduction of heterologous immunogenic proteins, protein domains and immunogenic epitopes to produce chimeric viruses is particularly useful to generate novel immune responses in an immunized host. Addition or substitution of an immunogenic gene or genome segment from one, donor pathogen within a recipient vector genome or antigenome can generate an immune response directed against the donor pathogen, the vector, or against both the donor pathogen and vector.

General methods and compositions useful for engineering chimeric viruses have been developed for HPIV3 (Durbin et al., Virology 235:323-332, 1997; Skiadopoulos et al., J. Virol. 72:1762-1768, 1998; Tao et al., J Virol 72:2955-2961, 1998; Skiadopoulos et al., J. Virol. 73:1374-1381, 1999; Skiadopoulos et al., Vaccine 18:503-510, 1999; Tao et al., Vaccine 17:1100-1108, 1999; Tao et al., Vaccine 18:1359-1366, 2000; U.S. Patent Application Serial No. 09/083,793, filed May 22, 1998; U.S. Patent Application Serial No. 09/458,813, filed December 10, 1999; U.S. Patent Application Serial No. 09/459,062, filed December 10, 1999; U.S. Provisional Application No. 60/047,575, filed May 23, 1997 (corresponding to International Publication No. WO 98/53078), and U.S. Provisional Application No. 60/059,385, filed September 19, 1997, each incorporated herein by reference). In particular, the above-incorporated references describe construction of chimeric PIV recombinants, e.g., having the HN and F genes of HPIV1 substituted into a partial HPIV3 background genome or antigenome, which is further modified to bear one or more of the attenuating mutations identified in HPIV3 JS cp45. One such chimeric recombinant incorporates all of the attenuating mutations identified in the L gene of cp45 outside of the heterologous (HPIV1) HN and F genes, yielding an attenuated, chimeric candidate. Additional representative disclosures for generating chimeric non-segmented negative-stranded RNA viruses are provided for RSV in the incorporated references noted above.

Chimeric non-segmented negative-stranded RNA viruses of the invention may also be constructed that express a chimeric protein, for example an immunogenic glycoprotein having a cytoplasmic tail and/or transmembrane domain specific to a vector fused to a heterologous ectodomain of a different non-segmented negative-stranded RNA virus or non-viral pathogen to provide a fusion protein that elicits an immune response against the heterologous pathogen. For example, a heterologous genome segment encoding a glycoprotein ectodomain from a HPIV1 or HPIV3 HN or F glycoprotein may be joined with a genome segment encoding the corresponding HPIV2 HN or F glycoprotein cytoplasmic and transmembrane domains to form a HPIV2-1 or HPIV2-3 chimeric glycoprotein that elicits an immune response against HPIV 1 or HPIV3. In more detailed embodiments, the heterologous genome segment or segments encode a glycoprotein ectodomain or immunogenic portion or epitope thereof, and optionally include other portions of the heterologous or "donor" glycoprotein, for example both an ectodomain and transmembrane region that are substituted for counterpart glycoprotein ecto- and transmembrane domains in the vector genome or antigenome. Further details concerning these aspects of the invention are exemplified in United States Patent Application Serial No. 09/459,062, filed on December 10, 1999 by Tao et al., incorporated herein by reference.

Another example of such a chimeric non-segmented negative-stranded RNA virus of the invention which expresses a chimeric protein which is a heterologous fusion protein or polypeptide fragment thereof that is effective to facilitate the fusion of the recombinant virus to a target cell membrane, which is fused to a cDNA encoding a portion of the viral G protein, particularly the carboxy terminal membrane proximal ectomain ("tail") portion thereof. In one embodiment, the heterologous fusion protein is CD4, and the virus is VSV. Further details concerning these aspects of the invention are exemplified in United States Patent No. 6,497,873, issued on December 24, 2002 to Whitt et al., incorporated herein by reference.

As used herein, the term "gene" generally refers to a portion of a non-segmented negative-stranded RNA viral genome encoding an MRNA, which typically begins at the upstream end with a gene-start (GS) signal and ends at the downstream end with the gene-end (GE) signal. The term gene is also interchangeable with the term "translational open reading frame", or ORF, particularly in the case where a protein is expressed from an additional ORF rather than from a unique mRNA. The viral genome also contains extragenic leader and trailer regions, possessing part of the promoters required for viral replication and transcription, as well as non-coding and intergenic regions. Transcription initiates at the 3' end and proceeds by a sequential stop-start mechanism that is guided by short conserved motifs found at the gene boundaries. The upstream end of each gene contains a gene-start (GS) signal, that directs initiation of its respective mRNA. The downstream terminus of each gene contains a gene-end (GE) motif that directs polyadenylation and termination.

By "genome segment" is meant any length of continuous nucleotides from the PIV genome, which might be part of an ORF, a gene, or an extragenic region, or a combination thereof. When a subject genome segment encodes an antigenic determinant, the genome segment encodes at least one immunogenic epitope capable of eliciting a humoral or cell mediated immune response in a mammalian host. The genome segment may also encode an immunogenic fragment or protein domain. In other aspects, the donor genome segment may encode multiple immunogenic domains or epitopes, including recombinantly synthesized sequences that comprise multiple, repeating or different, immunogenic domains or epitopes.

Exemplary genome sequences of heterologous viruses for use in construction of chimeric non-segmented negative-stranded RNA viruses of the invention have been described for the human PIV3 strains JS (GenBank accession number Z11575, incorporated herein by reference) and Washington (Galinski M.S. In Kingsbury, D.W. (Ed.), The Paramyxoviruses, pp. 537-568, Plenum Press, New York, 1991, incorporated herein by reference); for HPIV1/Wash64 (GenBank accession number AF457102, incorporated herein by reference); for HPIV2 (U.S. Provisional Application No. 60/412,053, filed September 18, 2002, incorporated herein by reference) for the bovine PIV3 (BPIV3) strain 910N (GenBank accession number D80487, incorporated herein by reference); for BPIV3 Kansas (GenBank accession number AF178654, incorporated herein by reference); for BPIV Shipping fever (GenBank accession number AF178655, incorporated herein by reference); for RSV A2 (GenBank accession number AF035006, incorporated herein by reference); and for HMPV (GenBank accession number AF371337, incorporated herein by reference). Further details concerning construction of chimeric non-segmented negative-stranded RNA viruses for use within the invention are provided, for example, in U.S. Patent Application Serial No. 09/083,793, filed May 22, 1998; U.S. Patent Application Serial No. 09/458,813, filed December 10, 1999; U.S. Patent Application Serial No. 09/459,062, filed December 10, 1999; U.S. Provisional Application No. 60/047,575, filed May 23, 1997 (corresponding to International Publication No. WO 98/53078), U.S. Provisional Application No. 60/059,385, filed September 19, 1997; U.S. Provisional Application No. 60/170,195 filed December 10, 1999; and U.S. Patent Application Serial No. 09/733,692, filed December 8, 2000 (corresponding to International Publication No. WO 01/42445A2), each incorporated herein by reference.

Chimeric non-segmented negative-stranded RNA viruses of the invention can be readily modified to express one or more major antigenic determinants of a wide range of pathogens. In this regard the invention provides for development of recombinant viruses incorporating antigenic determinants of, and capable of eliciting an effective immune response against, subgroup A and subgroup B respiratory syncytial viruses (RSV), HMPV, measles virus, human metapneumoviruses, mumps virus, human papilloma viruses, type 1 and type 2 human immunodeficiency viruses, herpes simplex viruses, cytomegalovirus, rabies virus, human metapneuomovirus, Epstein Barr virus, filoviruses, bunyaviruses, flaviviruses, corona viruses (e.g., SARS-associated human corona virus), alphaviruses and influenza viruses, among other pathogens. Pathogens that may be targeted for development of immunogenic compositions according to the methods of the invention include viral and bacterial pathogens, as well as protozoans and multicellular pathogens. Useful antigenic determinants from many important human pathogens in this context are known or readily identified for incorporation within chimeric viruses of the invention. Thus, major antigens have been identified for the foregoing exemplary pathogens, including the measles virus HA and F proteins; the F, G, SH and M2 proteins of RSV, mumps virus HN and F proteins, human papilloma virus L1, L2, E6, or E7 proteins, type 1 or type 2 human immunodeficiency virus gp120 and gp160 proteins, herpes simplex virus and cytomegalovirus gB, gC, gD, gE, gG, gH, gI, gJ, gK, gL, and gM proteins, rabies virus G protein, human metapneuomovirus F and G proteins, Epstein Barr Virus gp350 protein; filovirus G protein, bunyavirus G protein, flavivirus E and NS1 proteins, metapneumovirus G and F proteins, corona virus spike (S) and small membrane (E) proteins, and alphavirus E protein. These major antigens, as well as other antigens known in the art for the enumerated pathogens and others, are well characterized to the extent that many of their antigenic determinants, including the full length proteins and their constituent antigenic domains, fragments and epitopes, are identified, mapped and characterized for their respective immunogenic activities.

Among the numerous, exemplary mapping studies that identify and characterize major antigens of diverse pathogens for use within the invention are epitope mapping studies directed to the hemagglutinin-neuraminidase (HN) gene of HPIV3 (van Wyke Coelingh et al., J. Virol. 61:1473-1477, 1987, incorporated herein by reference). This report provides detailed antigenic structural analyses for 16 antigenic variants of HPIV3 variants selected by using monoclonal antibodies (MAbs) to the HN protein that inhibit neuraminidase, hemagglutination, or both activities. Each variant possessed a single-point mutation in the HN gene, coding for a single amino acid substitution in the HN protein. Operational and topographic maps of the HN protein correlated well with the relative positions of the substitutions. Computer-assisted analysis of the HN protein predicted a secondary structure composed primarily of hydrophobic β sheets interconnected by random hydrophilic coil structures. The HN epitopes were located in predicted coil regions. Epitopes recognized by MAbs which inhibit neuraminidase activity of the virus were located in a region which appears to be structurally conserved among several paramyxovirus HN proteins and which may represent the sialic acid-binding site of the HN molecule.

This exemplary work, employing conventional antigenic mapping methods, identified single amino acids that are important for the integrity of HN epitopes. Most of these epitopes are located in the C-terminal half of the molecule, as expected for a protein anchored at its N terminus (Elango et al., J. Virol. 57:481-489, 1986). Previously published operational and topographic maps of the PIV3 HN indicated that the MAbs employed recognized six distinct groups of epitopes (I to VI) organized into two topographically separate sites (A and B), which are partially bridged by a third site (C). These groups of epitopes represent useful candidates for antigenic determinants that may be incorporated, alone or in various combinations, within chimeric viruses of the invention. (See, also, Coelingh et al., Virology 143:569-582, 1985; Coelingh et al., Virology 162:137-143, 1988; Ray et al., Virology 148:232-236, 1986; Rydbeck et al., J. Gen. Virol. 67:1531-1542, 1986, each incorporated herein by reference).

Additional studies by van Wyke Coelingh et al. (J. Virol. 63:375-382, 1989) provide further information relating to selection of PIV antigenic determinants for use within the invention. In this study, twenty-six monoclonal antibodies (MAbs) (14 neutralizing and 12 nonneutralizing) were used to examine the antigenic structure, biological properties, and natural variation of the fusion (F) glycoprotein ofHPIV3. Analysis of laboratory-selected antigenic variants and of PIV3 clinical isolates indicated that the panel of MAbs recognizes at least 20 epitopes, 14 of which participate in neutralization. Competitive binding assays confirmed that the 14 neutralization epitopes are organized into three nonoverlapping principal antigenic regions (A, B, and C) and one bridge site (AB), and that the 6 nonneutralization epitopes form four sites (D, E, F, and G). Most of the neutralizing MAbs were involved in nonreciprocal competitive binding reactions, suggesting that they induce conformational changes in other neutralization epitopes.

Other antigenic determinants for use within the invention have been identified and characterized for respiratory syncytial virus (RSV). For example, Beeler et al., J. Virol. 63:2941-2950, 1989, incorporated herein by reference, employed eighteen neutralizing monoclonal antibodies (MAbs) specific for the fusion glycoprotein of the A2 strain of RSV to construct a detailed topological and operational map of epitopes involved in RSV neutralization and fusion. Competitive binding assays identified three nonoverlapping antigenic regions (A, B, and C) and one bridge site (AB). Thirteen MAb-resistant mutants (MARMs) were selected, and the neutralization patterns of the MAbs with either MARMs or RSV clinical strains identified a minimum of 16 epitopes. MARMs selected with antibodies to six of the site A and AB epitopes displayed a small-plaque phenotype, which is consistent with an alteration in a biologically active region of the F molecule. Analysis of MARMs also indicated that these neutralization epitopes occupy topographically distinct but conformationally interdependent regions with unique biological and immunological properties. Antigenic variation in F epitopes was then examined by using 23 clinical isolates (18 subgroup A and 5 subgroup B) in cross-neutralization assays with the 18 anti-F MAbs. This analysis identified constant, variable, and hypervariable regions on the molecule and indicated that antigenic variation in the neutralization epitopes of the RSV F glycoprotein is the result of a noncumulative genetic heterogeneity. Of the 16 epitopes, 8 were conserved on all or all but 1 of 23 subgroup A or subgroup B clinical isolates. These antigenic determinants, including the full length proteins and their constituent antigenic domains, fragments and epitopes, all represent useful candidates for integration within chimeric viruses of the invention to elicit novel immune responses as described above. (See also, Anderson et al., J. Infect. Dis. 151:626-633, 1985; Coelingh et al., J. Virol. 63:375-382, 1989; Fenner et al., Scand. J. Immunol. 24:335-340,1986; Fernie et al., Proc. Soc. Exp. Biol. Med. 171:266-271, 1982; Sato et al., J. Gen. Virol. 66:1397-1409, 1985; Walsh et al., J. Gen. Virol. 67:505-513, 1986; Olmsted et al., J. Virol. 63:411-420, 1989, U.S. Patent No. 5,639,853, each incorporated herein by reference).

To express antigenic determinants of heterologous pathogens within chimeric non-segmented negative-stranded RNA viruses, the invention provides numerous methods and constructs. In certain detailed embodiments, a transcription unit comprising an open reading frame (ORF) of a gene encoding an antigenic protein (e.g., the measles virus HA gene) is added to a vector genome or antigenome at various positions. In exemplary embodiments set forth below, chimeric viruses are engineered that incorporate heterologous nucleotide sequences encoding protective antigens from respiratory syncytial virus (RSV) to produce infectious, attenuated candidates. Chimeric non-segmented negative-stranded RNA viruses incorporating one or more RSV antigenic determinants, typically comprise a vector genome or antigenome combined with a heterologous gene or genome segment encoding an antigenic RSV glycoprotein, protein domain (e.g., a glycoprotein ectodomain) or one or more immunogenic epitopes. In one embodiment exemplified below, one or more genes or genome segments from RSV F and/or G genes is/are combined with a RSV vector genome or antigenome of a different subtype to form the chimeric (e.g., RSV A/B chimeric) virus.

The chimeric vector constructs of the invention provide certain advantages over other viral vector constructs. Most other mononegaviruses that have been used as vectors are not derived from human pathogens (e.g., murine PIV1 (Sendai virus) (Sakai et al., FEBS Lett. 456:221-6, 1999), vesicular stomatitis virus (VSV) which is a bovine pathogen (Roberts et al., J. Virol. 72:4704-11, 1998), and canine PIV2 (SV5) He et al., Virology 237:249-60, 1997)). For these nonhuman viruses, little or only weak immunity would be conferred against any human virus by antigens present in the vector backbone. Thus, a nonhuman virus vector expressing a supernumerary gene for a human pathogen would induce resistance only against that single human pathogen (however, this may be desirable in some instances).

In addition, many of the chimeric vector viruses recovered according to the methods of the invention (e.g., based on RSV or HPIV) can be administered to subjects via an intranasal route of administration, mimicking natural infection. Thus, the viruses and immunogenic compositions of the invention will induce both mucosal and systemic immunity and reduce the neutralizing and immunosuppressive effects of maternally-derived serum IgG that is present in infants.

As noted above, the invention embraces a wide range of modifications to recombinant non-segmented negative-stranded RNA viruses, yielding defined mutations that specify desired phenotypic changes. In more detailed aspects of the invention, these defined mutations are introduced into a cDNA copy of the viral genome or antigenome, typically using genomic or antigenomic cDNA subfragments to assemble a complete genome or antigenome cDNA. This construction method has the advantage that each region of the genome or antigenome can be manipulated separately, where small cDNA constructs provide for better ease of manipulation than large cDNA constructs, and can thereafter be readily assembled into a complete cDNA. Thus, the complete antigenome or genome cDNA, or a selected subfragment thereof, can be used as a template for oligonucleotide-directed mutagenesis. This can be through the intermediate of a single-stranded phagemid form, such as using the MUTA-gen® kit of Bio-Rad Laboratories (Richmond, CA), or a method using the double-stranded plasmid directly as a template such as the CHAMELEON® mutagenesis kit of Strategene (La Jolla, CA), or by the polymerase chain reaction employing either an oligonucleotide primer or a template which contains the mutation(s) of interest. A mutated subfragment can then be assembled into the complete antigenome or genome cDNA. A variety of other mutagenesis techniques are known and can be routinely adapted for use in producing the mutations of interest in a viral antigenome or genome cDNA of the invention.

Thus, in one illustrative embodiment mutations are introduced by using the MUTA-gene® phagemid in vitro mutagenesis kit available from Bio-Rad Laboratories. In brief, cDNA encoding a PIV genome or antigenome is cloned into the plasmid pTZ18U, and used to transform CJ236 cells (Life Technologies). Phagemid preparations are prepared as recommended by the manufacturer. Oligonucleotides are designed for mutagenesis by introduction of an altered nucleotide at the desired position of the genome or antigenome. The plasmid containing the genetically altered genome or antigenome is then amplified.

Certain substitutions, insertions, deletions or rearrangements of genes or genome segments within recombinant non-segmented negative-stranded RNA viruses of the invention (e.g., substitutions of a genome segment encoding a selected protein or protein region, for instance a cytoplasmic tail, transmembrane domain or ectodomain, an epitopic site or region, a binding site or region, an active site or region containing an active site, etc.) are made in structural or functional relation to an existing, "counterpart" gene or genome segment from the same or different virus or other source. Such modifications yield novel recombinants having desired phenotypic changes compared to wild-type or parental viral strains. For example, recombinants of this type may express a chimeric protein having a cytoplasmic tail and/or transmembrane domain of one non-segmented negative-stranded RNA virus fused to an ectodomain of a heterologous virus. Other exemplary recombinants of this type express duplicate protein regions, such as duplicate immunogenic regions.

As used herein, "counterpart" genes, genome segments, proteins or protein regions, are typically from heterologous sources (e.g., from different genes, or representing the same (i.e., homologous or allelic) gene or genome segment in a different viral species or strain). Typical counterparts selected in this context share gross structural features, e.g., each counterpart may encode a comparable protein or protein structural domain, such as a cytoplasmic domain, transmembrane domain, ectodomain, binding site or region, epitopic site or region, etc. Counterpart domains and their encoding genome segments embrace an assemblage of species having a range of size and sequence variations defined by a common biological activity among the domain or genome segment variants.

Counterpart genes and genome segments, as well as other polynucleotides disclosed herein for producing recombinant non-segmented negative-stranded RNA viruses within the invention, often share substantial sequence identity with a selected polynucleotide "reference sequence," e.g., with another selected counterpart sequence. As used herein, a "reference sequence" is a defined sequence used as a basis for sequence comparison, for example, a segment of a full-length cDNA or gene, or a complete cDNA or gene sequence. Generally, a reference sequence is at least 20 nucleotides in length, frequently at least 25 nucleotides in length, and often at least 50 nucleotides in length. Since two polynucleotides may each (1) comprise a sequence (i.e., a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window", as used herein, refers to a conceptual segment of at least 20 contiguous nucleotide positions wherein a polynucleotide sequence may be compared to a reference sequence of at least 20 contiguous nucleotides and wherein the portion of the polynucleotide sequence in the comparison window may comprise additions or deletions (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith & Waterman, (Adv. Appl. Math. 2:482, 1981), by the homology alignment algorithm of Needleman & Wunsch, (J.Mol. Biol. 48:443, 1970), by the search for similarity method of Pearson & Lipman, (Proc. Natl. Acad. Sci. U.S.A. 85:2444, 1988) (each incorporated herein by reference), by computerized implementations of these algorithms (GAP, BESTFIT, FASTA, and TFASTA in the Wisconsin Genetics Software Package Release 7.0, Genetics Computer Group, 575 Science Dr., Madison, WI, incorporated herein by reference), or by inspection, and the best alignment (i.e., resulting in the highest percentage of sequence similarity over the comparison window) generated by the various methods is selected. The term "sequence identity" means that two polynucleotide sequences are identical (i.e., on a nucleotide-by-nucleotide basis) over the window of comparison. The term "percentage of sequence identity" is calculated by comparing two optimally aligned sequences over the window of comparison, determining the number of positions at which the identical nucleic acid base (e.g., A, T, C, G, U, or I) occurs in both sequences to yield the number of matched positions, dividing the number of matched positions by the total number of positions in the window of comparison (i.e., the window size), and multiplying the result by 100 to yield the percentage of sequence identity. The terms "substantial identity" as used herein denotes a characteristic of a polynucleotide sequence, wherein the polynucleotide comprises a sequence that has at least 70 percent sequence identity, often 85 percent sequence identity, at times at least 90 to 95 percent sequence identity, and up to at least 99 percent sequence identity as compared to a reference sequence over a comparison window of at least 20 nucleotide positions, frequently over a window of at least 25-50 nucleotides, wherein the percentage of sequence identity is calculated by comparing the reference sequence to the polynucleotide sequence which may include deletions or additions which total 20 percent or less of the reference sequence over the window of comparison. The reference sequence may be a subset of a larger sequence.

In addition to these polynucleotide sequence relationships, proteins and protein regions encoded by recombinant non-segmented negative-stranded RNA viruses of the invention are also typically selected to have conservative relationships, i.e. to have substantial sequence identity or sequence similarity, with selected reference polypeptides. As applied to polypeptides, the term "sequence identity" means peptides share identical amino acids at corresponding positions. The term "sequence similarity" means peptides have identical or similar amino acids (i.e., conservative substitutions) at corresponding positions. The term "substantial sequence identity" means that two peptide sequences, when optimally aligned, such as by the programs GAP or BESTFIT using default gap weights, share at least 70 percent sequence identity, often 80 percent sequence identity, at times at least 90 percent sequence identity, and up to at least 95 percent sequence identity or more (e.g., 99 percent sequence identity). The term "substantial similarity" means that two peptide sequences share corresponding percentages of sequence similarity. Often, residue positions that are not identical differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Conservative amino acids substitution groups include: valine-leucine-isoleucine, phenylalanine-tyrosine, lysine-arginine, alanine-valine, and asparagine-glutamine. Abbreviations for the twenty naturally occurring amino acids used herein follow conventional usage (Immunology - A Synthesis, 2nd ed., E.S. Golub & D.R. Gren, eds., Sinauer Associates, Sunderland, MA, 1991, incorporated herein by reference). Stereoisomers (e.g., D-amino acids) of the twenty conventional amino acids, unnatural amino acids such as α, α-disubstituted amino acids, N-alkyl amino acids, lactic acid, and other unconventional amino acids may also be suitable components for polypeptides of the present invention. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, ω-N-methylarginine, and other similar amino acids and imino acids (e.g., 4-hydroxyproline). Moreover, amino acids may be modified by glycosylation, phosphorylation and the like.

To select candidate viruses according to the invention, the criteria of viability, attenuation and immunogenicity are determined according to well-known methods. Viruses that will be most desired in immunogenic compositions of the invention must maintain viability, have a stable attenuation phenotype, exhibit replication in an immunized host (albeit at lower levels), and effectively elicit production of an immune response in a recipient sufficient to elicit a desired immune response. The recombinant non-segmented negative-stranded RNA viruses of the invention are not only viable and appropriately attenuated, they are more stable genetically in vivo--retaining the ability to stimulate an immune response and in some instances to expand the immune response elicited by multiple modifications, e.g., induce an immune response against different viral strains or subgroups, or to stimulate a response mediated by a different immunologic basis, e.g., secretory versus serum immunoglobulins, cellular immunity, and the like.

Recombinant non-segmented negative-stranded RNA viruses of the invention can be tested in various well-known and generally accepted in vitro and in vivo models to confirm adequate attenuation, resistance to phenotypic reversion, and immunogenicity. In in vitro assays, the modified virus (e.g., a multiply attenuated, biologically derived or recombinant virus) is tested, e.g., for temperature sensitivity of virus replication, i.e. *ts* phenotype, and for the small plaque or other desired phenotype. Modified viruses are further tested in animal models of viral infection. A variety of animal models have been described and are summarized in various references incorporated herein, including useful rodent and non-human primate models for evaluating attenuation and immunogenic activity of viral candidates for use in immunogenic compositions and methods. Such models are widely accepted in the art, and the data obtained therefrom correlate well with infection, attenuation and immunogenicity of subject viruses of the invention in humans.

In accordance with the foregoing description, the invention also provides isolated, infectious recombinant non-segmented negative-stranded RNA viruses for use in immunogenic compositions. The attenuated virus which is a component of an immunogenic composition is in an isolated and typically purified form. By isolated is meant to refer to a recombinant non-segmented negative-stranded RNA virus which is in other than a native environment of a wild-type virus, such as the nasopharynx of an infected individual. More generally, isolated is meant to include the attenuated virus as a component of a cell culture or other artificial medium where it can be propagated and characterized in a controlled setting. For example, attenuated non-segmented negative-stranded RNA viruses of the invention may be produced by an infected cell culture, separated from the cell culture and added to a stabilizer.

For use in immunogenic compositions, recombinant non-segmented negative-stranded RNA viruses produced according to the present invention can be used directly in formulations, or lyophilized, as desired, using lyophilization protocols well known to the artisan. Lyophilized virus will typically be maintained at about 4°C. When ready for use the lyophilized virus is reconstituted in a stabilizing solution, e.g., saline or comprising SPG, Mg⁺⁺ and HEPES, with or without adjuvant, as further described below.

Immunogenic compositions of the invention contain as an active ingredient an immunogenically effective amount of a recombinant non-segmented negative-stranded RNA virus produced as described herein. The modified virus may be introduced into a host with a physiologically acceptable carrier and/or adjuvant. Useful carriers are well known in the art, and include, e.g., water, buffered water, 0.4% saline, 0.3% glycine, hyaluronic acid and the like. The resulting aqueous solutions may be packaged for use as is, or lyophilized, the lyophilized preparation being combined with a sterile solution prior to administration, as mentioned above. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, and the like. Acceptable adjuvants include incomplete Freund's adjuvant, MPL™ (3-O-deacylated monophosphoryl lipid A; Corixa, Hamilton MT) and IL-12 (Genetics Institute, Cambridge MA), among many other suitable adjuvants well known in the art.

Upon immunization with a recombinant non-segmented negative-stranded RNA virus composition as described herein (e.g., via aerosol, droplet, oral, topical, intramuscular, intranasal, pulmonary, subcutaneous, intravenous, or other route), the immune system of the host responds to the immunogenic composition by producing antibodies specific for immunogenic proteins of the subject virus or viral vector construct. As a result of the immunization with an immunogenically effective amount of a recombinant non-segmented negative-stranded RNA virus produced as described herein, the host becomes at least partially or completely immune to infection by the targeted virus, or resistant to developing moderate or severe infection therefrom.

The host to which the immunogenic compositions are administered can be any mammal which is susceptible to infection by the subject non-segmented negative-stranded RNA virus (or donor virus in the case of chimeric vector constructs) and which host is capable of generating an immune response to the antigens of the immunizing virus. Accordingly, the invention provides methods for creating immunogenic compositions for a variety of human and veterinary uses.

The compositions containing the recombinant non-segmented negative-stranded RNA viruses of the invention are administered to a host susceptible to or otherwise at risk for infection by the subject virus (or donor virus) to enhance the host's own immune response capabilities. Such an amount is defmed to be a "immunogenically effective dose." In this use, the precise amount of recombinant virus to be administered within an effective dose will depend on the host's state of health and weight, the mode of administration, the nature of the formulation, etc., but will generally range from about 10³ to about 10⁷ plaque forming units (PFU) or more of virus per host, more commonly from about 10⁴ to 10⁶ PFU virus per host. In any event, the formulations should provide a quantity of modified virus of the invention sufficient to elicit a detectable immune response in the host patient against the subject pathogen(s), e.g., as can be determined by hemagglutination inhibition, complement fixation, plaque neutralization, and/or enzyme-linked immunosorbent assay, among other methods.

The recombinant non-segmented negative-stranded RNA viruses produced in accordance with the present invention can be combined with viruses of other serotypes or strains to elicit a desired immune response against multiple viral serotypes or strains. Alternatively, an immune response against multiple viral serotypes or strains can be achieved by combining protective epitopes of multiple serotypes or strains engineered into one virus, as described herein. Typically when different viruses are administered they will be in admixture and administered simultaneously, but they may also be administered separately. Immunization with one strain may immunize against different strains of the same or different serotype.

In neonates and infants, multiple administrations may be required to elicit sufficient levels of immunity. Administration may begin within the first month of life, and at intervals throughout childhood, such as at two months, six months, one year and two years, as necessary to maintain an immune response against native (wild-type) viral infection. Similarly, adults who are particularly susceptible to repeated or serious infection, such as, for example, health care workers, day care workers, family members of young children, the elderly, individuals with compromised cardiopulmonary function, may require multiple immunizations to establish and/or maintain immune responses. Levels of induced immunity can be monitored by measuring amounts of neutralizing secretory and serum antibodies, and dosages adjusted or immunizations repeated as necessary to maintain desired levels of immune response. Further, different candidate viruses may be indicated for administration to different recipient groups.

In yet another aspect of the invention recombinant non-segmented negative-stranded RNA viruses are employed as vectors for transient gene therapy, e.g., of the respiratory tract. According to this embodiment the recombinant viral genome or antigenome incorporates a sequence that is capable of encoding a gene product of interest. The gene product of interest is under control of the same or a different promoter from that which controls viral expression. The infectious recombinant virus containing a sequence encoding the gene product of interest is administered by one or more routes of administration as described above. The virus is administered in an amount sufficient to result in the expression of therapeutic or prophylactic levels of the desired gene product. Representative gene products that may be incorporated in the recombinant virus and administered within this method are typically suitable for transient expression, including, for example, interleukin-2, interleukin-4, gamma-interferon, GM-CSF, G-CSF, erythropoietin, and other cytokines, glucocerebrosidase, phenylalanine hydroxylase, cystic fibrosis transmembrane conductance regulator (CFTR), hypoxanthine-guanine phosphoribosyl transferase, cytotoxins, tumor suppressor genes, antisense RNAs, and other candidate antigens.

The following examples are provided by way of illustration, not limitation.

All patents and publications cited herein are hereby incorporated by reference.

### Example I

### General Calcium-Phosphate Transfection Protocol for Rescue of Non-Segmented Negative Stranded RNA Viruses From Vero Cells

### Solutions

The following solutions are generally useful for host cell transfection:
A 2XBBS (per L) solution (2XBES-buffered saline) of 280 mM NaCl [16.4 g NaCl (or 56 ml 5MNaCl)], 50 mM BES [10.7 g BES (free acid form)], and 1.5 mM sodium phosphate [0.21 g Na₂HPO₄]. The BBS solution is adjusted to pH 6.95-6.98 with NaOH. The solution is then filter-sterilized and stored frozen.

A 2.5 M CaCl₂ solution of 36.8 g per 100 ml total volume is prepared and stored at - 20°C. The solution is filter-sterilized using nitrocellulose. Cellulose acetate filters are to be avoided because they clog. Alternatively, the transfection solutions are autoclaved for sterilization. However, the latter procedure may be less desirable, because the 2XBBS solution may change slightly during autoclaving.

The following solutions are generally useful for the medium:
A DMEM+FBS solution of DMEM (high glucose with glutamine; Gibco/BRL, [Grand Island, NY]), supplemented with 10% heat-inactivated and certified FBS, and 10-20µg/ml (optionally up to 50 µg/ml) gentamicin.

[An MEM+FBS solution of MEM (supplemented with glutamine, nonessential amino acids, 10% heat-inactivated and certified FBS, and 10-20µg/ml (optionally up to 50 µg/ml) 20-25mM Hepes buffer; Gibco/BRL) (Grand Island, NY) and optionally including 1X Fungizone).

An HBS solution of Hepes-buffered saline wash solution, 20mM hepes, pH 7.0,150 mM NaCl, 1mM MgCl₂.

### Methods

A generally useful host cell can be selected from split Vero cells, which are placed in DMEM+FBS the day before transfection so they will be approximately 50% confluent [80-90% for RSV] the following day (in six-well plates or 12.5 cm² flasks). Higher cell densities work less effectively. The following day, each culture is fed 1-4 hours before transfection with 4.5 ml of DMEM+FBS. The cells are then transferred to a CO₂ incubator set at 3% CO₂ and 32°C. The Vero cells can be grown longer than overnight as long as they are approximately 50% confluent at the time of transfection.

A CaCl₂/phosphate precipitate is obtained as follows: BBS and CaCl₂ were maintained at room temperature before starting. The DNA mix is prepared in a 5ml polypropylene tube containing a total volume of 250 µl, with plasmid DNA 2-20 µg total, and 25 µl CaCl₂. The DNAs for full-length rescue include 5µg of a full-length cDNA construct for the selected non-segmented negative-stranded RNA virus, 400 ng N protein, 300 ng P protein, 100-200 ng L protein, 200 ng M2 (for RSV only) protein, and 5-10µg pCl-Neo-Bcl-T7 plasmid (SEQ ID NO: 1; Figure 2). The efficiency of rescue in Vero cells is low, so 3-6 wells are transfected per full-length construct being rescued.

After all DNAlCaCl₂ solutions are prepared, 2XBBS is added. This is usually done by gently agitating a tube by continuous low-speed vortexing and adding 250 µl of 2XBBS dropwise down the side of the tube. This is repeated for all tubes, which are allowed to stand at room temperature for an additional 15-20 minutes to permit the DNA-Calcium-Phosphate precipitate to form. After the room temperature incubation, the precipitate is added dropwise to the cell culture medium and distributed evenly by rocking the plate. The medium is then incubated for three hours in an incubator set at 3% CO₂. A level of 3% CO₂ is important for the BBS/CaCl₂ transfection technique; 5% CO₂ works very poorly, if at all. The 3% CO₂ controls the pH of the medium and allows formation of an effective calcium-phosphate-DNA precipitate in the medium.

A heat shock procedure is then optionally carried out, for example at three hours after initiation of transfection. The cells are transferred to a water bath set at 44°C. The cells are sealed in a plastic storage bag so the cultures can be fully submersed in water. After three hours at 44°C, the cells are transferred back to a 32°C incubator set at 3% CO₂ and the incubation is continued overnight.

On the following day, the transfection medium is removed and the cells are washed two times with HBS. After washing, 2 ml of fresh DMEM+FBS are added. PBS and Hank's buffer works poorly for the wash step, probably because the phosphate in these buffers causes more CaCl₂ to precipitate out of the transfection medium.

A co-culture procedure is then optionally performed. The transfected cells are harvested at 48-72 hours post-transfection by scraping them into the medium and transferring the cells plus medium to a T25 flask that contained a 50% confluent monolayer of Vero cells. Six hours after initiating this co-culture, the medium is replaced with 4 ml of MEM+FBS. The cultures are then incubated for five days. If the medium begins to appear exhausted during this incubation period, 2 ml of media are removed and replaced with fresh MEM+FBS. It is not recommended that all of the media be replaced, in order to conserve any small amount of virus being generated during rescue which may be in the media. During this co-culture phase, CPE may become evident, but this is usually not the case. If no CPE is evident, the rescue can be continued.

The cells are harvested five days after initiating the co-culture. First, 0.5 ml of 2.18M Sucrose, 37.6 mM KH₂PO₄, 71.0 mM K₂HPO₄, 49.0 mM sodium glutamate are added to the medium and mixed by rocking the flask. Then the cells are scraped into the medium, pipetted up-and-down to mix, and then aliquoted into freezer tubes for shipping and then quick-frozen in a dry-ice/ethanol bath and stored at -80°C.

### Example II

### General Calcium-Phosphate Transfection Protocol for Rescue of Paramyxoviruses

The following alternative transfection procedure uses a calcium-phosphate transfection protocol based on the methods of Chen and Okayama, Mol. Cell. Biol. 7:2745-2752, 1987, incorporated herein by reference.

### Exemplary DNAs:

1. A full-length viral genomic cDNA clone containing a T7 promoter fused to the 5' end of the positive-sense strand and a ribozyme fused to the 3' end.
2. Protein expression plasmids, controlled by a T7 RNA polymerase promoter, that encode the nucleocapsid protein (N or NP), phosphoprotein protein (P), and polymerase protein (L).
3. A plasmid encoding the T7 RNA polymerase protein under control of the human cytomegalovirus immediate early transcriptional control region

### Exemplary calcium-phosphate transfection reagents:

1. 2X BES-buffered saline: 50 mM BES (pH 6.95-6.98), 280 mM NaCl, 1.5mM Na₂HPO₄.
2. 2.5 M CaCl₂.
3. Hepes-buffered saline wash solution (HBS)

### Exemplary Protocol:

1. Culture HEp2, Vero or other suitable cell line in Dulbecco's modified Eagle's medium (DMEM) containing 10% fetal bovine serum (FBS) in six-well plates to a cell density of approximately 50-75% confluence.
2. 1-2 hours prior to transfection, feed each well to be transfected with 4.5 ml of Dulbecco's modified Eagle's medium (DMEM) containing 10% fetal bovine serum (FBS).
3. Incubate the cells in a 32°C incubator set at 3% CO₂.
4. Prepare the calcium-phosphate-DNA transfection mixture as follows*:
5. Combine the following DNAs in a 5ml polypropylene tube: 400 ng T7-N, 300 ng T7-P, 100 ng T7 L, and 5 µg of viral genomic cDNA clone, 5µg of hCMV-T7 transient expression vector (e.g., pCl-Neo-Bcl-T7 plasmid). For RSV rescue, also include 100 ng of T7-M2 (polymerase elongation factor) expression plasmid.
6. Adjust the volume to 225 µl with water.
7. Add 25 µl 2.5M CaCl₂.
8. While gently vortexing the tube, add 250 µl of 2XBBS then allow the tube to stand at room temperature for 15-20 minutes.
   *Note that the amounts of each plasmid DNA may need to be optimized to achieve rescue. Additionally, although the calcium-phosphate transfection protocols of Examples 1 and 2 are broadly useful within the invention, alternate transfection protocols can also be successfully employed.
9. Remove the cells from the incubator and slowly add the calcium-phosphate-DNA mixture to the culture medium while gently swirling the medium. Immediately return the cells to the 32°C-3%CO₂ incubator.
10. Three hours after starting the transfection experiment, seal the culture dishes in a plastic bag and fully submerse in a water bath set at 44°C for three hours to induce the cellular heat shock response. The optional heat shock step often enhances the results of rescue. However, different cell types will respond better to different effective heat shock temperatures (e.g., within a range of about 40-45°C) or for different effective heat shock periods (e.g., within a range of about 30 min to 3 hours).
11. After heat shock, return the cells to the 32°C-3% CO₂ incubator and continue incubation overnight.
12. The following day, wash the cells 2 times with HBS and feed the cells with 2ml of DMEM or minimal essential medium (MEM) supplemented with 10% FBS.
   At this stage the protocol varies for some strains of PIV that require trypsin to promote the spread of virus. At 48 hours, the cells are washed with PBS to remove residual serum and the medium is replaced with serum-free medium containing approximately 0.5 µg
13. At 48 hours after initiating transfection, seed 10cm² plates with sufficient Vero cells to produce 50% confluent monolayers the following day (72 hours after the start of transfection). Prepare one 10cm² plate for each transfected culture.
14. 72 hours after transfection, scrape the transfected cells into the medium and transfer the cells plus medium to a 10 cm plate prepared the day before. This initiates a coculture between the transfected cells and the monolayer contained in the 10cm plate. Incubate the plate in a 37°C incubator set at 5% CO2; use an appropriate lower temperature for temperature-sensitive mutant viruses.
15. Four to 6 hours later, replace the medium with fresh DMEM supplemented with 10% FBS.
16. Monitor the cells for 5-7 days to detect evidence of cytopathic effect.

### Example III

### Rescue of Measles Virus (MV) in Vero Cells

The exemplary transfection protocol described in Example 1, above, was used to rescue measles virus in Vero cells. The T7 source was either plasmid pSC6-T7 (without MVA) or MVA/T7 (Wyatt et al 1995). The results indicating numerous successful rescues (+) of MV are shown below in Table 3 (Columns A-F represent different rescue experiments performed on different days, with each experiment represented by multiple transfections--up to twelve separate transfections (wells) for experiments E and F).

**Table 3 Rescue of Measles Virus with Plasmid-T7 or MVA/T7**

| | **Experiments** | | | | | | |
|---|---|---|---|---|---|---|---|
| **Well** | **A** | **B** | **C** | **D** | **E** | **F** | **T7 Source** |
| 1 | + | + | - | - | - | + | MVA/T7 |
| 2 | - | + | - | - | + | + | MVA/T7 |
| 3 | + | - | - | + | - | + | MVA/T7 |
| 4 | - | - | - | - | - | + | MVA/T7 |
| 5 | + | + | - | - | - | + | MVA/T7 |
| 6 | - | + | - | - | - | - | MVA/T7 |
| 7 | | | | | - | + | pSC6-T7 |
| 8 | | | | | + | + | pSC6-T7 |
| 9 | | | | | - | + | pSC6-T7 |
| 10 | | | | | - | + | pSC6-T7 |
| 11 | | | | | - | + | pSC6-T7 |
| 12 | | | | | - | + | pSC6-T7 |

### Example IV

### Rescue of Measles Virus (MV) Using Different Polymerase Sources

The instant example demonstrates that a transient T7 polymerase expression vector according to the methods herein provides sufficient levels of T7 RNA polymerase expression to support virus rescue. Cells were transfected using the protocol of Example I, above. When rescue was performed with the T7 RNA polymerase expression plasmid, the DNA was cotransfected along with the virus-specific plasmids. When infection with MVA-T7 (Wyatt et al, Virology 210:202-5, 1995) was used to provide T7 RNA polymerase, infection was performed simultaneously with transfection by adding virus to the medium at a multiplicity of infection of 2. The T7 expression plasmid (pSC6-T7) used in this experiment was previously described by Billeter et al., (Radeke et al, EMBO J. 14:5773-5784, 1995). It contained the T7 RNA polymerase gene under control of the hCMV transcriptional control region. The transient expression plasmid pCI-Neo-Bcl-T7 (SEQ ID NO: 1; Figure 2) was used in all subsequent viral rescue procedures presented herein. The viral genomic clone used in the instant example was p(+)MV that contains the cDNA sequence corresponding to the Edmonston lab strain of measles virus (Radecke et al., EMBO J. 14:5773-5784, 1995).

The positive results of the rescue of measles virus are shown in Table 4, below.

**Table 4 Rescue of Measles Virus with Plasmid-T7 or MVA/T7**

| Experiment | Wells transfected | Wells producing measles virus | Source of T7 RNA polymerase expression |
|---|---|---|---|
| #1 | 6 | 1 | MVA-T7 |
| | 6 | 1 | pSC6-T7 |
| #2 | 6 | 5 | MVA-T7 |
| | 6 | 6 | pSC6-T7 |

### Example V

### Rescue of Recombinant Parainfluenza Viruses (PIVs)

Using the T7 plasmid rescue system described in Example II above, a chimeric bovine-human PIV-3 (HBPIV3) strain was rescued in Vero cells. This exemplary PIV clone contains human PIV-3 F and HN genes as replacements in a bovine PIV-3 vector or backbone. Five of five independent rescue samples were positive as determined by a standard hemagglutination (fHAD) assay, which was performed by incubating the infected cells with guinea pig red blood cells (RBCs) to determine the presence of recombinant virus. The results were further confirmed by standard RT-PCR across the M/F gene junction to confirm that the recombinant virus was the expected human-bovine hybrid. A passage-2 virus stock was grown for RT-PCR purposes. The titers on that stock in Vero cells were 5-10 x 10⁷ pfu/ml.

In addition to the HBPIV3 chimeric virus, several other recombinant PIV constructs were rescued in accordance with the invention, as partially summarized in Table 5, below. In one exemplary embodiment, an attenuated, chimeric recombinant PIV strain PIV3cp45(3-1) (containing cold passage mutations of HPIV3 cp45 and the PIV1 F and G glycoprotein genes in place of the corresponding PIV3 genes) was rescued from five transfected Vero cell cultures that were then used to infect fresh cell monolayers. After virus adsorption, the cells were maintained in 0.5 µg trypsin per ml of serum-free medium for five days. The infected cultures were then subjected to a hemagglutination assay, and positive rescue results confirmed by absorption of RBCs by the cells infected with the recombinant PIV.

**Table 5**

| Virus | Description |
|---|---|
| PIV3cp45-456 | PIV3 containing attenuating mutations in the L gene and mutations responsible for the cold passage phenotype |
| PIV3cp45(3-1) | PIV3 containing the cold passage mutations and the PIV1 glycoprotein genes in place of the corresponding PIV3 genes |
| PIV3-2CT | PIV3 containing chimeric PIV2 glycoprotein genes. The cytoplasmic domains are encoded by PIV3 sequences and the transmembrane and extracellular domains are from PIV2 |
| HBPIV3 | bovine PIV containing the hPIV3 glycoprotein genes in place of the homologous bovine genes |

In another exemplary embodiment, a HPIV3-HPIV-2 chimeric construct was recovered containing chimeric glycoproteins with the HPIV2 F and HN transmembrane and extracellular domains fused to the HPIV3 cytoplasmic domains (designated PIV3-2CT), as described above. This chimeric construct was recovered from Vero cells and the recombinant virus identity was confirmed by RT-PCR and subsequent restriction site analysis.

Additional PIV recombinants that were recovered according to the methods of the invention include a BPIV strain designated "Shipping Fever" (SF) (Reisinger et al., J. Am. Vet. Med. Assoc. 135:147-152, 1959, incorporated herein by reference), and a recombinant HPIV containing attenuating mutations in the L gene and mutations responsible for a cold passage phenotype, designated PIV3cp45-456. Both were rescued in Vero cells, and their identity confirmed by RT-PCR and sequencing. PIV3cp45-456 has two mutations corrected in the HN gene from a previously-described PIV3cp45 recombinant that had two introduced mutations in HN, and thus departed from the sequence of the biologically-derived cp45 mutant. The PIV3cp45-456 clone corrects these two mutations that substituted Ile at residue 263, and Thr at residue 370, back to the biologically-derived genotype encoding Thr and Pro, respectively.

### Example VI

### Rescue of Vesicular Stomatitis Viruses in 293 Cells

Refinement of the transient RNA polymerase vector system of the invention allows for rescue of a broad repertoire of recombinant negative-strand RNA viruses for use in immunogenic compositions. In the following examples this technique is optimized for recovery of vesicular stomatitis virus (VSV) and related VSV vector strains. Although most VSV strains replicate very efficiently in a wide range of cell lines, rescue of recombinant VSV has heretofore proven to be very difficult. VSV rescue procedures described in the literature specify use of the BHK cell line and a vaccinia virus/T7 helper-virus, both of which are not desired for use in a process that will be used to produce immunogenic compositions for human clinical use.

Attempts to rescue recombinant VSV from qualified Vero cells were initially unsuccessful when a combined heat shock/plasmid T7 rescue protocol was used (as described above for rescue of parainfluenza virus, and measles virus). One of the factors considered limiting in this system was the poor transfection efficiency, particularly associated with Vero cells. This problem was addressed by two different modifications of the heat shock/plasmid T7 rescue system. In one modification, the calcium-phosphate transfection/heat shock methodology was largely unchanged, but a different host cell type was employed. Alternatively, a different transfection protocol was employed to optimize the rescue system for Vero Cells (see Example VII).

Briefly, successful rescue of VSV from 293 cells was achieved using the basic heat shock/plasmid-T7 system above, according to the following revised protocol.

### REAGENTS

### Plasmid DNAs:

1. Full-length viral genomic cDNA
2. pT7-N
3. pT7-P
4. pT7-L
5. pT7-M
6. pT7-G
7. pCI-Neo-bcl-T7 (p0061)

### Calcium -phosphate transfection reagents:

1. 2X BES-buffered saline: 50 mM BES (pH 6.95-6.98), 280 mM NaCl, 1.5mM Na2HPO4.
2. 2.5 M CaCl2.
3. Hepes-buffered saline wash solution (HBS): 20 mM hepes (pH7.0-7.5), 140 mM KCl, 1 mM MgCl2.

### Cell Culture Solutions:

1. DMEM supplemented with 10% certified, heat-inactivated FBS (DMEM/FBS).
2. Iscoves Modified Minimal Essential Medium (IMEM) supplemented with 10% certified, heat-inactivated FBS (IMEM/FBS).
3. Poly-L-Lysine: 0.01% in H20.
4. PBS.
5. Porcine trypsin/EDTA.

### PROCEDURES

### 293 Cell Culture:

293 cells can be difficult to culture, and there are number of different methods to handle them. The current method has been used successfully as part of a rescue system for VSV and modified VSV vector constructs.

### Routine Subculturing:

1. Remove medium and wash the confluent monolayer (10 cm plate) with 5 ml of warm PBS; Pipet gently along the side of the dish to prevent detachment of the cells (293 cells left at room temperature for too long, or in media that become basic (red), will detach).
2. Gently add 2 ml of trypsin and rock the plate to cover the entire monolayer.
   Aspirate the trypsin and allow the plate to stand at room temperature for about a minute. Tilt the plate on a 45-degree angle and tap it against the working surface of the hood to detach the cells. If the cells do not detach, incubate another minute at room temperature (make sure the cells detach at this stage so that vigorous pipeting can be avoided).
3. Gently add 5 ml of DMEM/FBS and pipet up and down slowly to disperse the cells.
4. Add 1 ml of cells to a plate containing 9 ml of DMEM/FBS.
5. Incubate at 37°C, 5% CO₂.

### Subculture for transfection:

1. Coat the desired number of plates with poly-L lysine. Add about 3-4 ml of 0.001 % poly-L lysine per plate and allow it to stand at room temperature for at least 30 minutes. Aspirate the poly-L lysine solution. Rinse the plate with 5 ml of medium.
2. Trypsinize the cells as described above. Use a split ratio that will yield a 50-75% confluent plate the following day (1:3 to 1:6).
3. After detaching the cells add IMEM/FBS and transfer the cells to the coated plate containing 9 ml of IMEM/FBS. It seems to be important to split the cells and allow overnight growth in IMEM/FBS before transfection.
4. Incubate at 37°C, 5% CO₂

### Transfection:

1. 1-3 hrs prior to transfection, feed the cells with 9 ml of IMEM/FBS and incubate the cells in a 32°C incubator set at 3% CO₂
2. Prepare the calcium-phosphate-DNA transfection mixture as follows:
   a. Combine the following DNAs in a 5ml polypropylene tube:
      i. 8 µg T7-N
      ii. 4 µg T7-P
      iii. 1.2 µg T7 L
      iv. 1.0 µg T7-M
      v. 1.0 µg T7-G
      vi, 10µg of viral genomic cDNA clone.
      vii. 10µg of hCMV-T7 expression vector.
   b. Adjust the volume to a final volume of 450 µl with water.
   c. Add 50 µl 2.5M CaCl₂.
   d. While gently vortexing the tube, add 500 µl of 2XBBS then allow the tube to stand at room temperature for 15-20 minutes.
3. Remove the cells from the incubator and slowly add the calcium-phosphate-DNA mixture to the culture medium and swirl gently to distribute the precipitate. Immediately return the cells to the 32°C-3%CO₂ incubator.
4. Three hours after initiating transfection, seal the culture dishes in a plastic bag and fully submerse in a water bath set at 43°C for 2 hours to induce the cellular heat shock response.
5. After heat shock, return the cells to the 32°C-3% CO₂ incubator and continue incubation overnight.
6. The following day, wash the cells 2 times with HBS and feed the cells with 10ml of IMEM/FBS. Incubate at 37°C, 5% CO₂.
7. At 48-72 hours after initiating transfection, set up sufficient T150 flasks containing 20 ml DMEM/FBS for transfer oftransfected cells to the larger vessel. One T150 flask for every 10cm plate that was transfected.
8. Transfer the transfected 293 cells by gently pipeting the culture medium over the monolayer to dislodge it from the cell surface. Avoid vigorous pipeting and use just enough force to dislodge the cells. After the cells are dislodged, pipet up and down about 5 times to reduce the size of the cell clumps then transfer the medium and cells to a T150 flask containing the 20 ml of IMEM/FBS.
9. Four to 6 hours later, replace the medium with fresh DMEM supplemented with 10% FBS (note that this step can be delayed until 24 hours if the cells are not adhering to the plate. Also, this step has been skipped successfully).
10. Monitor the cells for 5-7 days to detect evidence of cytopathic effect.
11. When CPE appears evident, transfer 50 ul of medium supernatant to well in a six well plate that contains medium and an established Vero cell monolayer. CPE should be visible the following day if rescue has occurred. (Note that this step is important because the 293 cells do at times detach from the surface of the T150 flask and appear VSV-infected when they actually are not).
12. After transferring the small sample to the Vero cell monolayer, harvest the cells and medium from the T150 flask and freeze at-70. The 293 cells can generally be harvested by pipeting the medium over the monolayer to detach the cells.

Using the revised protocol set forth above, various recombinant VSV constructs were recovered from 293 cells. Successfully recovered VSV constructs in this context included a basic VSV vector backbone construct, and two modified VSV vectors containing the HIV envelope gene, or the HIV gag gene, as described herein. Thus, it is clear that the transient RNA polymerase vector system of the invention is adaptable for a range of different recombinant viruses, including substantially modified (e.g., attenuated and vector) constructs, in different cell types. In particular, it is clear that 293 cells can support VSV rescue, and that improved transfection efficiency is likely important to achieving VSV rescue in other cells (e.g., Vero cells).

It is noteworthy that additional VSV and measles virus rescue experiments were performed with a further modified procedure involving addition of expression plasmids encoding selected viral structural genes. In the case of VSV rescue, plasmids that express the G and M genes were co-transfected into the host cells (as described in the modified protocol above). For MV rescue, the transfection mixture was supplemented with plasmids encoding M, F, and H. It is therefore contemplated that rescue of any of the non-segmented negative-stranded RNA viruses according to the invention may optionally include co-introduction and/or co-expression of any one, or any combination, of structural proteins, including proteins of the same virus or of a heterologous species, subtype or strain.

### Example VII

### Rescue of Vesicular Stomatitis Viruses in Vero Cells Via Alternate, Electroporation-Mediated Transfection

To further assess the role of transfection efficiency in the transient RNA polymerase expression system of the invention, electroporation was investigated as an alternative to calcium-phosphate transfection of Vero cells. Conditions for electroporation of Vero cells were determined and then incorporated into a modified heat shock/plasmid-T7 rescue protocol, detailed below.

### DNA Preparation:

1. For each electroporation, combine the following plasmid DNAs in a microfuge tube:
   50 µg plasmid expressing T7 (pCI-Neo-Bcl-T7).
   0 µg VSV Full Length plasmid.
   8 µg N plasmid.
   4 µg P plasmid.
   1 µg L plasmid.
   1 µg M plasmid (for VSV).
   1 µg G plasmid (for VSV).
   Also prepare DNAs for a Luciferase transfection control:
   50 µg (pCI-Neo-Bcl-T7).
   5 µg T7-Luciferase plasmid.
      a) While working in a biosafety hood, adjust the DNA volume to 250 µl with sterile, nuclease-free water.
      b) Add 50 µl of 3M Sodium Acetate (pH 5).
      c) Add 750 µl of 100% Ethanol and mix.
      d) Incubate at -20°C for 1 hour to overnight
      e) Pellet the DNA in a microfuge at 14,000 rpm, 4°C for 20 minutes
      f) Discard supernatant, while working in a biosafety hood, without disturbing the pellet.
      g) Quick-spin the tube to collect residual Ethanol at bottom and remove with pipet
      h) Let pellet air dry (in biosafety hood) for 5-10 minutes.
      i) Resuspend pellet with 50 µl of sterile, nuclease-free water.

### Solutions:

Trypsin/EDTA
Hank's buffered saline
1 mg per ml soybean trypsin inhibitor prepared in PBS
Media components (below)

Media

| Medium 1 | Medium 2 | Medium 3 |
|---|---|---|
| Dulbecco's modified minimal essential medium (DMEM) | Iscove's modified Dulbecco's medium (IMDM) | Dulbecco's modified minimal essential medium (DMEM) |
| 10% heat-inactivated fetal bovine serum | 220 uM 2-mercaptoethanol (tissue culture grade) | 10% heat-inactivated fetal bovine serum |
| 220 uM 2-mercaptoethanol (tissue culture grade) | 1% DMSO (tissue culture grade) | 220 uM 2-mercaptoethanol (tissue culture grade) |
| 1% Nonessential amino acids (10mM solution) | 1% Nonessential amino acids (10mM solution) | 1% Nonessential amino acids (10mM solution) |
| 1% sodium pyruvate (100 mM solution) | 1 % sodium pyruvate (100 mM solution) | 1 % sodium pyruvate (100 mM solution) |
| | | 50 ug/ml gentamicin |

### Preparation of Vero Cells:

Each electroporation requires cells from approximately 1.3 near-confluent T150 flasks.
1. Wash cell monolayer 1x with approximately 5 ml of Hank's Buffered Saline Solution.
2. Add 4ml trypsin/EDTA and rock the flask to evenly distribute the solution.
3. Incubate at room temperature 3-5 min.
4. Aspirate the trypsin solution.
5. Rap the sides of the flask to dislodge cells.
6. Use 10 ml of Media #1 to collect cells from flask(s) and transfer cells to a 50 ml conical tube.
7. Add 1 ml of trypsin inhibitor (1 mg/ml) and mix gently.
8. Centrifuge cells at 1000 rpm in a room temperature table-top centrifuge tube at 1000 rpm for 5 min.
9. Aspirate the supernatant.
10. Resuspend the cells in 10 ml of Medium 2.
11. Add 1ml of trypsin inhibitor and mix gently.
12. Collect cells at 1000 rpm in a room temperature table-top centrifuge as described above.
13. Aspirate medium.
14. Resuspend cells in medium 2. Gently resuspend in a final volume that will be equivalent to 0.8 ml per electroporation (take into account that the cell pellet will contribute volume).
15. Transfer 0.8ml of cell suspension to the centrifuge tube containing the plasmid DNA solution. Gently pipet up and down with a p1000 tip to mix the cells and DNA. Move to the electroporator station.

### Electroporation:

BTX 820 Square-Wave Electroporator
1. Pipet the cells/DNA up and down 3 times to mix gently and transfer to an electroporation cuvette.
2. Insert cuvette in electroporation stand.
3. Electroporate the cells (4 pulses, 70 msec, 140 Volts).
4. After electroporating all samples, return the cuvette(s) to the biosafety hood and add 1 ml of medium 1. Using a sterile plastic transfer pipet, transfer the cells from the cuvette to a 15 ml centrifuge tube containing 10 ml of medium 1.
5. Mix gently and collect the cells by centrifugation as described above.
6. spirate the medium.
7. esuspend the cells in 10 ml medium 1 and transfer the suspension to a T150 flask that contains 25 ml medium 1.
8. Incubate the cells at 37° C (5% CO₂) for 3 hours.
9. Heat shock the cells at 43°C (3-5% CO₂) for 3 hours.
10. eturn cells to 37°C for 2 hours.
11. Replace the medium with 30 ml of medium 3, and continue incubation at 37°C.
12. Inspect cells daily for signs of cytopathic effect. VSV replication should be evident as early as 3-4 days but may take as long as 6.

In accordance with the foregoing electroporation/heat shock/T7 plasmid procedure, recombinant VSV, MV and PIV were all successfully rescued from Vero cells. Notably, several successful VSV rescues were performed using a modified PCl-Neo-Bcl-T7 plasmid containing a one amino acid substitution that reportedly reduces premature termination (MacDonald et al., J. Mol. Biol. 238:145-158, 1994, incorporated herein by reference).

### Example VIII

### Rescue of Respiratory Syncytial Virus in Vero Cells

The present example illustrates successful recovery of fifty different recombinant RSV strains rescued using the transient RNA polymerase vector system of the invention (exemplified by the modified calcium phosphate/heat shock protocol set forth in Example II, above). The broad assemblage of recombinant RSV candidates thus recovered is illustrated in Table 6: including RSV recombinants modified by incorporation of one or more attenuating mutations from biologically derived RSV mutants; RSV modified by a partial or complete deletion of one or more gene(s) or genome segment(s) (e.g., of SH, NS1, NS2, M2-2) or other modification that knocks out or impairs expression of one or more gene(s); RSV-RSV chimeric constructs (e.g., RSV A2 strain background with RSV B glycoprotein(s) substituted therein); RSV with one or more gene insert(s); and RSV recombinants with one or more gene(s) positionally shifted relative to a corresponding gene position in a wild-type RSV gene map (i.e., by "gene shuffling"). The rescued strains identified in Table 6 primarily include variants and derivatives of the RSV A2 strain, but an RSV B 1 strain was also recovered successfully using the methods and compositions of the invention. Among the recovered RSV recombinants set forth in Table 6, various attenuated, multiply-attenuated, and chimeric viruses were recovered, as well as RSV recombinants having one or more gene deletions, all of which are described above and in the above-incorporated references.

**Table 6 RSV Rescued cDNA Clones**

| | **WV cDNA Construct/Virus (description)** | **Rescued** | | **Virus Sequence confirmed** † |
|---|---|---|---|---|
| | | MVA method* | Plasmid method | |
| 1 | rA2cp ΔSH M2/404 L248/404 (A2 backbone, cp mutation, deletion of SH gene, 404 mutation in M2 gene, 248 and 404 mutations in L gene) | Yes | Yes | - |
| 2 | rA2cp ΔSHM2/404 L248/1030 (A2 backbone, cp mutation, deletion of SH gene, 404 mutation in M2 gene, 248 and 1030 mutations in L gene) | - | Yes | - |
| 3 | rA2cp ΔSH M2/404 L1030 (A2 backbone, cp mutations, deletion of SH gene, 404 mutation in M2 gene, 1030 mutation in L gene | - | Yes | - |
| 4 | rA2cp ΔSH M2/404 (A2 backbone, cp mutations, deletion of SH gene, 404 mutation in M2 gene) | - | Yes | - |
| 5 | rA2cp ASH M2/404 L248 (A2 backbone, cp mutations, deletion of SH gene, 404 mutation in M2 gene, 248 mutation in L gene) | - | Yes | - |
| 6 | rA2cp ASH (A2 backbone, cp mutations, deletion of SH gene) | - | Yes | - |
| 7 | rA2cp ΔSH L248 (A2 backbone, cp mutations, deletion of SH gene, 248 mutation in L gene) | - | Yes | - |
| 8 | rA2cp ΔSH L404 (A2 backbone, cp mutations, deletion of SH gene, 404 mutation in L gene) | - | Yes | - |
| 9 | rA2cp ASH L248/1030 (A2 backbone, cp mutations, deletion of SH gene, 248 and 1030 mutations in L gene) | - | Yes | - |
| 10 | rA2cp ΔSH L1030 (A2 backbone, cp mutations, deletion of SH gene, 1030 mutation in L gene) | - | Yes | - |
| 11 | rA2cp ΔSH L/248/404 (A2 backbone, cp mutations, deletion of SH gene, 248 and 404 mutations in L gene) | - | Yes | - |
| 12 | rA2cp ASH L/404/1030 (A2 backbone, cp mutations, deletion of SH gene, 404 and 1030 mutations in L gene) | - | Yes | - |
| 13 | rA2cp M2/404 L248/404/1030 (A2 backbone, cp mutations, 404 mutation in M2 gene, 248, 404 and 1030 mutations in L gene) | Yes | Yes | - |
| 14 | rA2cpΔSH/404/M2/L248 (A2 backbone, cp mutations, deletion of SH gene, 404 mutation in M2 gene, 248 mutation in L gene) | - | Yes | - |
| 15 | rA2cpASH M2/404 L404 (A2 backbone, cp mutations, deletion of SH gene, 404 mutation in M2 gene, 404 mutation in L gene) | - | Yes | - |
| 16 | rA2cpΔSH M2/404 L404/1030 (A2 backbone, cp mutations, deletion of SH gene, 404 mutation in M2 gene, 404 and 1030 mutations in L gene) | - | Yes | - |
| 17 | rA2cpΔSH L248/404/1030 (A2 backbone, cp mutations, deletion of SH gene, 248, 404, and 1030 mutations in L gene) | - | Yes | Yes |
| 18 | rA2cpΔSHcpFstb L248/404/1030 (A2 backbone, cp mutations, deletion of SH gene, cp mutation in F stabilized, 248, 404, and 1030 mutations in L gene) | Yes | Yes | - |
| 19 | rA2cp M2/404 L530/1009 (A2 backbone, cp mutations, deletion of SH gene, 404 in M2 gene, 530 and 1009 mutations in L gene) | Yes | Yes | - |
| 20 | rA2cp ΔNS2 M2/404 L530/1009 (A2 backbone, cp mutations, deletion of NS2 gene, 404 in M2 gene, 530 and 1009 mutations in L gene) | - | Yes | - |
| 21 | rA2cp ΔNS2 M2/404 L248/404 (A2 backbone, cp mutations, deletion of NS2 gene, 404 in M2 gene, 248 and 404 mutations in L gene) | Yes | Yes | - |
| 22 | rA2cpΔNS2 530/1009 (A2 backbone, cp mutations, deletion of NS2 gene, 530 and 1009 mutations in L gene) | Yes | Yes | - |
| 23 | rA2cp L530/1009/955 M2/404 (A2 backbone, cp mutations, 404 in M2 gene, 530, 1009, and 955 mutations in L gene) | - | Yes | Yes |
| 24 | rA2cpΔNS2 L530/1009/955 (A2 backbone, cp mutations, deletion of NS2 gene, 530, 1009, and 955 mutations in L gene) | - | Yes | - |
| 25 | rA2cp530/1009/955 (A2 backbone, cp mutations, 530, 1009, and 955 mutations in L gene) | Yes | Yes | Yes |
| 26 | rA2cpΔNS1 (A2 backbone, cp mutations, deletion of NS1 gene) | - | Yes | Yes |
| 27 | rA2cpΔNS2 (A2 backbone, cp mutations, deletion of NS2 gene) | Yes | Yes | Yes |
| 28 | rA2wtΔNS1 (A2 backbone, deletion of NS1 gene) | - | Yes | Yes |
| 29 | rA2cpΔNS1ΔNS2 (A2 backbone, cp mutations, deletion of NS1 and NS2 genes) | - | Yes | - |
| 30 | rA2ΔM2/2 (A2 backbone, deletion of M2-2 ORF) | - | Yes | Yes |
| 31 | rA2wtΔNS2 (A2 backbone, deletion of NS2 gene) | - | Yes | Yes |
| 32 | rA2wt (backbone based on RSV A2 strain) | - | Yes | Yes |
| 33 | rA2cpNPM (A2 backbone, cp mutations, N gene inserted in 3^{rd} position, P in 4^{th} position, and M gene in 5^{th} position - wt order) | - | Yes | Yes |
| 34 | rA2cpPNM (A2 backbone, cp mutations, P gene inserted in 3^{rd} position, N in 4^{th} position, and M gene in 5^{th} position) | - | Yes | Yes |
| 35 | rA2cpPMN (A2 backbone, cp mutations, P gene inserted in 3^{rd} position, M in 4^{th} position, and N gene in 5^{th} position) | - | Yes | Yes |
| 36 | rB1 (backbone based on RSV B1 strain) | - | Yes | Yes |
| 37 | rB1 ΔNS1 (B1 backbone, deletion of NS1 gene) | - | Yes | Yes |
| 38 | rBlt ΔNS2 (B 1 backbone, deletion of NS2 gene) | - | Yes | Yes |
| 39 | rA2 B1M/G (A2 backbone, replacement of M, SH and G genes with B1 M, SH and G genes) | - | Yes | - |
| 40 | rA2ΔNS1 B1M/G (A2 backbone, replacement of M, SH and G genes with B1 M, SH and G genes, deletion of NS1 gene) | - | Yes | - |
| 41 | rA2ΔNS2 B1M/G (A2 backbone, replacement of M, SH and G genes with B1 M, SH and G genes, deletion of NS2 gene) | - | Yes | - |
| 42 | rA2ΔNS1ΔNS2 B1M/G (A2 backbone, replacement of M, SH and G genes with B1 M, SH and G genes, deletion of NS1 and NS2 genes) | - | Yes | Yes |
| 43 | rA2cpG(B ORF)248/404/1030 (A2 backbone, cp mutations, 404 mutation in M2 gene, 248, 404 and 1030 mutations in L gene, replacement of A2 G ORF with subgroup B (V9511) G ORF) | - | Yes | - |
| 44 | rABcp 530/1009/955 V9511GF (A2 backbone, cp mutations, 530, 1009 and 955 mutations in L gene, replacement of A2 G and F ORFs with subgroup B (V9511) G and F ORF, respectively) | - | Yes | Yes |
| 45 | rABwt ΔNS1 V9511GF (A2 backbone, deletion of NS1 gene, replacement of A2 G and F ORFs with subgroup B (V9511) G and F ORF, respectively) | - | Yes | - |
| 46 | rABwt ANS2 V9511GF (A2 backbone, deletion of NS2 gene, replacement of A2 G and F ORFs with subgroup B (V9511) G and F ORF, respectively) | - | Yes | - |
| 47 | rA2cpGΔ1^{st} AUG (A2 backbone, cp mutations, deletion of 1^{st} initiation codon of G ORF) | - | Yes | Yes |
| 48 | rA2cpGΔ2^{nd} AUG (A2 backbone, cp mutations, deletion of 2^{nd} initiation codon of G ORF) | - | Yes | Yes |
| 49 | rA2cpG4CtoR (A2 backbone, cp mutations, change of 4 cysteine residues in G ORF to alanine) | - | Yes | - |
| 50 | rA2cpG1F2 530/1009/955 (A2 backbone, cp mutations, 530, 1009, and 955 mutations in L gene, G gene translocated to 1^{st} position in genome, F translocated to 2^{nd} position in genome) | - | Yes | Yes |

| | | | | |
|---|---|---|---|---|
| *All viruses rescued by MVA method are sequence correct †Refers to plasmid rescued constructs only. | | | | |

### Example IX

### Rescue of Additional Negative-Strand Viruses by Plasmid T7/Electroporation/Heat Shock Method

The present example desribes successful recovery of additional negative-stranded RNA viruses via the modified, plasmid T7/electroporation/heat shock system illustrated in Example VII, above. This modified recovery method has been further applied herein to generate a large number of additional, attenuated recombinant VSV (rVSV) strains, as well as a variety of rVSV and bPIV vectors modified to encode heterologous antigens. In addition to rVSV, the plasmid T7/electroporation/heat shock method has also been used to rescue a variety of other exemplary, recombinant negative-stranded RNA viruses from Vero cells (Table 7, below). As described above for rVSV, the composition of the electroporated DNAs in these exemplary embodiments optionally included plasmids encoding the matrix protein and viral glycoproteins in addition to CMV-T7, genomic cDNA, N, P, and L. The optional addition of plasmids encoding the matrix protein and glycoproteins can provide a significant advantage when recovering viruses that have otherwise proven difficult to rescue. As is also further discussed above, optional plasmids encoding one or more additional structural proteins (e.g., M, SH, NS1, NS2, F, and/or G of RSV, and M, HN, and/or F of PIV) may also be included within these (electroporation) methods and compositions of the invention.

**Table 7 Additional Negative-Strand RNA Viruses Recovered by Plasmid T7/Electroporation/Heat Shock Method**

| | **Recombinant Virus** | **Support plasmids used in rescue** |
|---|---|---|
| 1 | Canine Distemper Virus (Onderstepoort strain) | N, P, L, M, F, H |
| 2 | Mumps virus (Jeryl Lynn Strain) | NP, P, L |
| 3 | Measles virus (Edmonston Rubeovax strain) | N, P, L, M, F, H |
| 4 | Respiratory syncytial virus | N, P, L, M2 |
| | • rA2cpΔSH/M2-404/L248-404 | |
| 5 | Bovine PIV (Shipping Fever strain) | N, P, L |
| 6 | Bovine-human hybrid PIV (bPIV SF strain F and HN genes replaced by hPIV3 homologs) | N, P, L |
| | In addition to the h/bPIV chimeric virus, three strains containing RSV A or RSV B genes have been isolated | |
| | • h/bPIV-Ga1 | RSV-A2 G gene in position 1 |
| | • h/bPIV-Fa1 | RSV-A2 F gene in position 1 |
| | • h/bPIV3-Fb1 | RSV-B F gene in position 1 |

With regard to rVSVs, various additional constructs have been generated and recovered using the plasmid T7/electroporation/heat shock method. After these rVSV recombinants are rescued, replication rates are examined *in vitro,* and relative virulence is evaluated in small animal model systems predictive of activity (e.g., attenuation and immunogenicity) in humans, to determine whether the virus is attenuated sufficiently for use in immunogenic compositions and methods of the invention.

In exemplary embodiments, a number of rVSVs were generated and recovered that contained a sixth gene encoding a heterologous antigen, for example a HIV GAG protein or HSV-2 gD glycoprotein. Five general strategies were demonstrated to be effective for introduction of modifications that impede viral replication, including: (1) Transposition or 'shuffling' of genes involved in transcription and RNA replication (e.g., the N gene) or virion maturation (e.g., M or G) into downstream positions that result in less transcription of the corresponding mRNAs (see, e.g., Ball et al., J. Virol. 73:4705-4712, 1999; Finke et al., J. Gen. Virol. 84:1613-1621, 2003; and Wertz et al., Proc. Natl. Acad. Sci. USA 95:3501-3506, 1998, each incorporated herein by reference); (2) Truncation of the cytoplasmic tail domain (CT deletions) of a viral attachment protein (e.g., G protein) (see, e.g., Roberts et al., J. Virol. 73:3723-3732, 1999, incorporated herein by reference); (3) Replacement of genes in the vector backbone with homologs encoding temperature-sensitive polypeptides (see, e.g., Pringle, J. Virol. 5:559-567, 1970; Gallione et al., J. Virol. 54:374-382, 1985, each incorporated herein by reference); (4) Introduction of mutations into the gene encoding the M protein that reduce the virion maturation rate and cytopathology (see, e.g., Jayakar et al., J. Virol. 76:8011-8018, 2002, incorporated herein by reference); and (5) Combinations of CT deletions with shuffled genes, *ts* mutations, or M mutations. Table 8, below, sets forth various exemplary, attenuated rVSV strains that were rescued according to these methods.

With regard to strategy (3) above, the introduction of temperature sensitive mutations can be readily achieved by substitution of wild-type or prototypic genes with mutant genes identified in a biologically derived *ts* mutant virus. For generating exemplary rVSV recombinants with such *ts* mutations, BHK cells were infected with exemplary, biologically-derived ts mutants tsG11, tsG31, tsG41 or tsO45 (see, e.g., Gallione et al., J. Virol. 54:374-382, 1985; Whitt et al., J. Virol. 64:4907-4913, 1990; Pringle, J. Virol. 5:559-567, 1970, each incorporated herein by reference) were incubated at permissive temperature (32°C) until cytopathic effect was evident in approximately 75% of the culture, at which time the cells were scraped into the medium and collected by low-speed centrifugation. Total infected-cell RNA was extracted from the cell pellet by the guanidinium thiocyanate-phenol-chloroform extraction method, and the purified RNA was used for reverse transcription and PCR amplification to prepare protein-coding sequence that was subsequently used to replace the homologous gene in the rVSV cDNA clone. The sequence of the cloned *ts* gene was verified by comparing its sequence to the sequence determined directly from the genomic RNA of the corresponding biologically-derived ts virus. Subsequent to sequence verification, the rVSV strains encoding one or more ts genes were rescued using the electroporation/ heat shock protocol described above except that the cells were incubated at 32°C rather than 37°C (except during heat shock) to account for the expected *ts* phenotype. Five to 9 days following electroporation, cytopathic effect was evident in successful rescue trials, at which time the new strain was harvested and used to initiate plaque purification. After 3 rounds of plaque purification, a virus stock was amplified and the genomic sequence was determined. Virus with the desired sequence was then subjected to plaque assays at several temperatures (32°C, 37°C, and 39°C) to evaluate the effect of the inserted *ts* gene.

**Table 8 Additional rVSV Strains Recovered by Plasmid T7/Electroporation/Heat Shock Method**

| ***shuffles*** | |
|---|---|
| rVSV-M5 | M gene shuffled to position five |
| rVSV-G5 | G gene shuffled to position five |
| rVSV-GAG1-N3-CT1 | N gene shuffled to position 3 combined with a CT deletion. Note: The 1 in the CT1 nomenclature refers to the truncated cytoplasmic domain that contains only a single amino acid. It does not refer to gene position |
| rVSV-GAG1-N4-CT1 | N gene shuffled to position 4 combined with a CT deletion |
| **ts Mutations** | |
| rVSV-tsN | N gene replaced with tsN from virus *ts*41 |
| rVSV-GAG1-tsN | HIV-1 GAG gene inserted at position 1, N gene replaced with *ts*N from virus *ts*41 |
| rVSV-tsL | L gene replaced with tsL from virus *ts*11 |
| rVSV-GAG1-tsL | HIV-1 GAG gene inserted at position 1, L gene replaced with *ts*L from virus *ts*11 |
| rVSV-gD-tsL | Herpes type 2 gD gene inserted at position 1, L gene replaced with tsL from virus *ts*11 |
| rVSV-tsN+L | N and L replaced with tsN from virus *ts*41*,* and *ts*L from virus *ts*11*,* respectively |
| rVSV-GAG1-tsN+L | HIV-1 GAG gene inserted at position 1, N and L replaced with *ts*N from virus *ts*41, and *ts*L from virus *ts*11*,* respectively |
| rVSV-tsG | G gene replaced with tsG from *ts*O45 |
| rVSV-GAG1-tsG | HIV-1 GAG gene inserted at position 1, G gene replaced with tsG from *ts*O45 |
| rVSV-tsM | M gene replaced with tsM from *ts*31. |
| rVSV-GAG 1-N4-CT9-tsL | N gene shuffled to position 4, C-terminal tail of G protein shortened to 9-amino acids (CT9), HIV GAG gene inserted at position 1 |
| ***Noncytopathic M gene mutation*** | |
| *rVSV-GAGI-M_{ncp12}-CT1* | The ncp12 mutations in M prevent translation from two downstream ATG codons. The met codons (33 and 55) are substituted with ala codons. |

| | |
|---|---|
| *Note on nomenclature: The VSV genome contains 5 genes arranged 3'-N(1)-P(2)-M(3)-G(4)-L(5)-5'. With the noted exception of CT modifications, above, numeric designations refer to final gene position in the subject recombinant virus. Thus, for example, a virus designated as "rVSV-M5" contains the M gene shuffled into the last or 5th position. A vector derivative of rVSV-M5 that features a gene insert encoding an HIV GAG from the first gene position contains 6 genes and is consistently designated rVSV-GAG1-M6. Each of the VSV recombinants identified in Table 8 was initially created in the Indiana serotype backbone, while most of the subject recombinants have also been recovered using a companion vector prepared from the New Jersey (NJ) serotype background. Designations of "ts" followed by a specified gene or product (e.g., "tsG") denote that the recombinant virus has incorporated a gene or mutation identified in a biologically derived mutant virus, as described in the incorporated references. Typically, a known attenuated, biologically derived virus is employed to construct a cDNA copy of the mutant gene for incorporation into a recombinant, attenuated virus. Alternatively, where the locus or loci that specify a target mutation in a biologically derived mutant virus is/are known, the subject mutation can be incorporated into the recombinant virus directly, e.g., by site-directed mutagenesis. | |

Additional rVSVs and other viral constructs were generated and recovered that express a foreign or "heterologous" antigen or antigenic determinant-yielding desired changes in immunogenic specificity. For example, recombinant VSV, bovine PIV (bPIV), and hPIV3-cp45 were designed and evaluated as potential vectors to deliver antigens from HIV-1, influenza virus types A and B, herpes simplex virus type 2, corona virus, and RSV types A and B. These vectors were rescued for subsequent immunogencity and safety studies as described herein. The rescued VSV-HIV GAG constructs and an exemplary VSV-HSV gD construct are listed in Table 8, above, while Table 9, below, summarizes exemplary vectors designed to express glycoproteins from viral respiratory pathogens (RSV, Influenza, and corona virus).

**Table 9 VSV and bPIV Vectors Expressing Glycoproteins of Viral Respiratory Pathogens Recovered by Plasmid T7/Electroporation/Heat Shock Method**

| **Vector** | **Notes** |
|---|---|
| ***RSV genes*** | |
| rVSV-Ga5 | RSV A2 G gene in position 5 |
| rVSV-Gb5 | RSV B G gene in position 5 |
| rVSV-Fa5 | RSV A2 F gene in position 5 |
| rVSV-Fb5 | RSV B G gene in position 5 |
| h/bPIV-Ga1 | bPIV (SF strain) vector in which the HN and F glycoproteins have been replaced with the human homologs. RSV A2 G gene expressed from position 1. (SF) |
| h/bPIV-Fa1 | bPIV (Shipping Fever strain) vector in which the HN and F glycoproteins have been replaced with the human homologs. RSV A2 F gene expressed from position 1 |
| h/bPIV-Fb1 | bPIV (Shipping Fever strain) vector in which the HN and F glycoproteins have been replaced with the human homologs. RSV B G gene expressed from position 1 |
| | |

| ***Ihfluenza virus genes*** | |
|---|---|
| rVSV-HA5_{PAN} | HA from A/Panama in position 5 |
| rVSV-HA5_{NC} | HA from A/New Caledonia in position 5 |
| rVSV-HA5_{HK} | HA from B/Hong Kong in position 5 |
| rVSV-NA5_{PAN} | NA from A/Panama in position 5 |
| rVSV-NA5_{NC} | NA from A/New Caledonia in position 5 |
| rVSV-NA5_{HK} | NA from B/Hong Kong in position 5 |

| ***Corona virus genes*** | |
|---|---|
| bPIV-corona virus-E1 | bPIV containing the corona virus E glycoprotein gene in position 1. |

| | |
|---|---|
| Note that the VSV vectors listed above have been created in the Indiana serotype backbone. In most cases a companion vector has been isolated that was prepared in the NJ serotype backbone. | |

### Example X

### Rescue of Replication-Defective VSV Vectors Lacking a G gene or Genome Segment

As noted above, it is often desirable to construct vector recombinants that are unable to spread significantly from the site of vaccination, but which retain the ability to infect cells and express a target antigen. Exemplary vectors that lack a structural gene, such as the M or G gene, can readily achieve this goal. The M or G gene defect will desirably prevent spread of the immunizing vector after infection by blocking complete virion maturation, but will not interfere with transcription and gene expression mediated by N, P, and L proteins. rVSVs lacking all or most of the G gene have been described, and are attractive candidates for use in immunogenic compositions and methods of the invention (see, e.g., Roberts et al., J. Virol. 73:3723-3732, 1999, Jeetendra et al., J. Virol. 76:12300-12311, 2002; Robison et al., J. Virol. 74:2239-2246, 2000, each incorporated herein by reference). To facilitate rescue and propagation of these replication-defective candidates, the present example describes a modified plasmid T7/pelectroporation/heat shock method. To exemplify the utility of this modified protocol for production and development of replication-defective candidates, two types of mutant viruses have been engineered and recovered. One exemplary mutant type completely lacks the G gene (designated as "ΔG"). The second mutant encodes a G protein (designated "G-stem") that lacks the extracellular domain of G needed for virus attachment. The mutated gene encodes the first 19 amino acids of G protein, most of which are cleaved off after the protein is inserted into the smooth endoplasmic reticulum, fused to the last 91 residues, which include the transmembrane and intracellular domains.
The modified rescue method for recovery of replication- or growth-defective negative-strand viruses (exemplified by ΔG or G-stem viruses) is as follows.

### VSV/Vero Electroporation Rescue procedure:

### Solutions

Trypsin/EDTA
Hank's buffered saline
1 mg per ml soybean trypsin inhibitor prepared in PBS
Medium components (below)

### Media

| Medium 1 | Medium 2 | Medium 3 |
|---|---|---|
| Dulbecco's modified minimal essential medium (DMEM) | Iscove's modified Dulbecco's medium (IMDM) | Dulbecco's modified minimal essnetial medium (DMEM) |
| 10% heat-inactivated fetal bovine serum | 220 uM 2-mercaptoethanol (tissue culture grade) | 10% heat-inactivated fetal bovine serum |
| 220 uM 2-mercaptoethanol (tissue culture grade) | 1% DMSO (tissue culture grade) | 220 uM 2-mercaptoethanol (tissue culture grade) |
| 1% Nonessential amino acids (10mM solution) | 1% Nonessential amino acids (10mM solution) | 1% Nonessential amino acids (10mM solution) |
| 1% sodium pyruvate (100 mM solution) | 1% sodium pyruvate (100 mM solution) | 1% sodium pyruvate (100 mM solution) |
| | | 50 ug/ml gentamicin |

### Preparation of Vero cells

Each electroporation requires cells from approximately 1.3 near-confluent T150 flasks.
1) Wash cell monolayer 1x with approximately 5 ml of Hank's Buffered Saline Solution.
2) Add 4ml trypsin/EDTA and rock the flask to evenly distribute the solution.
3) Incubate at room temperature 3-5 min.
4) Aspirate the trypsin solution.
5) Tap the sides of the flask to dislodge cells.
6) Use 10 ml of Media #1 to collect cells from flask(s) and transfer cells to a 50 ml conical tube.
7) Add 1 ml of trypsin inhibitor (1 mg/ml) and mix gently.
8) Centrifuge cells at 1000 rpm at room temp. in table-top centrifuge tube at 1000 rpm for 5 min.
9) Aspirate the supernatant.
10) Resuspend the cells in 10 ml of Medium 2.
11) Add 1ml of trypsin inhibitor and mix gently.
12) Collect cells at 1000 rpm in a room temperature table-top centrifuge as described above.
13) Aspirate medium.
14) Resuspend cells in medium 2. Gently resuspend in a final volume that will be equivalent to 0.7 ml per electroporation. (take into account that the cell pellet will contribute volume).
15) Transfer 0.7ml of cell suspension to the centrifuge tube containing the plasmid DNA solution. Gently pipet up and down with a p 1000 tip to mix the cells and DNA. Move to the electroporator station.

### DNA preparation:

For each rescue electroporation, electroporate
50 µg CMV-G
a) While working in a biosafety hood, adjust the DNA volume to 250 µl with sterile, nuclease-free water
b) Add 50 µl of 3M Sodium Acetate (pH 5)
c) Add 750 µl of 100% Ethanol and mix
d) Incubate at -20°C for 1 hour to overnight
e) Pellet the DNA in a microfuge at 14,000 rpm, 4°C for 20 minutes
f) Discard supernatant, while working in a biosafety hood, without disturbing the pellet
g) Quick-spin the tube to collect residual Ethanol at bottom and remove with pipet
h) Let pellet air dry (in biosafety hood) for 5-10 minutes
i) Resuspend pellet with 50 µl of sterile, nuclease-free water

### Electroporation

### BTX 820 Square-Wave Electroporator

1) Pipet the cells/DNA up and down 3 times to mix gently and transfer to an electroporation cuvette.
2) Insert cuvette in electroporation stand.
3) Electroporate the cells (4 pulses, 70 msec, 140 Volts).
4) After electroporating all samples, return the cuvette(s) to the biosafety hood and add 1 ml of medium 1. Using a sterile plastic transfer pipet, transfer the cells from the cuvette to a 15 ml centrifuge tube containing 10 ml of medium 1.
5) Mix gently and collect the cells by centrifugation as described above.
6) Aspirate the medium.
7) Resuspend the cells in 10 ml medium 1 and transfer the suspension to 2 T150 flask that contains 20 ml medium 1.
8) Incubate the cells at 37° C (5% CO₂) for 3 hours.
9) Heat shock the cells at 43°C (3-5% CO₂) for 3 hours.
10) Return cells to 37°C over night.
11) Replace the medium with 20 ml of medium 3.
12) Aspirate1 T150 that contains rescue material. Add 10mls of medium 3. Scrape cells into medium and transfer to 1 T150 of G expressing cells and continue incubation at 37°C
13) Inspect cells daily for signs of cytopathic effect. VSV replication should be evident as early as 3-4 days but may take as long as 6.

The principal difference in the foregoing, modified electroporation method compared to the alternate, exemplary method described in Example VII, is the inclusion of a coculture step in which the cells used for rescue are grown with cells engineered to express abundant G protein. Briefly, VSV rescue is initiated by electoporating Vero cells with CMV-T7 plasmid, viral genomic cDNA, and vectors encoding N, P, L, M and G (the virus rescue cells). These cells are subsequently heat shocked for 3 hours before overnight incubation. When the virus rescue electroporation is performed, a second set of Vero cells is electorporated to prepare cells for coculture that will transiently express large amounts of G protein. These coculture cells are electorporated with a relatively substanital amount of a CMV expression plasmid that encodes VSV G protein. The day following electroporation, the cells transfected with the rescue mixture are scraped into the mudium and transferred onto the monolayer of G protein expressing coculture cells. Viral cytopathic effect generally is evident in 2-4 days later.

Although the foregoing invention has been described in detail by way of example, it will be apparent to the artisan that certain changes and modifications may be practiced within the scope of the appended claims, which are presented by way of illustration not limitation.

## Claims

1. An infectious, non-segmented, negative-stranded RNA virus of the Order Mononegavirales produced by the process of: introducing into a suitable host cell a viral cDNA expression vector comprising a polynucleotide encoding a genome or antigenome of a non-segmented negative-stranded RNA virus operably linked with an expression control sequence to direct synthesis of viral RNA transcripts from said cDNA expression vector, one or more support vector(s) that encode(s) and direct(s) expression of an N protein, a P protein, and an L protein, and a transient expression vector that encodes and directs transient expression of an RNA polymerase, wherein one or more of said viral cDNA expression vector and a transiently-transfected expression vector encoding the RNA polymerase are introduced into said host cell by electroporation; and further wherein after one or more of said viral cDNA expression vector and a transiently-transfected expression vector encoding the RNA polymerase are introduced into said host cell, the host cell is exposed to an effective heat shock under conditions sufficient to increase the recovery of the recombinant virus; and recovering assembled infectious, non-segmented, negative-stranded RNA virus from said host cells.

2. The infectious, non-segmented, negative-stranded RNA virus produced according to the process of claim 1, wherein said virus is a Vesicular Stomatitis Virus (VSV).

3. The infectious VSV produced according to the process of claim 2, wherein said RNA polymerase is a T7 RNA polymerase or;
wherein said transient expression vector that encodes and directs transient expression of said RNA polymerase is a plasmid or;
wherein
(a) said viral cDNA expression vector and said transient expression vector that encodes and directs transient expression of the RNA polymerase are combined in a DNA transfection mixture and added to a host cell culture simultaneously to achieve coordinate transfection;
(b) said viral cDNA expression vector is introduced into the host cell prior to introduction of a transiently-transfected expression vector encoding the RNA polymerase; or
(c) said viral cDNA expression vector is introduced into the host cell after introduction of a transiently-transfected expression vector encoding the RNA polymerase but before said RNA polymerase has accumulated in said host cell in detectable levels or;
wherein the infectious VSV is a complete virus or;
wherein the polynucleotide molecule encoding the genome or antigenome encodes the sequence of a wild-type VSV or;
wherein the VSV is recombinantly modified by introduction of one or more attenuating mutation(s) or;
wherein the VSV is modified to encode a non-viral molecule selected from a cytokine, a T-helper epitope, a restriction site marker, or a protein of a microbial pathogen or parasite capable of eliciting an immune response in a mammalian host or;
wherein the polynucleotide encoding the genome or antigenome is modified by addition, substitution, or translocation of a gene or genome segment to a position that is more promoter-proximal or promoter-distal compared to a wild type gene order position of said gene or genome segment within the genome or antigenome of said VSV or;
wherein the process further comprises co-introduction into and/or co-expression in said host cell of any one, or any combination of, structural protein(s) of the same or of a different VSV or other non-segmented negative-stranded RNA virus.

4. The infectious VSV produced according to the process of claim 2, wherein after a period of time sufficient to permit expression of said viral cDNA expression vector, said one or more support vectors that encode(s) and direct(s) expression of the N protein, P protein, and L protein, and said transient expression vector that encode(s) and direct(s) expression of the RNA polymerase, the host cell is co-cultured with a plaque expansion cell of the same or different cell type to allow spread of rescued VSV to the plaque expansion cell.

5. The infectious VSV produced according to the process of claim 4, wherein said host cell is transferred to at least one layer of plaque expansion cells to establish a co-culture of host and plaque expansion cells.

6. An immunogenic composition comprising an immunogenically effective amount of an isolated, infectious VSV produced according to the process of claim 2 in a pharmaceutically acceptable carrier.

7. The immunogenic composition of claim 6,
wherein the VSV is formulated in a dose of 10³ to 10⁷ PFU or;
wherein the host cell is co-cultured with a plaque expansion cell of the same or different cell type to allow spread of rescued VSV to the plaque expansion cell or; further comprising co-introduction into and/or co-expression in said host cell of any one, or any combination of, structural protein(s) of the same or different VSV or other non-segmented negative-stranded RNA virus.

8. A growth-or replication-defective, non-segmented, negative-stranded RNA virus of the Order Mononegavirales produced by the process of: introducing into a suitable host cell a viral cDNA expression vector comprising a polynucleotide encoding a genome or antigenome of a growth- or replication-defective non-segmented negative-stranded RNA virus operably linked with an expression control sequence to direct synthesis of viral RNA transcripts from said cDNA expression vector, one or more support vector(s) that encode(s) and direct(s) expression of an N protein, a P protein, and an L protein, and a transient expression vector that encodes and directs transient expression of an RNA polymerase, wherein one or more of said viral cDNA expression vector and a transiently-transfected expression vector encoding the RNA polymerase are introduced into said host cell by electroporation; and further wherein after one or more of said viral cDNA expression vector and a transiently-transfected expression vector encoding the RNA polymerase are introduced into said host cell, the host cell is exposed to an effective heat shock under conditions sufficient to increase the recovery of the recombinant virus; and recovering assembled growth- or replication-defective, non-segmented, negative-stranded RNA virus from said host cells.

9. The growth-or replication-defective, non-segmented, negative-stranded RNA virus of the Order Mononegavirales produced by the process of claim 8, wherein said virus is a Vesicular Stomatitis Virus (VSV).

10. An immunogenic composition comprising an immunogenically effective amount of an isolated, growth- or replication-defective VSV produced according to the process of claim 9 in a pharmaceutically acceptable carrier.

11. Use of an immunogenically effective amount of an isolated, growth- or replication-defective VSV produced according to the process of claim 9 in a pharmaceutically acceptable carrier in the preparation of a medicament for stimulating the immune system of a mammalian subject to induce an immune response in the subject against a VSV.

12. A method for producing an infectious, non-segmented, negative-stranded RNA virus of the Order Mononegavirales comprising the steps of:
(a) introducing a viral cDNA expression vector comprising a polynucleotide encoding a genome or antigenome of a non-segmented negative-stranded RNA virus operably linked with an expression control sequence to direct synthesis of viral RNA transcripts from said cDNA expression vector into a host cell, wherein said host cell expresses an N protein, a P protein, and an L protein, and transiently expresses an RNA polymerase from a transiently-transfected expression vector; and
(b) recovering assembled infectious, non-segmented, negative-stranded RNA virus from said host cells.

13. The method of claim 12
wherein said RNA polymerase is a T7 RNA polymerase or;
wherein said transiently-transfected expression vector is a plasmid or;
wherein said viral cDNA expression vector and a transient expression vector that encodes and directs transient expression of the RNA polymerase are combined in a DNA transfection mixture and added to a host cell culture simultaneously to achieve coordinate transfection or;
wherein said viral cDNA expression vector is introduced into the host cell prior to introduction of a transiently-transfected expression vector encoding the RNA polymerase or;
wherein said viral cDNA expression vector is introduced into the host cell after introduction of a transiently-transfected expression vector encoding the RNA polymerase but before said RNA polymerase has accumulated in said host cell in detectable levels or;
wherein one or more of said viral cDNA expression vector and a transiently-transfected expression vector encoding the RNA polymerase are introduced into said host cell by calcium phosphate transfection or electroporation or;
wherein after one or more of said viral cDNA expression vector and a transiently-transfected expression vector encoding the RNA polymerase are introduced into said host cell, and the host cell is exposed to an effective heat shock under conditions sufficient to increase the recovery of the recombinant virus, preferably wherein cell is exposed to a heat shock temperature above 37°C or wherein the host cell is exposed to a heat shock temperature in the range of about 37°C to about 50°C or wherein the host cell is exposed to a heat shock temperature in the range of from about 41°C to about 47°C or wherein the host cell is subjected to effective heat shock for at least about 5 minutes preferably wherein the host cell is subjected to effective heat shock for between about 15 minutes to about 200 minutes preferably wherein the host cell is subjected to effective heat shock for between about 30 minutes to about 150 minutes or;
wherein after a period of time sufficient to permit expression of said viral cDNA expression vector and a transiently-transfected expression vector encoding and directing expression of the RNA polymerase, the host cell is co-cultured with a plaque expansion cell of the same or different cell type to allow spread of rescued virus to the plaque expansion cell preferably wherein said host cell is transferred to at least one layer of plaque expansion cells to establish a co-culture of host and plaque expansion cells or wherein said host cell is of a different cell type than said plaque expansion cell or wherein said host cell and plaque expansion cell are each selected from HEp-2, HeLa, HEK, BHK, FRhL-DBS2, LLC-MK2, MRC-5, and Vero cells or;
wherein said host cell is a eukaryotic cell or;
wherein said host cell is a vertebrate cell or;
wherein said host cell is selected from HEp-2, HeLa, HEK, BHK, FRhL-DBS2, LLC-MK2, MRC-5, and Vero cells or;
wherein the infectious, non-segmented, negative-stranded RNA virus is a complete virus or;
wherein one or more of said N, P and L proteins is/are of a heterologous non-segmented, negative-stranded RNA virus or;
wherein the polynucleotide molecule encoding the genome or antigenome encodes the sequence of a wild-type non-segmented negative-stranded RNA virus or;
wherein the non-segmented, negative-stranded RNA virus is a member of the family paramyxoviridae or;
wherein the non-segmented, negative-stranded RNA virus is a member of the family rhabdoviridae or;
wherein the non-segmented, negative-stranded RNA virus is selected from a respiratory syncytial virus (RSV), human parainfluenza virus (HPIV), bovine parainfluenza virus (BPIV), chimeric human-bovine PIV (HBPIV), measles virus (MV), or vesicular stomatitis virus (VSV) or;
wherein the non-segmented negative-stranded RNA virus is recombinantly modified by introduction of one or more attenuating mutation(s)preferably wherein the non-segmented negative-stranded RNA virus incorporates one or more recombinantly-introduced, temperature sensitive (ts) attenuating mutations or wherein the non-segmented negative-stranded RNA virus incorporates one or more attenuating mutation(s) identified in a biologically derived mutant non-segmented negative-stranded RNA virus or wherein the non-segmented negative-stranded RNA virus incorporates one or more attenuating mutation(s) corresponding to one or more mutations identified in a heterologous, mutant non-segmented negative-stranded RNA virus or wherein the non-segmented negative-stranded RNA virus incorporates at least one attenuating mutation specified by two or three nucleotide changes in a codon specifying the mutation or;
wherein the non-segmented negative-stranded RNA virus is recombinantly modified by a nucleotide modification specifying a phenotypic change selected from a change in growth characteristics, attenuation, temperature-sensitivity, cold-adaptation, plaque size, host-range restriction, or a change in immunogenicity preferably wherein the non-segmented negative-stranded RNA virus incorporates a recombinant modification that alters one or more C, C', D, E, F, G, HA, HN, L, M, M2-1, M2-2, NS1, NS2, SH, V, Y1, and/or Y2 open reading frames (ORFs) and/or a 3' leader, 5' trailer, and/or intergenic region within the genome or antigenome of the virus or;
wherein one or more gene(s) of the non-segmented negative-stranded RNA virus is deleted in whole or in part or expression of the gene(s) is reduced or ablated by a mutation in an RNA editing site, by a frameshift mutation, by a mutation that alters a translation start site, by introduction of one or more stop codons in an open reading frame (ORF) of the gene, or by a mutation in a transcription signal or;
wherein the non-segmented negative-stranded RNA virus is modified to encode a non-viral molecule selected from a cytokine, a T-helper epitope, a restriction site marker, or a protein of a microbial pathogen or parasite capable of eliciting an immune response in a mammalian host or;
wherein the polynucleotide encoding the genome or antigenome comprises a partial or complete vector genome or antigenome combined with one or more heterologous gene(s) or genome segment(s) encoding one or more antigenic determinant(s) of one or more heterologous pathogen(s) to form a chimeric genome or antigenome preferably wherein said one or more heterologous gene(s) or genome segment(s) encoding the antigenic determinant(s) is/are added as supernumerary gene(s) or genome segment(s) adjacent to or within a noncoding region of the partial or complete vector genome or antigenome, or wherein said one or more heterologous gene(s) or genome segment(s) encoding the antigenic determinant(s) is/are substituted for one or more counterpart gene(s) or genome segment(s) in a partial vector genome or antigenome or wherein the heterologous gene or genome segment is added or substituted at a position corresponding to a wild-type gene order position of a counterpart gene or genome segment within the partial or complete vector genome or antigenome or wherein the heterologous gene or genome segment is added or substituted at a position that is more promoter-proximal or promoter-distal compared to a wild-type gene order position of a counterpart gene or genome segment within the partial or complete vector genome or antigenome or wherein the vector genome or antigenome is modified to encode a chimeric glycoprotein incorporating one or more heterologous antigenic domains, fragments, or epitopes of a heterologous non-segmented negative-stranded RNA virus to form the chimeric genome or antigenome or wherein the heterologous pathogen is selected from measles virus, subgroup A and subgroup B respiratory syncytial viruses, human parainfluenza viruses, mumps virus, human papilloma viruses, type 1 and type 2, human immunodeficiency viruses, herpes simplex viruses, cytomegalovirus, rabies virus, human metapneuomovirus, Epstein Barr virus, filoviruses, bunyaviruses, flaviviruses, alphaviruses, and influenza viruses or wherein the chimeric genome or antigenome is recombinantly modified by introduction of one or more attenuating mutation(s)or wherein the chimeric genome or antigenome incorporates one or more attenuating mutation(s) identified in a biologically derived mutant non-segmented negative-stranded RNA virus or wherein the chimeric genome or antigenome is recombinantly modified by a nucleotide modification specifying a phenotypic change selected from a change in growth characteristics, attenuation, temperature-sensitivity, cold-adaptation, plaque size, host-range restriction, or a change in immunogenicity or wherein the chimeric genome or antigenome incorporates a recombinant modification that alters one or more C, C', D, E, F, G, HA, HN, L, M, M2-1, M2-2, NS1, NS2, SH, V, Y1, and/or Y2 open reading frames (ORFs) and/or a 3' leader, 5' trailer, and/or intergenic region within the genome or antigenome of the virus or wherein one or more gene(s) within the chimeric genome or antigenome is deleted in whole or in part or expression of the gene(s) is reduced or ablated by a mutation in an RNA editing site, by a frameshift mutation, by a mutation that alters a translation start site, by introduction of one or more stop codons in an open reading frame (ORF) of the gene, or by a mutation in a transcription signal or wherein the chimeric genome or antigenome is modified to encode a non-viral molecule selected from a cytokine, a T-helper epitope, a restriction site marker, or a protein of a microbial pathogen or parasite capable of eliciting an immune response in a mammalian host or;
wherein the polynucleotide encoding the genome or antigenome is modified by addition, substitution, or translocation of a gene or genome segment to a position that is more promoter-proximal or promoter-distal compared to a wild-type gene order position of said gene or genome segment within the genome or antigenome of said non-segmented negative-stranded RNA virus or;
wherein the polynucleotide encoding the genome or antigenome is modified to incorporate a heterologous regulatory element comprising an extragenic 3' leader or 5' trailer region, a gene-start signal, gene-end signal, editing region, intergenic region, or a 3' or 5' non-coding region or;
wherein the polynucleotide encoding the genome or antigenome is modified to incorporate a polynucleotide insertion of between 150 nucleotides (nts) and 4,000 nucleotides in length in a non-coding region (NCR) of the genome or antigenome or as a separate gene unit (GU), said polynucleotide insertion lacking a complete open reading frame (ORF) and specifying an attenuated phenotype in said non-segmented negative-stranded RNA virus or;
further comprising co-introduction into and/or co-expression in said host cell of any one, or any combination of, structural protein(s) of the same or of a different non-segmented negative-stranded RNA virus.

14. A method for producing an infectious, non-segmented, negative-stranded RNA virus of the Order Mononegavirales comprising the steps of:
introducing into a suitable host cell a viral cDNA expression vector comprising a polynucleotide encoding a genome or antigenome of a non-segmented negative-stranded RNA virus operably linked with an expression control sequence to direct synthesis of viral RNA transcripts from said viral cDNA expression vector, and one or more transient expression vectors that encode(s) and direct(s) transient expression of an RNA polymerase, an N protein, a P protein, and an L protein under conditions sufficient to permit the co-expression of said vectors and production of the recombinant virus;
transferring said host cell into co-culture with a plaque expansion cell; and
recovering assembled infectious, non-segmented, negative-stranded RNA virus from said co-culture; preferably wherein the host cell is co-cultured with a plaque expansion cell of the same or different cell type to allow spread of rescued virus to the plaque expansion cell or; wherein said host cell and plaque expansion cell are each selected from HEp-2, HeLa, HEK, BHK, FRhL-DBS2, LLC-MK2, MRC-5, and Vero cells or; wherein after one or more of said viral cDNA expression vector and a transient expression vector encoding the RNA polymerase are introduced into said host cell, the host cell is exposed to an effective heat shock under conditions sufficient to increase the recovery of the recombinant virus or; further comprising co-introduction into and/or co-expression in said host cell of any one, or any combination of, structural protein(s) of the same or different non-segmented negative-stranded RNA virus or;
a method for producing an infectious, non-segmented, negative-stranded RNA virus of the Order Mononegavirales comprising the steps of:
(a) introducing a viral cDNA expression vector comprising a polynucleotide encoding a genome or antigenome of a non-segmented negative-stranded RNA virus operably linked with an expression control sequence to direct synthesis of viral RNA transcripts from said cDNA expression vector, into a host cell transiently expressing:
(1) an RNA polymerase,
(2) an N protein,
(3) a P protein, and
(4) an L protein,
wherein each of said RNA polymerase and N, P, and L proteins is expressed from a transiently transfected expression vector; and
(b) recovering assembled infectious, non-segmented, negative-stranded RNA virus from said host cells oreferalby wherein the host cell is co-cultured with a plaque expansion cell of the same or different cell type to allow spread of rescued virus to the plaque expansion cell or wherein said host cell and plaque expansion cell are each selected from HEp-2, HeLa, HEK, BHK, FRhL-DBS2, LLC-MK2, MRC-5, and Vero cells or wherein after one or more of said viral cDNA expression vector and a transient expression vector encoding the RNA polymerase are introduced into said host cell, the host cell is exposed to an effective heat shock under conditions sufficient to increase the recovery of the recombinant virus or further comprising co-introduction into and/or co-expression in said host cell of any one, or any combination of, structural protein(s) of the same or different non-segmented negative-stranded RNA virus or;
a method for producing an infectious, non-segmented, negative-stranded RNA virus of the Order Mononegavirales comprising the steps of:
introducing into a suitable host cell a viral cDNA expression vector comprising a polynucleotide encoding a genome or antigenome of a non-segmented negative-stranded RNA virus operably linked with an expression control sequence to direct synthesis of viral RNA transcripts from said viral cDNA expression vector, and one or more transient expression vectors that encode(s) and direct(s) transient expression of an RNA polymerase, an N protein, a P protein, and an L protein; and
recovering assembled infectious, non-segmented, negative-stranded RNA virus from said host cells preferably wherein at least one of the N, P and L proteins is encoded by an expression control plasmid or wherein the host cell is co-cultured with a plaque expansion cell of the same or different cell type to allow spread of rescued virus to the plaque expansion cell or wherein said host cell and plaque expansion cell are each selected from HEp-2, HeLa, HEK, BHK, FRhL-DBS2, LLC-MK2, MRC-5, and Vero cells or wherein after one or more of said viral cDNA expression vector and a transient expression vector encoding the RNA polymerase are introduced into said host cell, the host cell is exposed to an effective heat shock under conditions sufficient to increase the recovery of the recombinant virus or further comprising co-introduction into and/or co-expression in said host cell of any one, or any combination of, structural protein(s) of the same or different non-segmented negative-stranded RNA virus.

15. A composition for producing an infectious, non-segmented, negative-stranded RNA virus of the Order Mononegavirales comprising:
(a) a viral cDNA expression vector comprising a polynucleotide encoding a genome or antigenome of a non-segmented negative-stranded RNA virus operably linked with an expression control sequence to direct synthesis of viral RNA transcripts from said cDNA in a suitable host cell;
(b) one or more support vectors that encode(s) and direct(s) expression in said host cell of:
(1) an N protein,
(2) a P protein, and
(3) an L protein; and
(c) a transient expression vector that encodes and directs transient expression in said host cell of an RNA polymerase
preferably wherein said viral cDNA expression vector, said one or more support vectors encoding N, P, and L, and said transient expression vector that encodes and directs transient expression of said RNA polymerase are provided in a cell-free co-transfection mixture or;
wherein said viral cDNA expression vector, said one or more support vectors encoding N, P, and L, and said transient expression vector that encodes and directs transient expression of said RNA polymerase are transfected into said host cell or;
wherein said RNA polymerase is a T7 RNA polymerase or wherein said transient expression vector that encodes and directs transient expression of said RNA polymerase is a plasmid or;
further comprising a plaque expansion cell of the same or different cell type as the host cell to allow spread of rescued virus from the host cell to the plaque expansion cell preferably further comprising at least one layer of plaque expansion cells to establish a co-culture of host and plaque expansion cells or wherein said host cell is of a different cell type than said plaque expansion cell or wherein said host cell and plaque expansion cell are each selected from HEp-2, HeLa, HEK, BHK, FRhL-DBS2, LLC-MK2, MRC-5, and Vero cells or;
wherein said host cell is a eukaryotic cell or;
wherein said host cell is a vertebrate cell or;
wherein said host cell is selected from HEp-2, HeLa, HEK, BHK, FRhL-DBS2, LLC-MK2, MRC-5, and Vero cells or;
wherein the infectious, non-segmented, negative-stranded RNA virus is a complete virus or;
wherein one or more of said N, P and L proteins is/are of a heterologous non-segmented, negative-stranded RNA virus or;
wherein the polynucleotide molecule encoding the genome or antigenome encodes the sequence of a wild-type non-segmented negative-stranded RNA virus or;
wherein the non-segmented, negative-stranded RNA virus is a member of the family paramyxoviridae or;
wherein the non-segmented, negative-stranded RNA virus is a member of the family rhabdoviridae or;
wherein the non-segmented, negative-stranded RNA virus is selected from a respiratory syncytial virus (RSV), human parainfluenza virus (HPIV), bovine parainfluenza virus (BPIV), chimeric human-bovine PIV (HBPIV), measles virus (MV), or vesicular stomatitis virus (VSV) or;
wherein the non-segmented negative-stranded RNA virus is recombinantly modified by introduction of one or more attenuating mutation(s) preferably wherein the non-segmented negative-stranded RNA virus incorporates one or more recombinantly-introduced, temperature sensitive (ts) attenuating mutations or wherein the non-segmented negative-stranded RNA virus incorporates one or more attenuating mutation(s) identified in a biologically derived mutant non-segmented negative-stranded RNA virus or wherein the non-segmented negative-stranded RNA virus incorporates one or more attenuating mutation(s) corresponding to one or more mutations identified in a heterologous, mutant non-segmented negative-stranded RNA virus or wherein the non-segmented negative-stranded RNA virus incorporates at least one attenuating mutation specified by two or three nucleotide changes in a codon specifying the mutation or;
wherein the non-segmented negative-stranded RNA virus is recombinantly modified by a nucleotide modification specifying a phenotypic change selected from a change in growth characteristics, attenuation, temperature-sensitivity, cold-adaptation, plaque size, host-range restriction, or a change in immunogenicity or;
wherein the non-segmented negative-stranded RNA virus incorporates a recombinant modification that alters one or more C, C', D, E, F, G, HA, HN, L, M, M2-1, M2-2, NS1, NS2, SH, V, Y1, and/or Y2 open reading frames (ORFs) and/or a 3' leader, 5' trailer, and/or intergenic region within the genome or antigenome of the virus or;
wherein one or more gene(s) of the non-segmented negative-stranded RNA virus is deleted in whole or in part or expression of the gene(s) is reduced or ablated by a mutation in an RNA editing site, by a frameshift mutation, by a mutation that alters a translation start site, by introduction of one or more stop codons in an open reading frame (ORF) of the gene, or by a mutation in a transcription signal or;
wherein the non-segmented negative-stranded RNA virus is modified to encode a non-viral molecule selected from a cytokine, a T-helper epitope, a restriction site marker, or a protein of a microbial pathogen or parasite capable of eliciting an immune response in a mammalian host or;
wherein the polynucleotide encoding the genome or antigenome comprises a partial or complete vector genome or antigenome combined with one or more heterologous gene(s) or genome segment(s) encoding one or more antigenic determinant(s) of one or more heterologous pathogen(s) to form a chimeric genome or antigenome preferably wherein said one or more heterologous gene(s) or genome segment(s) encoding the antigenic determinant(s) is/are added as supernumerary gene(s) or genome segment(s) adjacent to or within a noncoding region of the partial or complete vector genome or antigenome, or wherein said one or more heterologous gene(s) or genome segment(s) encoding the antigenic determinant(s) is/are substituted for one or more counterpart gene(s) or genome segment(s) in a partial vector genome or antigenome or wherein the heterologous gene or genome segment is added or substituted at a position corresponding to a wild-type gene order position of a counterpart gene or genome segment within the partial or complete vector genome or antigenome or wherein the heterologous gene or genome segment is added or substituted at a position that is more promoter-proximal or promoter-distal compared to a wild-type gene order position of a counterpart gene or genome segment within the partial or complete vector genome or antigenome or wherein the vector genome or antigenome is modified to encode a chimeric glycoprotein incorporating one or more heterologous antigenic domains, fragments, or epitopes of a heterologous non-segmented negative-stranded RNA virus to form the chimeric genome or antigenome or the heterologous pathogen is selected from measles virus, subgroup A and subgroup B respiratory syncytial viruses, human parainfluenza viruses, mumps virus, human papilloma viruses, type 1 and type 2, human immunodeficiency viruses, herpes simplex viruses, cytomegalovirus, rabies virus, human metapneuomovirus, Epstein Barr virus, filoviruses, bunyaviruses, flaviviruses, alphaviruses, and influenza viruses or wherein the chimeric genome or antigenome is recombinantly modified by introduction of one or more attenuating mutation(s) or wherein the chimeric genome or antigenome incorporates one or more attenuating mutation(s) identified in a biologically derived mutant non-segmented negative-stranded RNA virus or wherein the chimeric genome or antigenome is recombinantly modified by a nucleotide modification specifying a phenotypic change selected from a change in growth characteristics, attenuation, temperature-sensitivity, cold-adaptation, plaque size, host-range restriction, or a change in immunogenicity or wherein the chimeric genome or antigenome incorporates a recombinant modification that alters one or more C, C', D, E, F, G, HA, HN, L, M, M2-1, M2-2, NS1, NS2, SH, V, Y1, and/or Y2 open reading frames (ORFs) and/or a 3' leader, 5' trailer, and/or intergenic region within the genome or antigenome of the virus or wherein one or more gene(s) within the chimeric genome or antigenome is deleted in whole or in part or expression of the gene(s) is reduced or ablated by a mutation in an RNA editing site, by a frameshift mutation, by a mutation that alters a translation start site, by introduction of one or more stop codons in an open reading frame (ORF) of the gene, or by a mutation in a transcription signal or wherein the chimeric genome or antigenome is modified to encode a non-viral molecule selected from a cytokine, a T-helper epitope, a restriction site marker, or a protein of a microbial pathogen or parasite capable of eliciting an immune response in a mammalian host or;
wherein the polynucleotide encoding the genome or antigenome is modified by addition, substitution, or translocation of a gene or genome segment to a position that is more promoter-proximal or promoter-distal compared to a wild-type gene order position of said gene or genome segment within the genome or antigenome of said non-segmented negative-stranded RNA virus or;
wherein the polynucleotide encoding the genome or antigenome is modified to incorporate a heterologous regulatory element comprising an extragenic 3' leader or 5' trailer region, a gene-start signal, gene-end signal, editing region, intergenic region, or a 3' or 5' non-coding region or;
wherein the polynucleotide encoding the genome or antigenome is modified to incorporate a polynucleotide insertion of between 150 nucleotides (nts) and 4,000 nucleotides in length in a non-coding region (NCR) of the genome or antigenome or as a separate gene unit (GU), said polynucleotide insertion lacking a complete open reading frame (ORF) and specifying an attenuated phenotype in said non-segmented negative-stranded RNA virus or;
wherein one or more expression vector(s) encode(s) one, or any combination of, structural protein(s) of the same or different non-segmented negative-stranded RNA virus.

16. An infectious, non-segmented, negative-stranded RNA virus of the Order Mononegavirales produced by the process of:
introducing into a suitable host cell a viral cDNA expression vector comprising a polynucleotide encoding a genome or antigenome of a non-segmented negative-stranded RNA virus operably linked with an expression control sequence to direct synthesis of viral RNA transcripts from said cDNA expression vector, one or more support vector(s) that encode(s) and direct(s) expression of an N protein, a P protein, and an L protein, and a transient expression vector that encodes and directs transient expression of an RNA polymerase; and
recovering assembled infectious, non-segmented, negative-stranded RNA virus from said host cells preferably wherein said RNA polymerase is a T7 RNA polymerase or;
wherein said transient expression vector that encodes and directs transient expression of said RNA polymerase is a plasmid or;
wherein said viral cDNA expression, said one or more support vector(s) that encode(s) and direct(s) expression of the N protein, P protein and L protein, and said transient expression vector that encodes and directs transient expression of the RNA polymerase are combined in a DNA transfection mixture and added to a host cell culture simultaneously to achieve coordinate transfection or;
wherein said viral cDNA expression vector and said one or more support vector(s) that encode(s) and direct(s) expression of the N protein, P protein and L protein are introduced into the host cell prior to introduction of the transient expression vector encoding the RNA polymerase or;
wherein said viral cDNA expression vector and said one or more support vector(s) that encode(s) and direct(s) expression of the N protein, P protein and L protein are introduced into the host cell after introduction of the transient expression vector encoding the RNA polymerase but before said RNA polymerase has accumulated in said host cell in detectable levels or;
wherein one or more of (a) said viral cDNA expression vector and (b) said one or more support vector(s) that encode(s) and direct(s) expression of the RNA polymerase, N protein, P protein, and L protein are introduced into said host cell by calcium phosphate transfection or electroporation or;
wherein after one or more of (a) said viral cDNA expression vector and (b) said one or more support vectors that encode(s) and direct(s) expression of the N protein, P protein, and L protein, and said transient expression vector that encodes and directs expression of said RNA polymerase are introduced into said host cell, the host cell is exposed to an effective heat shock under conditions sufficient to increase the recovery of the recombinant virus preferably wherein the host cell is exposed to a heat shock temperature above 37°C or wherein the host cell is exposed to a heat shock temperature in the range of about 37°C to about 50°C or wherein the host cell is exposed to a heat shock temperature in the range of from about 41°C to about 47°C or wherein the host cell is subjected to effective heat shock for at least about 5 minutes preferably wherein the host cell is subjected to effective heat shock for between about 15 minutes to about 200 minutes preferably wherein the host cell is subjected to effective heat shock for between about 30 minutes to about 150 minutes or;
wherein after a period of time sufficient to permit expression of said viral cDNA expression vector, said one or more support vectors that encode(s) and direct(s) expression of the N protein, P protein, and L protein, and said transient expression vector that encode(s) and direct(s) expression of the RNA polymerase, the host cell is co-cultured with a plaque expansion cell of the same or different cell type to allow spread of rescued virus to the plaque expansion cell preferably wherein said host cell is transferred to at least one layer of plaque expansion cells to establish a co-culture of host and plaque expansion cells or wherein said host cell is of a different cell type than said plaque expansion cell or;
wherein said host cell and plaque expansion cell are each selected from HEp-2, HeLa, HEK, BHK, FRhL-DBS2, LLC-MK2, MRC-5, and Vero cells or;
wherein said host cell is a eukaryotic cell or;
wherein said host cell is a vertebrate cell or;
wherein said host cell is selected from HEp-2, HeLa, HEK, BHK, FRhL-DBS2, LLC-MK2, MRC-5, and Vero cells or;
wherein the infectious, non-segmented, negative-stranded RNA virus is a complete virus or;
wherein one or more of said N, P and L proteins is/are of a heterologous non-segmented, negative-stranded RNA virus or;
wherein the polynucleotide molecule encoding the genome or antigenome encodes the sequence of a wild-type non-segmented negative-stranded RNA virus or;
wherein the non-segmented, negative-stranded RNA virus is a member of the family paramyxoviridae or;
wherein the non-segmented, negative-stranded RNA virus is a member of the family rhabdoviridae or;
wherein the non-segmented, negative-stranded RNA virus is selected from a respiratory syncytial virus (RSV), human parainfluenza virus (HPIV), bovine parainfluenza virus (BPIV), chimeric human-bovine PIV (HBPIV), measles virus (MV), or vesicular stomatitis virus (VSV) or;
wherein the non-segmented negative-stranded RNA virus is recombinantly modified by introduction of one or more attenuating mutation(s) preferably wherein the non-segmented negative-stranded RNA virus incorporates one or more recombinantly-introduced, temperature sensitive (ts) attenuating mutations or wherein the non-segmented negative-stranded RNA virus incorporates one or more attenuating mutation(s) identified in a biologically derived mutant non-segmented negative-stranded RNA virus or wherein the non-segmented negative-stranded RNA virus incorporates one or more attenuating mutation(s) corresponding to one or more mutations identified in a heterologous, mutant non-segmented negative-stranded RNA virus or wherein the non-segmented negative-stranded RNA virus incorporates at least one attenuating mutation specified by two or three nucleotide changes in a codon specifying the mutation or;
wherein the non-segmented negative-stranded RNA virus is recombinantly modified by a nucleotide modification specifying a phenotypic change selected from a change in growth characteristics, attenuation, temperature-sensitivity, cold-adaptation, plaque size, host-range restriction, or a change in immunogenicity preferably wherein the non-segmented negative-stranded RNA virus incorporates a recombinant modification that alters one or more C, C', D, E, F, G, HA, HN, L, M, M2-1, M2-2, NS1, NS2, SH, V, Y1, and/or Y2 open reading frames (ORFs) and/or a 3' leader, 5' trailer, and/or intergenic region within the genome or antigenome of the virus or;
wherein one or more gene(s) of the non-segmented negative-stranded RNA virus is deleted in whole or in part or expression of the gene(s) is reduced or ablated by a mutation in an RNA editing site, by a frameshift mutation, by a mutation that alters a translation start site, by introduction of one or more stop codons in an open reading frame (ORF) of the gene, or by a mutation in a transcription signal or;
wherein the non-segmented negative-stranded RNA virus is modified to encode a non-viral molecule selected from a cytokine, a T-helper epitope, a restriction site marker, or a protein of a microbial pathogen or parasite capable of eliciting an immune response in a mammalian host or;
wherein the polynucleotide encoding the genome or antigenome comprises a partial or complete vector genome or antigenome combined with one or more heterologous gene(s) or genome segment(s) encoding one or more antigenic determinant(s) of one or more heterologous pathogen(s) to form a chimeric genome or antigenome preferably wherein said one or more heterologous gene(s) or genome segment(s) encoding the antigenic determinant(s) is/are added as supernumerary gene(s) or genome segment(s) adjacent to or within a noncoding region of the partial or complete vector genome or antigenome, or wherein said one or more heterologous gene(s) or genome segment(s) encoding the antigenic determinant(s) is/are substituted for one or more counterpart gene(s) or genome segment(s) in a partial vector genome or antigenome or wherein the heterologous gene or genome segment is added or substituted at a position corresponding to a wild-type gene order position of a counterpart gene or genome segment within the partial or complete vector genome or antigenome or wherein the heterologous gene or genome segment is added or substituted at a position that is more promoter-proximal or promoter-distal compared to a wild-type gene order position of a counterpart gene or genome segment within the partial or complete vector genome or antigenome or wherein the vector genome or antigenome is modified to encode a chimeric glycoprotein incorporating one or more heterologous antigenic domains, fragments, or epitopes of a heterologous non-segmented negative-stranded RNA virus to form the chimeric genome or antigenome or wherein the heterologous pathogen is selected from measles virus, subgroup A and subgroup B respiratory syncytial viruses, human parainfluenza viruses, mumps virus, human papilloma viruses, type 1 and type 2, human immunodeficiency viruses, herpes simplex viruses, cytomegalovirus, rabies virus, human metapneuomovirus, Epstein Barr virus, filoviruses, bunyaviruses, flaviviruses, alphaviruses, and influenza viruses or wherein the chimeric genome or antigenome is recombinantly modified by introduction of one or more attenuating mutation(s) or wherein the chimeric genome or antigenome incorporates one or more attenuating mutation(s) identified in a biologically derived mutant non-segmented negative-stranded RNA virus or wherein the chimeric genome or antigenome is recombinantly modified by a nucleotide modification specifying a phenotypic change selected from a change in growth characteristics, attenuation, temperature-sensitivity, cold-adaptation, plaque size, host-range restriction, or a change in immunogenicity or wherein the chimeric genome or antigenome incorporates a recombinant modification that alters one or more C, C', D, E, F, G, HA, HN, L, M, M2-1, M2-2, NS1, NS2, SH, V, Y1, and/or Y2 open reading frames (ORFs) and/or a 3' leader, 5' trailer, and/or intergenic region within the genome or antigenome of the virus. Or wherein one or more gene(s) within the chimeric genome or antigenome is deleted in whole or in part or expression of the gene(s) is reduced or ablated by a mutation in an RNA editing site, by a frameshift mutation, by a mutation that alters a translation start site, by introduction of one or more stop codons in an open reading frame (ORF) of the gene, or by a mutation in a transcription signal or wherein the chimeric genome or antigenome is modified to encode a non-viral molecule selected from a cytokine, a T-helper epitope, a restriction site marker, or a protein of a microbial pathogen or parasite capable of eliciting an immune response in a mammalian host or;
wherein the polynucleotide encoding the genome or antigenome is modified by addition, substitution, or translocation of a gene or genome segment to a position that is more promoter-proximal or promoter-distal compared to a wild-type gene order position of said gene or genome segment within the genome or antigenome of said non-segmented negative-stranded RNA virus or;
wherein the polynucleotide encoding the genome or antigenome is modified to incorporate a heterologous regulatory element comprising an extragenic 3' leader or
5' trailer region, a gene-start signal, gene-end signal, editing region, intergenic region, or a 3' or 5' non-coding region or;
wherein the polynucleotide encoding the genome or antigenome is modified to incorporate a polynucleotide insertion of between 150 nucleotides (nts) and 4,000 nucleotides in length in a non-coding region (NCR) of the genome or antigenome or as a separate gene unit (GU), said polynucleotide insertion lacking a complete open reading frame (ORF) and specifying an attenuated phenotype in said non-segmented negative-stranded RNA virus or;
wherein the process further comprises co-introduction into and/or co-expression in said host cell of any one, or any combination of, structural protein(s) of the same or of a different non-segmented negative-stranded RNA virus or;
an immunogenic composition comprising an immunogenically effective amount of an isolated, infectious, non-segmented, negative-stranded RNA virus produced according to the process of claim 112 in a pharmaceutically acceptable carrier preferably wherein the non-segmented negative-stranded RNA virus is formulated in a dose of 10³ to 10⁷ PFU or formulated for administration to the upper respiratory tract or formulated for administration by spray, droplet or aerosol or wherein the host cell is co-cultured with a plaque expansion cell of the same or different cell type to allow spread of rescued virus to the plaque expansion cell or wherein said host cell and plaque expansion cell are each selected from HEp-2, HeLa, HEK, BHK, FRhL-DBS2, LLC-MK2, MRC-5, and Vero cells or wherein after one or more of said viral cDNA expression vector and a transient expression vector encoding the RNA polymerase are introduced into said host cell, the host cell is exposed to an effective heat shock under conditions sufficient to increase the recovery of the recombinant virus orfurther comprising co-introduction into and/or co-expression in said host cell of any one, or any combination of, structural protein(s) of the same or different non-segmented negative-stranded RNA virus.

17. A method for producing a growth- or replication-defective non-segmented, negative-stranded RNA virus of the Order Mononegavirales comprising the steps of:
(a) introducing a viral cDNA expression vector comprising a polynucleotide encoding a genome or antigenome of a growth- or replication-defective non-segmented negative-stranded RNA virus operably linked with an expression control sequence to direct synthesis of viral RNA transcripts from said cDNA expression vector into a host cell, wherein said host cell expresses an N protein, a P protein, and an L protein, and transiently expresses an RNA polymerase from a transiently-transfected expression vector; and
(b) recovering assembled growth- or replication-defective, non-segmented, negative-stranded RNA virus from said host cells preferably
wherein said virus is incapable of completing a single round of replication after it is assembled in said host cell or
wherein said virus is capable of growth and/or replication in vitro, but is growth- or replication-defective in vivo or;
wherein said transiently-transfected expression vector is a plasmid or;
wherein said viral cDNA expression vector and a transient expression vector that encodes and directs transient expression of the RNA polymerase are combined in a DNA transfection mixture and added to a host cell culture simultaneously to achieve coordinate transfection or;
wherein said viral cDNA expression vector and one or more support vector(s) encoding the N, P, L proteins are introduced into the host cell prior to introduction of a transiently-transfected expression vector encoding the RNA polymerase or;
wherein one or more of said viral cDNA expression vector and a transiently-transfected expression vector encoding the RNA polymerase is/are introduced into said host cell by calcium phosphate transfection or electroporation or;
wherein after one or more of said viral cDNA expression vector and a transiently-transfected expression vector encoding the RNA polymerase are introduced into said host cell, the host cell is exposed to an effective heat shock under conditions sufficient to increase the recovery of the recombinant virus preferably wherein the host cell is exposed to a heat shock temperature above 37°C or wherein the host cell is subjected to effective heat shock for at least about 5 minutes or;
wherein after a period of time sufficient to permit expression of said viral cDNA expression vector and a transiently-transfected expression vector encoding and directing expression of the RNA polymerase, the host cell is co-cultured with a plaque expansion cell of the same or different cell type to allow spread of rescued virus to the plaque expansion cell or
wherein said host cell is transferred to at least one layer of plaque expansion cells to establish a co-culture of host and plaque expansion cells or;
wherein said host cell is of a different cell type than said plaque expansion cell or; wherein the replication-defective, non-segmented, negative-stranded RNA virus is a subviral particle or;
wherein one or more of said N, P and L proteins is/are of a heterologous non-segmented, negative-stranded RNA virus or;
wherein the non-segmented, negative-stranded RNA virus is a member of the family paramyxoviridae or;
wherein the non-segmented, negative-stranded RNA virus is a member of the family rhabdoviridae or;
wherein the non-segmented negative-stranded RNA virus is recombinantly modified by introduction of one or more attenuating mutation(s)or;
wherein the non-segmented negative-stranded RNA virus is recombinantly modified by a nucleotide modification specifying a phenotypic change selected from a change in growth characteristics, attenuation, temperature-sensitivity, cold-adaptation, plaque size, host-range restriction, or a change in immunogenicity or;
wherein one or more gene(s) of the non-segmented negative-stranded RNA virus is deleted in whole or in part or expression of the gene(s) is reduced or ablated by a mutation in an RNA editing site, by a frameshift mutation, by a mutation that alters a translation start site, by introduction of one or more stop codons in an open reading frame (ORF) of the gene, or by a mutation in a transcription signal or;
wherein the polynucleotide encoding the genome or antigenome comprises a partial or complete vector genome or antigenome combined with one or more heterologous gene(s) or genome segment(s) encoding one or more antigenic determinant(s) of one or more heterologous pathogen(s) to form a chimeric genome or antigenome preferably wherein the heterologous gene or genome segment is added or substituted at a position that is more promoter-proximal or promoter-distal compared to a wild-type gene order position of a counterpart gene or genome segment within the partial or complete vector genome or antigenome.
Or wherein the chimeric genome or antigenome is recombinantly modified by introduction of one or more attenuating mutation(s) or wherein the chimeric genome or antigenome is recombinantly modified by a nucleotide modification specifying a phenotypic change selected from a change in growth characteristics, attenuation, temperature-sensitivity, cold-adaptation, plaque size, host-range restriction, or a change in immunogenicity or;
wherein the polynucleotide encoding the genome or antigenome is modified by addition, substitution, or translocation of a gene or genome segment to a position that is more promoter-proximal or promoter-distal compared to a wild-type gene order position of said gene or genome segment within the genome or antigenome of said non-segmented negative-stranded RNA virus or;
wherein the polynucleotide encoding the genome or antigenome is modified to incorporate a heterologous regulatory element comprising an extragenic 3' leader or 5' trailer region, a gene-start signal, gene-end signal, editing region, intergenic region, or a 3' or 5' non-coding region or;
wherein the polynucleotide encoding the genome or antigenome is modified to incorporate a polynucleotide insertion of between 150 nucleotides (nts) and 4,000 nucleotides in length in a non-coding region (NCR) of the genome or antigenome or as a separate gene unit (GU), said polynucleotide insertion lacking a complete open reading frame (ORF) and specifying an attenuated phenotype in said non-segmented negative-stranded RNA virus or;
further comprising co-introduction into and/or co-expression in said host cell of any one, or any combination of, structural protein(s) of the same or different non-segmented negative-stranded RNA virus.

18. A method for producing a growth- or replication-defective, non-segmented, negative-stranded RNA virus of the Order Mononegavirales comprising the steps of:
introducing into a suitable host cell a viral cDNA expression vector comprising a polynucleotide encoding a genome or antigenome of a growth- or replication-defective non-segmented negative-stranded RNA virus operably linked with an expression control sequence to direct synthesis of viral RNA transcripts from said viral cDNA expression vector, and one or more transient expression vectors that encode(s) and direct(s) transient expression of an RNA polymerase, an N protein, a P protein, and an L protein under conditions sufficient to permit the co-expression of said vectors and production of the recombinant virus;
transferring said host cell into co-culture with a plaque expansion cell; and
recovering assembled growth- or replication-defective, non-segmented, negative-stranded RNA virus from said co-culture preferably wherein the replication-defective, non-segmented, negative-stranded RNA virus is incapable of completing a round of replication after it is assembled in said host cell, or is capable of growth and/or replication in vitro, but is growth- or replication-defective in vivo or;
a method for producing a growth- or replication-defective, non-segmented, negative-stranded RNA virus of the Order Mononegavirales comprising the steps of:
(a) introducing a viral cDNA expression vector comprising a polynucleotide encoding a genome or antigenome of a growth- or replication-defective non-segmented negative-stranded RNA virus operably linked with an expression control sequence to direct synthesis of viral RNA transcripts from said cDNA expression vector, into a host cell transiently expressing:
(1) an RNA polymerase,
(2) an N protein,
(3) a P protein, and
(4) an L protein,
wherein each of said RNA polymerase and N, P, and L proteins is expressed from a transiently transfected expression vector; and
(b) recovering assembled growth- or replication-defective, non-segmented, negative-stranded RNA virus from said host cells preferably wherein the replication-defective, non-segmented, negative-stranded RNA virus is incapable of completing a round of replication after it is assembled in said host cell, or is capable of growth and/or replication in vitro, but is growth- or replication-defective in vivo or further comprising co-introduction into and/or co-expression in said host cell of any one, or any combination of, structural protein(s) of the same or different non-segmented negative-stranded RNA virus or;
a method for producing a growth- or replication-defective, non-segmented, negative-stranded RNA virus of the Order Mononegavirales comprising the steps of:
introducing into a suitable host cell a viral cDNA expression vector comprising a polynucleotide encoding a genome or antigenome of a growth- or replication-defective non-segmented negative-stranded RNA virus operably linked with an expression control sequence to direct synthesis of viral RNA transcripts from said viral cDNA expression vector, and one or more transient expression vectors that encode(s) and direct(s) transient expression of an RNA polymerase, an N protein, a P protein, and an L protein; and
recovering assembled growth- or replication-defective, non-segmented, negative-stranded RNA virus from said host cells preferably further comprising co-introduction into and/or co-expression in said host cell of any one, or any combination of, structural protein(s) of the same or different non-segmented negative-stranded RNA virus.

19. A composition for producing a growth- or replication-defective, non-segmented, negative-stranded RNA virus of the Order Mononegavirales comprising:
(a) a viral cDNA expression vector comprising a polynucleotide encoding a genome or antigenome of a growth- or replication-defective non-segmented negative-stranded RNA virus operably linked with an expression control sequence to direct synthesis of viral RNA transcripts from said cDNA in a suitable host cell;
(b) one or more expression vectors that encode(s) and direct(s) transient expression in said host cell of:
(1) an N protein,
(2) a P protein, and
(3) an L protein; and
(c) a transient expression vector that encodes and directs transient expression in said host cell of an RNA polymerase preferably wherein said virus is incapable of completing a single round of replication after it is assembled in said host cell, or is capable of growth and/or replication in vitro, but is growth- or replication-defective in vivo or wherein said RNA polymerase is a T7 RNA polymerase or wherein said transiently-transfected expression vector is a plasmid or further comprising at least one layer of plaque expansion cells to establish a co-culture of host and plaque expansion cells or wherein the replication-defective, non-segmented, negative-stranded RNA virus is a subviral particle or wherein the non-segmented, negative-stranded RNA virus is a member of the family paramyxoviridae or wherein the non-segmented, negative-stranded RNA virus is a member of the family rhabdoviridae or wherein the non-segmented negative-stranded RNA virus is recombinantly modified by introduction of one or more attenuating mutation(s) or wherein the non-segmented negative-stranded RNA virus is recombinantly modified by a nucleotide modification specifying a phenotypic change selected from a change in growth characteristics, attenuation, temperature-sensitivity, cold-adaptation, plaque size, host-range restriction, or a change in immunogenicity or wherein one or more gene(s) of the non-segmented negative-stranded RNA virus is deleted in whole or in part or expression of the gene(s) is reduced or ablated by a mutation in an RNA editing site, by a frameshift mutation, by a mutation that alters a translation start site, by introduction of one or more stop codons in an open reading frame (ORF) of the gene, or by a mutation in a transcription signal or wherein the polynucleotide encoding the genome or antigenome comprises a partial or complete vector genome or antigenome combined with one or more heterologous gene(s) or genome segment(s) encoding one or more antigenic determinant(s) of one or more heterologous pathogen(s) to form a chimeric genome or antigenome or wherein the polynucleotide encoding the genome or antigenome is modified by addition, substitution, or translocation of a gene or genome segment to a position that is more promoter-proximal or promoter-distal compared to a wild-type gene order position of said gene or genome segment within the genome or antigenome of said non-segmented negative-stranded RNA virus or wherein one or more expression vectors additionally encode(s) one, or any combination of, structural protein(s) of the same or different non-segmented negative-stranded RNA virus.

20. A growth- or replication-defective, non-segmented, negative-stranded RNA virus of the Order Mononegavirales produced by the process of:
introducing into a suitable host cell a viral cDNA expression vector comprising a polynucleotide encoding a genome or antigenome of a growth- or replication-defective non-segmented negative-stranded RNA virus operably linked with an expression control sequence to direct synthesis of viral RNA transcripts from said cDNA expression vector, one or more support vector(s) that encode(s) and direct(s) expression of an N protein, a P protein, and an L protein, and a transient expression vector that encodes and directs transient expression of an RNA polymerase; and
recovering assembled growth- or replication-defective, non-segmented, negative-stranded RNA virus from said host cells or;
an immunogenic composition comprising an immunogenically effective amount of an isolated, growth- or replication-defective, non-segmented, negative-stranded RNA virus produced according to the process of claim 227 in a pharmaceutically acceptable carrier or;
a method for stimulating the immune system of a mammalian subject to induce an immune response in the subject against a non-segmented negative-stranded RNA virus which comprises administering to the subject an immunogenically effective amount of an isolated, growth- or replication-defective, non-segmented, negative-stranded RNA virus produced according to the process of claim 227 in a pharmaceutically acceptable carrier.


SEQUENCE LISTING

<110> WYETH

<120> IMPROVED METHOD FOR THE RECOVERY OF NON-SEGMENTED,
      NEGATIVE-STRANDED RNA VIRUSES FROM cDNA

<130> 2302-001-04

<140> PCT/US04/018305
<141> 2004-06-08

<150> 60/477,389
<151> 2003-06-09

<160> 90

<170> PatentIn Ver. 3.3

<210> 1
<211> 8111
<212> DNA
<213> Artificial Sequence

<220>
<223> Description of Artificial Sequence: Synthetic
      nucleotide sequence construct

<400> 1
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttgct agcctcgagt 1080
gatcagaatt cgccaccatg gacacgatta acatcgctaa gaacgacttc tctgacatcg 1140
aactggctgc tatcccgttc aacactctgg ctgaccatta cggtgagcgt ttagctcgcg 1200
aacagttggc ccttgagcat gagtcttacg agatgggtga agcacgcttc cgcaagatgt 1260
ttgagcgtca acttaaagct ggtgaggttg cggataacgc tgccgccaag cctctcatca 1320
ctaccctact ccctaagatg attgcacgca tcaacgactg gtttgaggaa gtgaaagcta 1380
agcgcggcaa gcgcccgaca gccttccagt tcctgcaaga aatcaagccg gaagccgtag 1440
cgtacatcac cattaagacc actctggctt gcctaaccag tgctgacaat acaaccgttc 1500
aggctgtagc aagcgcaatc ggtcggccca ttgaggacga ggctcgcttc ggtcgtatcc 1560
gtgaccttga agctaagcac ttcaagaaaa acgttgagga acaactcaac aagcgcgtag 1620
ggcacgtcta caagaaagca tttatgcaag ttgtcgaggc tgacatgctc tctaagggtc 1680
tactcggtgg cgaggcgtgg tcttcgtggc ataaggaaga ctctattcat gtaggagtac 1740
gctgcatcga gatgctcatt gagtcaaccg gaatggttag cttacaccgc caaaatgctg 1800
gcgtagtagg tcaagactct gagactatcg aactcgcacc tgaatacgct gaggctatcg 1860
caacccgtgc aggtgcgctg gctggcatct ctccgatgtt ccaaccttgc gtagttcctc 1920
ctaagccgtg gactggcatt actggtggtg gctattgggc taacggtcgt cgtcctctgg 1980
cgctggtgcg tactcacagt aagaaagcac tgatgcgcta cgaagacgtt tacatgcctg 2040
aggtgtacaa agcgattaac attgcgcaaa acaccgcatg gaaaatcaac aagaaagtcc 2100
tagcggtcgc caacgtaatc accaagtgga agcattgtcc ggtcgaggac atccctgcga 2160
ttgagcgtga agaactcccg atgaaaccgg aagacatcga catgaatcct gaggctctca 2220
ccgcgtggaa acgtgctgcc gctgctgtgt accgcaagga caaggctcgc aagtctcgcc 2280
gtatcagcct tgagttcatg cttgagcaag ccaataagtt tgctaaccat aaggccatct 2340
ggttccctta caacatggac tggcgcggtc gtgtttacgc tgtgtcaatg ttcaacccgc 2400
aaggtaacga tatgaccaaa ggactgctta cgctggcgaa aggtaaacca atcggtaagg 2460
aaggttacta ctggctgaaa atccacggtg caaactgtgc gggtgtcgat aaggttccgt 2520
tccctgagcg catcaagttc attgaggaaa accacgagaa catcatggct tgcgctaagt 2580
ctccactgga gaacacttgg tgggctgagc aagattctcc gttctgcttc cttgcgttct 2640
gctttgagta cgctggggta cagcaccacg gcctgagcta taactgctcc cttccgctgg 2700
cgtttgacgg gtcttgctct ggcatccagc acttctccgc gatgctccga gatgaggtag 2760
gtggtcgcgc ggttaacttg cttcctagtg aaaccgttca ggacatctac gggattgttg 2820
ctaagaaagt caacgagatt ctacaagcag acgcaatcaa tgggaccgat aacgaagtag 2880

ttaccgtgac cgatgagaac actggtgaaa tctctgagaa agtcaagctg ggcactaagg 2940
cactggctgg tcaatggctg gcttacggtg ttactcgcag tgtgactaag cgttcagtca 3000
tgacgctggc ttacgggtcc aaagagttcg gcttccgtca acaagtgctg gaagatacca 3060
ttcagccagc tattgattcc ggcaagggtc tgatgttcac tcagccgaat caggctgctg 3120
gatacatggc taagctgatt tgggaatctg tgagcgtgac ggtggtagct gcggttgaag 3180
caatgaactg gcttaagtct gctgctaagc tgctggctgc tgaggtcaaa gataagaaga 3240
ctggagagat tcttcgcaag cgttgcgctg tgcattgggt aactcctgat ggtttccctg 3300
tgtggcagga atacaagaag cctattcaga cgcgcttgaa cctgatgttc ctcggtcagt 3360
tccgcttaca gcctaccatt aacaccaaca aagatagcga gattgatgca cacaaacagg 3420
agtctggtat cgctcctaac tttgtacaca gccaagacgg tagccacctt cgtaagactg 3480
tagtgtgggc acacgagaag tacggaatcg aatcttttgc actgattcac gactccttcg 3540
gtaccattcc ggctgacgct gcgaacctgt tcaaagcagt gcgcgaaact atggttgaca 3600
catatgagtc ttgtgatgta ctggctgatt tctacgacca gttcgctgac cagttgcacg 3660
agtctcaatt ggacaaaatg ccagcacttc cggctaaagg taacttgaac ctccgtgaca 3720
tcttagagtc ggacttcgcg ttcgcgtaac tctagagtcg acccgggcgg ccgcttccct 3780
ttagtgaggg ttaatgcttc gagcagacat gataagatac attgatgagt ttggacaaac 3840
cacaactaga atgcagtgaa aaaaatgctt tatttgtgaa atttgtgatg ctattgcttt 3900
atttgtaacc attataagct gcaataaaca agttaacaac aacaattgca ttcattttat 3960
gtttcaggtt cagggggaga tgtgggaggt tttttaaagc aagtaaaacc tctacaaatg 4020
tggtaaaatc cgataaggat cgatccgggc tggcgtaata gcgaagaggc ccgcaccgat 4080
cgcccttccc aacagttgcg cagcctgaat ggcgaatgga cgcgccctgt agcggcgcat 4140
taagcgcggc gggtgtggtg gttacgcgca gcgtgaccgc tacacttgcc agcgccctag 4200
cgcccgctcc tttcgctttc ttcccttcct ttctcgccac gttcgccggc tttccccgtc 4260
aagctctaaa tcgggggctc cctttagggt tccgatttag tgctttacgg cacctcgacc 4320
ccaaaaaact tgattagggt gatggttcac gtagtgggcc atcgccctga tagacggttt 4380
ttcgcccttt gacgttggag tccacgttct ttaatagtgg actcttgttc caaactggaa 4440
caacactcaa ccctatctcg gtctattctt ttgatttata agggattttg ccgatttcgg 4500
cctattggtt aaaaaatgag ctgatttaac aaatatttaa cgcgaatttt aacaaaatat 4560
taacgtttac aatttcgcct gatgcggtat tttctcctta cgcatctgtg cggtatttca 4620
caccgcatac gcggatctgc gcagcaccat ggcctgaaat aacctctgaa agaggaactt 4680
ggttaggtac cttctgaggc ggaaagaacc agctgtggaa tgtgtgtcag ttagggtgtg 4740
gaaagtcccc aggctcccca gcaggcagaa gtatgcaaag catgcatctc aattagtcag 4800
caaccaggtg tggaaagtcc ccaggctccc cagcaggcag aagtatgcaa agcatgcatc 4860
tcaattagtc agcaaccata gtcccgcccc taactccgcc catcccgccc ctaactccgc 4920
ccagttccgc ccattctccg ccccatggct gactaatttt ttttatttat gcagaggccg 4980
aggccgcctc ggcctctgag ctattccaga agtagtgagg aggctttttt ggaggcctag 5040
gcttttgcaa aaagcttgat tcttctgaca caacagtctc gaacttaagg ctagagccac 5100
catgattgaa caagatggat tgcacgcagg ttctccggcc gcttgggtgg agaggctatt 5160
cggctatgac tgggcacaac agacaatcgg ctgctctgat gccgccgtgt tccggctgtc 5220
agcgcagggg cgcccggttc tttttgtcaa gaccgacctg tccggtgccc tgaatgaact 5280
gcaggacgag gcagcgcggc tatcgtggct ggccacgacg ggcgttcctt gcgcagctgt 5340
gctcgacgtt gtcactgaag cgggaaggga ctggctgcta ttgggcgaag tgccggggca 5400
ggatctcctg tcatctcacc ttgctcctgc cgagaaagta tccatcatgg ctgatgcaat 5460
gcggcggctg catacgcttg atccggctac ctgcccattc gaccaccaag cgaaacatcg 5520
catcgagcga gcacgtactc ggatggaagc cggtcttgtc gatcaggatg atctggacga 5580
agagcatcag gggctcgcgc cagccgaact gttcgccagg ctcaaggcgc gcatgcccga 5640
cggcgaggat ctcgtcgtga cccatggcga tgcctgcttg ccgaatatca tggtggaaaa 5700
tggccgcttt tctggattca tcgactgtgg ccggctgggt gtggcggacc gctatcagga 5760
catagcgttg gctacccgtg atattgctga agagcttggc ggcgaatggg ctgaccgctt 5820
cctcgtgctt tacggtatcg ccgctcccga ttcgcagcgc atcgccttct atcgccttct 5880
tgacgagttc ttctgagcgg gactctgggg ttcgaaatga ccgaccaagc gacgcccaac 5940
ctgccatcac gatggccgca ataaaatatc tttattttca ttacatctgt gtgttggttt 6000
tttgtgtgaa tcgatagcga taaggatccg cgtatggtgc actctcagta caatctgctc 6060
tgatgccgca tagttaagcc agccccgaca cccgccaaca cccgctgacg cgccctgacg 6120
ggcttgtctg ctcccggcat ccgcttacag acaagctgtg accgtctccg ggagctgcat 6180
gtgtcagagg ttttcaccgt catcaccgaa acgcgcgaga cgaaagggcc tcgtgatacg 6240
cctattttta taggttaatg tcatgataat aatggtttct tagacgtcag gtggcacttt 6300
tcggggaaat gtgcgcggaa cccctatttg tttatttttc taaatacatt caaatatgta 6360
tccgctcatg agacaataac cctgataaat gcttcaataa tattgaaaaa ggaagagtat 6420
gagtattcaa catttccgtg tcgcccttat tccctttttt gcggcatttt gccttcctgt 6480
ttttgctcac ccagaaacgc tggtgaaagt aaaagatgct gaagatcagt tgggtgcacg 6540
agtgggttac atcgaactgg atctcaacag cggtaagatc cttgagagtt ttcgccccga 6600
agaacgtttt ccaatgatga gcacttttaa agttctgcta tgtggcgcgg tattatcccg 6660
tattgacgcc gggcaagagc aactcggtcg ccgcatacac tattctcaga atgacttggt 6720
tgagtactca ccagtcacag aaaagcatct tacggatggc atgacagtaa gagaattatg 6780
cagtgctgcc ataaccatga gtgataacac tgcggccaac ttacttctga caacgatcgg 6840
aggaccgaag gagctaaccg cttttttgca caacatgggg gatcatgtaa ctcgccttga 6900
tcgttgggaa ccggagctga atgaagccat accaaacgac gagcgtgaca ccacgatgcc 6960
tgtagcaatg gcaacaacgt tgcgcaaact attaactggc gaactactta ctctagcttc 7020
ccggcaacaa ttaatagact ggatggaggc ggataaagtt gcaggaccac ttctgcgctc 7080
ggcccttccg gctggctggt ttattgctga taaatctgga gccggtgagc gtgggtctcg 7140
cggtatcatt gcagcactgg ggccagatgg taagccctcc cgtatcgtag ttatctacac 7200
gacggggagt caggcaacta tggatgaacg aaatagacag atcgctgaga taggtgcctc 7260
actgattaag cattggtaac tgtcagacca agtttactca tatatacttt agattgattt 7320
aaaacttcat ttttaattta aaaggatcta ggtgaagatc ctttttgata atctcatgac 7380
caaaatccct taacgtgagt tttcgttcca ctgagcgtca gaccccgtag aaaagatcaa 7440
aggatcttct tgagatcctt tttttctgcg cgtaatctgc tgcttgcaaa caaaaaaacc 7500

accgctacca gcggtggttt gtttgccgga tcaagagcta ccaactcttt ttccgaaggt 7560
aactggcttc agcagagcgc agataccaaa tactgtcctt ctagtgtagc cgtagttagg 7620
ccaccacttc aagaactctg tagcaccgcc tacatacctc gctctgctaa tcctgttacc 7680
agtggctgct gccagtggcg ataagtcgtg tcttaccggg ttggactcaa gacgatagtt 7740
accggataag gcgcagcggt cgggctgaac ggggggttcg tgcacacagc ccagcttgga 7800
gcgaacgacc tacaccgaac tgagatacct acagcgtgag ctatgagaaa gcgccacgct 7860
tcccgaaggg agaaaggcgg acaggtatcc ggtaagcggc agggtcggaa caggagagcg 7920
cacgagggag cttccagggg gaaacgcctg gtatctttat agtcctgtcg ggtttcgcca 7980
cctctgactt gagcgtcgat ttttgtgatg ctcgtcaggg gggcggagcc tatggaaaaa 8040
cgccagcaac gcggcctttt tacggttcct ggccttttgc tggccttttg ctcacatggc 8100
tcgacagatc t 8111

<210> 2
<211> 41
<212> DNA
<213> Human parainfluenza virus 1

<400> 2
gcttggtata tataataata ttaaatagta ttttagggtt t 41

<210> 3
<211> 41
<212> DNA
<213> Human parainfluenza virus 1

<400> 3
gtttggaata tataataata ttaaatagta ttttagggtt a 41

<210> 4
<211> 41
<212> DNA
<213> Human parainfluenza virus 3

<400> 4
gtctgggaat ataaatttaa ctttaaatta acttaggatt a 41

<210> 5
<211> 41
<212> DNA
<213> Human parainfluenza virus 3

<400> 5
gcttggtaat ataaatttaa cttaaaatta acttaggatt t 41

<210> 6
<211> 14
<212> PRT
<213> Human parainfluenza virus 1

<400> 6
Thr Asn Gly Val Asn Ala Asp Ala Lys Tyr Val Ile Tyr Asn
1               5                   10

<210> 7
<211> 14
<212> PRT
<213> Human parainfluenza virus 1

<400> 7
Thr Asn Gly Val Asn Ala Asp Val Lys Tyr Val Ile Tyr Asn
1               5                   10

<210> 8
<211> 14
<212> PRT
<213> Human parainfluenza virus 3

<400> 8
Thr Asn Gly Ser Asn Ala Asp Val Lys Tyr Val Ile Tyr Met
1               5                   10

<210> 9
<211> 14
<212> PRT
<213> Human parainfluenza virus 3

<400> 9
Thr Asn Gly Ser Asn Ala Asp Ala Lys Tyr Val Ile Tyr Met
1               5                   10


<210> 10
<211> 12
<212> PRT
<213> Human parainfluenza virus 1

<400> 10
Glu Leu Gly Val Thr Asp Thr Ala Lys Glu Arg Leu
1               5                   10


<210> 11
<211> 12
<212> PRT
<213> Human parainfluenza virus 1

<400> 11
Glu Leu Gly Val Thr Asp Thr Ala Lys Glu Arg Leu
1               5                   10


<210> 12
<211> 12
<212> PRT
<213> Human parainfluenza virus 3

<400> 12
Glu Leu Gly Val Thr His Glu Ser Lys Glu Ser Leu
1               5                   10


<210> 13
<211> 12
<212> PRT
<213> Human parainfluenza virus 3

<400> 13
Glu Leu Gly Val Thr His Glu Ala Lys Glu Ser Leu
1               5                   10


<210> 14
<211> 21
<212> PRT
<213> Human parainfluenza virus 1

<400> 14
Lys Gln Arg His Met Leu Glu Thr Leu Ile Asn Lys Val Tyr Thr Gly
1               5                   10                  15

Pro Leu Gly Glu Glu
            20


<210> 15
<211> 21
<212> PRT
<213> Human parainfluenza virus 1

<400> 15
Lys Gln Arg His Met Leu Glu Ser Leu Ile Asn Lys Val Tyr Thr Gly
1               5                   10                  15

Pro Leu Gly Glu Glu
            20

<210> 16
<211> 21
<212> PRT
<213> Human parainfluenza virus 3

<400> 16
Gln Gln Lys Gln Lys Ile Glu Ile Leu Ile Arg Lys Leu Tyr Arg Glu
1               5                   10                  15

Asp Leu Gly Glu Glu
            20


<210> 17
<211> 21
<212> PRT
<213> Human parainfluenza virus 3

<400> 17
Gln Gln Lys Gln Lys Ile Glu Thr Leu Ile Arg Lys Leu Tyr Arg Glu
1               5                   10                  15

Asp Leu Gly Glu Glu
            20


<210> 18
<211> 15
<212> PRT
<213> Human parainfluenza virus 1

<400> 18
Thr Leu Ala Asp Leu Ala Leu Thr Asn Ser Ile Ser Val Asn Leu
1               5                   10                  15


<210> 19
<211> 15
<212> PRT
<213> Human parainfluenza virus 1

<400> 19
Thr Leu Ala Asp Leu Ala Leu Pro Asn Ser Ile Ser Val Asn Leu
1               5                   10                  15


<210> 20
<211> 15
<212> PRT
<213> Human parainfluenza virus 3

<400> 20
Ser Met Ala Ser Leu Ser Leu Pro Asn Thr Ile Ser Ile Asn Leu
1               5                   10                  15


<210> 21
<211> 15
<212> PRT
<213> Human parainfluenza virus 3

<400> 21
Ser Met Ala Ser Leu Ser Leu Thr Asn Thr Ile Ser Ile Asn Leu
1               5                   10                  15


<210> 22
<211> 9
<212> PRT
<213> Human parainfluenza virus 1

<400> 22
Cys Gly Leu Ile Gly Val Asn Gly Ile
1               5

<210> 23
<211> 12
<212> PRT
<213> Human parainfluenza virus 1

<400> 23
Val Gly Pro Ala Val Thr Ile Arg Pro Val Asp Ile
1               5                   10


<210> 24
<211> 9
<212> PRT
<213> Human parainfluenza virus 1

<400> 24
Cys Gly Leu Ile Gly Ile Asn Gly Ile
1               5


<210> 25
<211> 12
<212> PRT
<213> Human parainfluenza virus 1

<400> 25
Val Gly Pro Ala Val Ser Ile Arg Pro Val Asp Ile
1               5                   10


<210> 26
<211> 9
<212> PRT
<213> Human parainfluenza virus 3

<400> 26
Cys Ser Thr Ile Gly Ile Asn Gly Met
1               5


<210> 27
<211> 12
<212> PRT
<213> Human parainfluenza virus 3

<400> 27
Leu Asn Asn Ser Val Ala Leu Asp Pro Ile Asp Ile
1               5                   10


<210> 28
<211> 9
<212> PRT
<213> Human parainfluenza virus 3

<400> 28
Cys Ser Thr Ile Gly Val Asn Gly Met
1               5


<210> 29
<211> 12
<212> PRT
<213> Human parainfluenza virus 3

<400> 29
Leu Asn Asn Ser Val Thr Leu Asp Pro Ile Asp Ile
1               5                   10


<210> 30
<211> 14
<212> PRT
<213> Human parainfluenza virus 1

<400> 30
Arg Gln Val Val Asn Val Leu Ile Ala Ile Asn Asn Tyr Leu

1                   5                       10


<210> 31
<211> 14
<212> PRT
<213> Human parainfluenza virus 1

<400> 31
Arg Gln Val Val Asn Val Leu Ile Arg Ile Asn Asn Tyr Leu
1                   5                       10


<210> 32
<211> 14
<212> PRT
<213> Human parainfluenza virus 3

<400> 32
Arg Arg Met Val Asn Ser Ile Ile Val Val Asp Lys Gly Leu
1                   5                       10


<210> 33
<211> 14
<212> PRT
<213> Human parainfluenza virus 3

<400> 33
Arg Arg Met Val Asn Ser Ile Ile Ala Val Asp Lys Gly Leu
1                   5                       10


<210> 34
<211> 13
<212> PRT
<213> Human parainfluenza virus 1

<400> 34
Ile Gly Gly Phe Asn His Met Ser Thr Ala Arg Cys Phe
1                   5                       10


<210> 35
<211> 14
<212> PRT
<213> Human parainfluenza virus 1

<400> 35
Pro Gly Asp Ser Ser Phe Phe Asp Trp Ala Ser Asp Pro Tyr
1                   5                       10


<210> 36
<211> 13
<212> PRT
<213> Human parainfluenza virus 1

<400> 36
Ile Gly Gly Phe Asn Tyr Met Ser Thr Ala Arg Cys Phe
1                   5                       10


<210> 37
<211> 14
<212> PRT
<213> Human parainfluenza virus 1

<400> 37
Pro Gly Asp Ser Ser Phe Leu Asp Trp Ala Ser Asp Pro Tyr
1                   5                       10


<210> 38
<211> 13
<212> PRT
<213> Human parainfluenza virus 3

<400> 38
Val Gly Gly Phe Asn Tyr Met Ala Met Ser Arg Cys Phe
1               5                   10

<210> 39
<211> 14
<212> PRT
<213> Human parainfluenza virus 3

<400> 39
Pro Gly Glu Ser Ser Phe Leu Asp Trp Ala Ser Asp Pro Tyr
1               5                   10

<210> 40
<211> 13
<212> PRT
<213> Human parainfluenza virus 3

<400> 40
Val Gly Gly Phe Asn His Met Ala Met Ser Arg Cys Phe
1               5                   10

<210> 41
<211> 14
<212> PRT
<213> Human parainfluenza virus 3

<400> 41
Pro Gly Glu Ser Ser Phe Phe Asp Trp Ala Ser Asp Pro Tyr
1               5                   10

<210> 42
<211> 13
<212> PRT
<213> Human parainfluenza virus 1

<400> 42
Pro Val Tyr Gly Pro Asn Ile Ser Asn Gln Asp Lys Ile
1               5                   10

<210> 43
<211> 13
<212> PRT
<213> Human parainfluenza virus 1

<400> 43
Pro Val Tyr Gly Pro Asn Leu Ser Asn Gln Asp Lys Ile
1               5                   10

<210> 44
<211> 13
<212> PRT
<213> Human parainfluenza virus 3

<400> 44
Pro Ile Tyr Gly Pro Asn Thr Ala Ser Gln Asp Gln Ile
1               5                   10

<210> 45
<211> 13
<212> PRT
<213> Human parainfluenza virus 3

<400> 45
Pro Ile Tyr Gly Pro Asn Ile Ala Ser Gln Asp Gln Ile
1               5                   10

<210> 46

<211> 17
<212> PRT
<213> Human parainfluenza virus 1

<400> 46
Lys Thr Lys Leu Arg Asp Ser Gln Lys Arg Tyr Glu Glu Val His Pro
1               5                   10                  15

Tyr

<210> 47
<211> 17
<212> PRT
<213> Human parainfluenza virus 1

<400> 47
Lys Thr Lys Leu Arg Asp Phe Gln Lys Arg Tyr Glu Glu Val His Pro
1               5                   10                  15

Tyr

<210> 48
<211> 17
<212> PRT
<213> Murine parainfluenza virus

<400> 48
Lys Thr Lys Leu Lys Asp Phe Gln Lys Arg Tyr Glu Glu Val His Pro
1               5                   10                  15

Tyr

<210> 49
<211> 17
<212> PRT
<213> Murine parainfluenza virus

<400> 49
Lys Thr Lys Leu Lys Asp Ser Gln Lys Arg Tyr Glu Glu Val His Pro
1               5                   10                  15

Tyr

<210> 50
<211> 18
<212> PRT
<213> Human parainfluenza virus 1

<400> 50
Ser Phe Ile Gly Phe Lys Leu Leu Lys Phe Ile Glu Pro Gln Leu Asp
1               5                   10                  15

Glu Asp

<210> 51
<211> 18
<212> PRT
<213> Human parainfluenza virus 1

<400> 51
Ser Phe Ile Gly Phe Lys Phe Leu Lys Phe Ile Glu Pro Gln Leu Asp
1               5                   10                  15

Glu Asp

<210> 52
<211> 18
<212> PRT
<213> Human parainfluenza virus 3

<400> 52
Ser Phe Ile Gly Ile Lys Phe Asn Lys Phe Ile Glu Pro Gln Leu Asp
1               5                   10                  15

Glu Asp

<210> 53
<211> 18
<212> PRT
<213> Human parainfluenza virus 3

<400> 53
Ser Phe Ile Gly Ile Lys Leu Asn Lys Phe Ile Glu Pro Gln Leu Asp
1               5                   10                  15

Glu Asp

<210> 54
<211> 12
<212> DNA
<213> Respiratory syncytial virus

<400> 54
aaatcagtta aa                                                          12

<210> 55
<211> 12
<212> DNA
<213> Respiratory syncytial virus

<400> 55
aaatcaatta aa                                                          12

<210> 56
<211> 21
<212> DNA
<213> Respiratory syncytial virus

<400> 56
atatcaaata tagaaactgt g                                                21

<210> 57
<211> 21
<212> DNA
<213> Respiratory syncytial virus

<400> 57
atatcaaata tagcaactgt g                                                21

<210> 58
<211> 12
<212> DNA
<213> Respiratory syncytial virus

<400> 58
ggagattgta ta                                                          12

<210> 59
<211> 12
<212> DNA
<213> Respiratory syncytial virus

<400> 59

ggagattaca ta                                                          12

<210> 60
<211> 30
<212> DNA
<213> Respiratory syncytial virus

<400> 60
catataagga ttgctaattc tgaattagaa                                      30

<210> 61
<211> 30
<212> DNA
<213> Respiratory syncytial virus

<400> 61
tacataagga ttgctaattc tgagctcgaa                                      30

<210> 62
<211> 18
<212> DNA
<213> Respiratory syncytial virus

<400> 62
actcatgctc aagcagat                                                   18

<210> 63
<211> 18
<212> DNA
<213> Respiratory syncytial virus

<400> 63
actcatgctt tagcagat                                                   18

<210> 64
<211> 18
<212> DNA
<213> Respiratory syncytial virus

<400> 64
cttccattgg attgtaac                                                   18

<210> 65
<211> 18
<212> DNA
<213> Respiratory syncytial virus

<400> 65
cttccactcg agtgtaac                                                   18

<210> 66
<211> 27
<212> DNA
<213> Respiratory syncytial virus

<400> 66
cgtttctatc gtgagtttcg gttgcct                                         27

<210> 67
<211> 27
<212> DNA
<213> Respiratory syncytial virus

<400> 67
cgtctatatc gtgagtttcg gttacct                                         27

<210> 68
<211> 12

<212> DNA
<213> Respiratory syncytial virus

<400> 68
ctcaaaaatg at                                                          12


<210> 69
<211> 12
<212> DNA
<213> Respiratory syncytial virus

<400> 69
ctcaaaatag at                                                          12


<210> 70
<211> 18
<212> DNA
<213> Respiratory syncytial virus

<400> 70
gccactgaga tgatgagg                                                    18


<210> 71
<211> 18
<212> DNA
<213> Respiratory syncytial virus

<400> 71
gccactgaga tggtcagg                                                    18


<210> 72
<211> 21
<212> DNA
<213> Respiratory syncytial virus

<400> 72
acccttgggt taacatatga a                                                21


<210> 73
<211> 21
<212> DNA
<213> Respiratory syncytial virus

<400> 73
accttagggt taacaaatga a                                                21


<210> 74
<211> 18
<212> DNA
<213> Respiratory syncytial virus

<400> 74
ggggcaaata tgtcacga                                                    18


<210> 75
<211> 18
<212> DNA
<213> Respiratory syncytial virus

<400> 75
ggggcaaaca tgtcacga                                                    18


<210> 76
<211> 18
<212> DNA
<213> Respiratory syncytial virus

<400> 76
ggggcaaata tgtctaga                                                    18

<210> 77
<211> 9
<212> DNA
<213> Respiratory syncytial virus

<400> 77
ccaacatca                                                              9


<210> 78
<211> 9
<212> DNA
<213> Respiratory syncytial virus

<400> 78
ccgacgtca                                                              9


<210> 79
<211> 22
<212> DNA
<213> Respiratory syncytial virus

<400> 79
taatttaaaa ttaaggagag at                                              22


<210> 80
<211> 23
<212> DNA
<213> Respiratory syncytial virus

<400> 80
taatttaaaa cttaaggaga gat                                             23


<210> 81
<211> 21
<212> DNA
<213> Respiratory syncytial virus

<400> 81
ggggcaaata caaagatggc t                                               21


<210> 82
<211> 21
<212> DNA
<213> Respiratory syncytial virus

<400> 82
ggggcaaata caaccatggc t                                               21


<210> 83
<211> 15
<212> DNA
<213> Respiratory syncytial virus

<400> 83
atcctcactg cagtc                                                      15


<210> 84
<211> 15
<212> DNA
<213> Respiratory syncytial virus

<400> 84
atcctcaccg cagtc                                                      15


<210> 85
<211> 26
<212> DNA

<213> Respiratory syncytial virus

<400> 85
ttatcacaaa aagccatgac caactt                                          26


<210> 86
<211> 26
<212> DNA
<213> Respiratory syncytial virus

<400> 86
ttatcacaaa aggccttgac caactt                                          26


<210> 87
<211> 26
<212> DNA
<213> Respiratory syncytial virus

<400> 87
acgcgaaaaa atgcgtacaa caaact                                          26


<210> 88
<211> 26
<212> DNA
<213> Respiratory syncytial virus

<400> 88
acgggaaaaa atgcgtacaa caaact                                          26


<210> 89
<211> 24
<212> DNA
<213> Respiratory syncytial virus

<400> 89
tagttacaaa aaaaaggaaa gggt                                            24


<210> 90
<211> 23
<212> DNA
<213> Respiratory syncytial virus

<400> 90
tagttacaaa aaaaggaaag ggt                                             23